# EUROPEAN PATENT APPLICATION

(11) **EP 1 500 643 A1**
(43) Date of publication of application: **26.01.2005**
(21) Application number: 03715598.3
(22) Date of filing: 28.03.2003
(51) Int. Cl.: C07C 235/46, C07C 235/60, C07C 237/30, C07C 237/42, C07C 317/44, C07D 207/14, C07D 211/06, C07D 213/04, C07D 223/12, C07D 309/14, C07D 335/02, C07D 451/04, A61K 31/165, A61K 31/351, A61K 31/382, A61K 31/40, A61K 31/44, A61K 31/55, A61P 1/16, A61P 9/00, A61P 11/06

(54) **BENZAMIDE DERIVATIVES**

(30) Priority: 03.04.2002 JP 2002101081
(71) Applicant: Sumitomo Pharmaceuticals Company, Limited, Osaka 541-8510 (JP)
(72) Inventor: IMAZAKI, Naonori, Suita-shi, Osaka 564-0053 (JP); KITANO, Masafumi, Takatsuki-shi, Osaka 569-0081 (JP); FUJIBAYASHI, Tatsuya, Toyonaka-shi, Osaka 561-0802 (JP); ASANO, Shigehiro, Nishinomiya-shi, Hyogo 662-0831 (JP)
(74) Representative: Duckett, Anthony Joseph
(86) International application number: PCT/JP2003/003978
(87) International publication number: WO 2003/082808

(57) **Abstract**

A compound represented by formula (1): 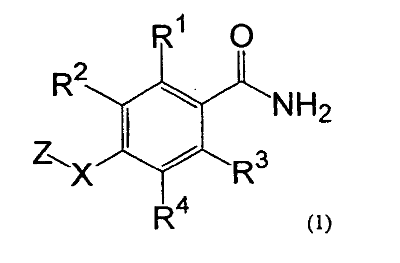 wherein X is a single bond or a substituted or unsubstituted lower alkylene group; Z is a saturated or unsaturated monocyclic hydrocarbon ring group or the like; and each of R¹, R², R³ and R⁴, which may be the same or different, is a hydrogen atom, a halogen atom, a nitro group, a cyano group, a carboxyl group, a substituted or unsubstituted alkyl group, or the like, a prodrug of said compound, or a pharmaceutically acceptable salt of said compound or prodrug has inhibitory effect on Rho kinase and hence is useful for treating diseases which are such that morbidity due to them is expected to be improved by inhibition of Rho kinase and secondary effects such as inhibition of the Na⁺/H⁺ exchange transport system caused by the Rho kinase inhibition, for example, hypertension.

## Description

### TECHNICAL FIELD

The present invention relates to a Rho kinase inhibitor (ROCK-II inhibitor, ROCα inhibitor) containing a novel substituted benzamide derivative, a prodrug thereof or a pharmaceutically acceptable salt of said derivative or prodrug.

It is known that Rho kinase participates in vasoconstriction (Nature, 389, 990-994, 1997, Circ. Res., 87, 195-2000, 2000), platelet aggregation (FEBS Lett., 466, 70-74, 2000, Blood, 94, 1665-1672, 1999), bronchial smooth muscle contraction (Am. J. Resp. Cell Mol. Biol. 20, 1190-1200, 1999), vascular smooth muscle proliferation•emigration (Circ. Res., 84, 1186-1193, 1999, Atherosclerosis, 155, 321-327, 2001), endothelial proliferation•emigration (Biochem. Biophys. Res. Commun., 269, 633-640, 2000), stress fiber formation (Science. 275, 1308-1311, 1997, J. Cell Biol. 150, 797-806, 2000), cardiac hypertrophy (Hypertension. 35, 313-318, 2000), Na/H exchange transport system activation (EMBO J., 17, 4712-4722, 1998), adducin activation (J. Biol. Chem., 273, 5542-5548, 1998), ocular hypertension (Invest. Ophthalmol. Visual Sci., 42, 137-44, 2001), erectile dysfunction (Nature Medicine., 7, 119-22, 2001), premature birth, retinopathy, inflammation, immune diseases, AIDS, fertilization and implantation of fertilized ovum, osteoporosis, brain functional disorder, infection of digestive tracts with bacteria (International Publication WO98/06433), and the like.

Therefore, a compound having inhibitory effect on Rho kinase is useful as a therapeutic agent for diseases which are such that morbidity due to them is expected to be improved by inhibition of Rho kinase and secondary effects such as inhibition of the Na⁺/H⁺ exchange transport system caused by the Rho kinase inhibition, for example, hypertension, peripheral circulatory disorder, angina pectoris, cerebral vasospasm, premature birth and asthma which are improved by smooth muscle relaxing effect, and diseases (chronic arterial obstruction and cerebrovascular accident) caused by hyperaggregability of platelet; diseases such as arteriosclerosis, fibroid lung, fibroid liver, liver failure, fibroid kidney, renal glomerulosclerosis, kidney failure, organ hypertrophy, prostatic hypertrophy, complications of diabetes, blood vessel restenosis, and cancer, which diseases are improved by inhibitory effect on cell over-proliferation•emigration•fibrosing (e.g. fibroblast proliferation, smooth muscle cell proliferation, mesangium cell proliferation and hemoendothelial cell proliferation); cardiac hypertrophy; heart failure; ischemic diseases; inflammation; autoimmune diseases; AIDS; osteopathy such as osteoporosis; brain functional disorder; infection of digestive tracts with bacteria; sepsis; adult respiratory distress syndrome; retinopathy; glaucoma; and erectile dysfunction.

### BACKGROUND ART

As compounds having inhibitory activity against Rho kinase, there are exemplified the compounds disclosed in International Patent Laid-Open Nos. WO98/06433, WO99/64011 and WO00/57914. These compounds are different in structure from the compounds of the present invention.

### DISCLOSURE OF THE INVENTION

A problem to be solved by the present invention is to provide a compound that has inhibitory activity against Rho kinase and is useful as a therapeutic agent for the diseases described above.

The present inventors earnestly investigated in order to solve the above problem, and consequently found that a compound represented by general formula (1), a prodrug thereof or a pharmaceutically acceptable salt of said compound or prodrug (they are hereinafter abbreviated as the compounds of the present invention if necessary) has an excellent inhibitory effect on Rho kinase. That is, the present invention relates to the following items [1] to [22].
[1] A compound represented by formula (1): 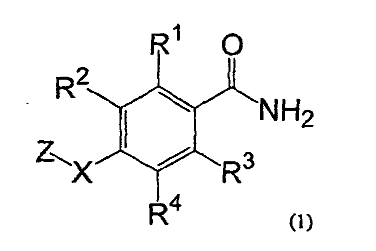 wherein X is a single bond or a substituted or unsubstituted lower alkylene group (the -CH₂- group(s) of said lower alkylene group may be substituted by one or more groups which may be the same or different and are selected from groups represented by the formulas: -O-, -S(O)ₙ-, -N(R⁵)-, -N(R⁶)C(=O)-, -C(=O)N(R⁶)-, -N(R⁶)S(O)₂-, -S(O)₂N(R⁶)- and -C(=O)-, benzene ring and cycloalkane rings (the -CH₂- group(s) in said cycloalkane ring may be substituted by one or more groups which may be the same or different and are selected from groups represented by the formulas: -O-, -S-, -N(R⁷)- and -C(=O)-), and a combination(s) of any adjacent two carbon atoms of said lower alkylene group may form one or more double or triple bonds which may be the same or different);
   Z is a saturated or unsaturated monocyclic hydrocarbon ring group, a saturated or unsaturated polycyclic hydrocarbon ring group, a saturated or unsaturated monocyclic heterocyclic group, or a saturated or unsaturated polycyclic heterocyclic group (these groups may be unsubstituted or may have a substituent(s));
   each of R¹, R², R³ and R⁴, which may be the same or different, is a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, a halogen atom, a nitro group, a cyano group, a carboxyl group, a substituted or unsubstituted alkyl group, a substituted or unsubstituted alkenyl group, a substituted or unsubstituted alkynyl group, a substituted or unsubstituted alkoxycarbonyl group, a substituted or unsubstituted acyl group, or a group represented by the formula: -OR⁹, -N(R¹⁰)R¹¹-, -C(=O)N(R¹⁰)R¹¹, -S(O)₂N(R¹⁰)R¹¹ or -S(O)ₘR¹²;
   n and m are independently 0, 1 or 2;
   R⁵ (or R⁵s) and R⁷ (or R⁷s), which may be the same or different, and are a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, a substituted or unsubstituted alkyl group, a substituted or unsubstituted acyl group, a substituted or unsubstituted alkoxycarbonyl group, or a group represented by the formula: -C(=O)N(R¹⁰)R¹¹, -S(O)₂N(R¹⁰)R¹¹ or -S(O)ₘR¹², independently, when it exists more than one;
   R⁶ (or R⁶s) and R⁹ (or R⁹s), which may be the same or different, and are a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, a substituted or unsubstituted alkyl group, a substituted or unsubstituted acyl group, or a substituted or unsubstituted alkoxycarbonyl group, independently, when it exists more than one;
   R¹⁰ (or R¹⁰s) and R¹¹ (or R¹¹s), which may be the same or different, and are a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, a substituted or unsubstituted alkyl group, a substituted or unsubstituted acyl group, or a substituted or unsubstituted alkoxycarbonyl group, independently, when it exists more than one, or R¹⁰ and R¹¹, when taken together with the nitrogen atom to which they are bonded, represent a saturated 3- to 8-membered cyclic amino group which may contain other heteroatom(s) in the ring (said cyclic amino group may be substituted by one or more substituted or unsubstituted alkyl groups, -S(O)ₘR¹² groups or -OR⁹ groups) ; and
   R¹² is a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, or a substituted or unsubstituted alkyl group;
   said compound not including the following compounds:
   (i) compounds in which X is a group represented by the formula: -O- or -S-, and Z is pyrrolidin-3-yl having a substituent at the 4-position, piperidin-3-yl having a substituent at the 4-position, piperidin-4-yl having a substituent at the 3-position, homopiperidin-3-yl having a substituent at the 4-position, homopiperidin-4-yl having a substituent at the 5-position, or homopiperidin-4-yl having a substituent at the 3-position, said substituent of each of them being an oxo group, a hydroxyl group, a substituted or unsubstituted alkoxy group, a substituted or unsubstituted alkenyloxy group, a substituted or unsubstituted alkynyloxy group, a substituted or unsubstituted aralkyloxy group, an alkenyloxy group substituted by a substituted or unsubstituted aryl group, an alkynyloxy group substituted by a substituted or unsubstituted aryl group, a substituted or unsubstituted aryloxy group, a substituted or unsubstituted acyloxy group, a benzyloxycarbonyloxy group, or an alkylcarbamoyloxy group, and
   (ii) compounds in which X is a group represented by the formula: -O-, and Z is a group represented by the formula: 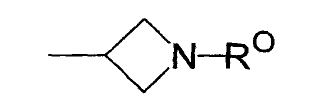
   wherein R⁰ is a hydrogen atom, a substituted or unsubstituted carbamoyl group, or a substituted or unsubstituted thiocarbamoyl group,
   a prodrug of said compound, or a pharmaceutically acceptable salt of said compound or prodrug.
[2] A compound, a prodrug thereof or a pharmaceutically acceptable salt of the compound or prodrug according to the above item [1], wherein X is a group represented by the formula: -N(R⁵)-, -C(=O)N(R⁶)-, -CH₂N(R⁵)-, -CH₂- or -O-.
[3] A compound, a prodrug thereof or a pharmaceutically acceptable salt of the compound or prodrug according to the above item [1], wherein X is a group represented by the formula: -N(R⁵)-.
[4] A compound, a prodrug thereof or a pharmaceutically acceptable salt of the compound or prodrug according to the above item [1], wherein X is a group represented by the formula: -O-.
[5] A compound, a prodrug thereof or a pharmaceutically acceptable salt of the compound or prodrug according to the above item [1], [2], [3] or [4], wherein at least one of R¹ and R³ is a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a halogen atom, a nitro group, a cyano group, a carboxyl group, a substituted or unsubstituted alkyl group, a substituted or unsubstituted alkenyl group, a substituted or unsubstituted alkynyl group, a substituted or unsubstituted alkoxycarbonyl group, a substituted or unsubstituted acyl group, or a group represented by the formula: -OR⁹, -N(R¹⁰)R¹¹, -C(=O)N(R¹⁰)R¹¹, -S(O)₂N(R¹⁰)R¹¹ or -S(O)ₘR¹².
[6] A compound, a prodrug thereof or a pharmaceutically acceptable salt of the compound or prodrug according to the above item [1], [2], [3] or [4], wherein at least one of R² and R⁴ is a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a halogen atom, a nitro group, a cyano group, a carboxyl group, a substituted or unsubstituted alkyl group, a substituted or unsubstituted alkenyl group, a substituted or unsubstituted alkynyl group, a substituted or unsubstituted alkoxycarbonyl group, a substituted or unsubstituted acyl group, or a group represented by the formula: -OR⁹, -N(R¹⁰)R¹¹, -C(=O)N(R¹⁰)R¹¹, -S(O)₂N(R¹⁰)R¹¹ or -S(O)ₘR¹².
[7] A compound, a prodrug thereof or a pharmaceutically acceptable salt of the compound or prodrug according to the above item [1], [2], [3] or [4], wherein each of R¹ and R⁴, which may be the same or different, or each of R³ and R², which may be the same or different, is a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a halogen atom, a nitro group, a cyano group, a carboxyl group, a substituted or unsubstituted alkyl group, a substituted or unsubstituted alkenyl group, a substituted or unsubstituted alkynyl group, a substituted or unsubstituted alkoxycarbonyl group, a substituted or unsubstituted acyl group, or a group represented by the formula: -OR⁹, -N(R¹⁰)R¹¹, -C(=O)N(R¹⁰)R¹¹, -S(O)₂N(R¹⁰)R¹¹ or -S(O)ₘR¹².
[8] A compound, a prodrug thereof or a pharmaceutically acceptable salt of the compound or prodrug according to the above item [1], [2], [3], [4], [5] or [7], wherein at least one of R¹ and R³ is a group represented by the formula: -OR⁹ or a halogen atom.
[9] A compound, a prodrug thereof or a pharmaceutically acceptable salt of the compound or prodrug according to the above item [1], [2], [3], [4], [5] or [7], wherein at least one of R¹ and R³ is a methoxy group.
[10] A compound, a prodrug thereof or a pharmaceutically acceptable salt of the compound or prodrug according to the above item [1], [2], [3], [4], [5] or [7], wherein at least one of R¹ and R³ is a fluorine atom.
[11] A compound, a prodrug thereof or a pharmaceutically acceptable salt of the compound or prodrug according to the above item [1], [2], [3], [4], [6] or [7], wherein at least one of R² and R⁴ is a halogen atom.
[12] A compound, a prodrug thereof or a pharmaceutically acceptable salt of the compound or prodrug according to the above item [1], [2], [3], [4], [6] or [7], wherein at least one of R² and R⁴ is a chlorine atom.
[13] A compound, a prodrug thereof or a pharmaceutically acceptable salt of the compound or prodrug according to the above item [1], [2], [3], [4] or [7], wherein R¹ is a group represented by the formula: -OR⁹ or a halogen atom and R⁴ is a halogen atom; or R³ is a group represented by the formula: -OR⁹ or a halogen atom and R² is a halogen atom.
[14] A compound, a prodrug thereof or a pharmaceutically acceptable salt of the compound or prodrug according to the above item [1], [2], [3], [4] or [7], wherein R¹ is a methoxy group and R⁴ is a chlorine atom; or R³ is a methoxy group and R² is a chlorine atom.
[15] A compound, a prodrug thereof or a pharmaceutically acceptable salt of the compound or prodrug according to the above item [1], [2], [3], [4] or [7], wherein R¹ is a fluorine atom and R⁴ is a chlorine atom; or R³ is a fluorine atom and R² is a chlorine atom.
[16] A compound, a prodrug thereof or a pharmaceutically acceptable salt of the compound or prodrug according to any one of the above items [1] to [15], wherein Z is a substituted or unsubstituted saturated monocyclic heterocyclic group or a substituted saturated monocyclic hydrocarbon ring group, and the substituent(s) of the saturated monocyclic hydrocarbon ring group is a group represented by the formula: -N(R^{Z1})R^{Z2} wherein R^{Z1} (or R^{Z1}s) and R^{Z2} (or R^{Z2}s), which may be the same or different, and are a hydrogen atom, a substituted or unsubstituted alkyl group or a substituted or unsubstituted aralkyl group, independently, when it exists more than one.
[17] A compound, a prodrug thereof or a pharmaceutically acceptable salt of the compound or prodrug according to the above item [1], said compound being 4-[trans-(4-aminocyclohexyl)amino]-2-fluorobenzamide, 4-[trans-(4-aminocyclohexyl)amino]-5-chloro-2-fluorobenzamide, 5-chloro-4-{[trans-4-(isopropylamino)cyclohexyl]amino}-2-methoxybenzamide, 4-[trans-(4-aminocyclohexyl)amino]-2,5-difluorobenzamide or 5-chloro-2-fluoro-4-(piperidin-4-ylamino)benzamide.
[18] A medicament comprising a compound, a prodrug thereof or a pharmaceutically acceptable salt of the compound or prodrug according to any one of the above items [1] to [17].
[19] A Rho kinase inhibitor comprising a compound, a prodrug thereof or a pharmaceutically acceptable salt of the compound or prodrug according to any one of the above items [1] to [17].
[20] A pharmaceutical composition for the treatment of hypertension, peripheral circulatory disorder, angina pectoris, cerebral vasospasm, premature birth, asthma, cerebrovascular accident, arteriosclerosis, fibroid lung, fibroid liver, fibroid kidney, renal glomerulosclerosis, kidney failure, prostatic hypertrophy, complications of diabetes, blood vessel restenosis, cancer, cardiac hypertrophy, heart failure, ischemic diseases, inflammation, autoimmune diseases, AIDS, osteopathy, brain functional disorder, infection of digestive tracts with bacteria, sepsis, adult respiratory distress syndrome, retinopathy, glaucoma, or erectile dysfunction, which comprises a compound represented by formula (1): 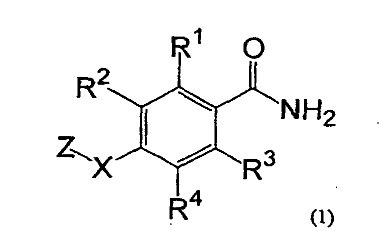 wherein X is a single bond or a substituted or unsubstituted lower alkylene group (the -CH₂- group(s) of said lower alkylene group may be substituted by one or more groups which may be the same or different and are selected from groups represented by the formulas: -O-, -S(O)ₙ-, -N(R⁵)-, -N(R⁶)C(=O)-, -C(=O)N(R⁶)-, -N(R⁶)S(O)₂-, -S(O)₂N(R⁶)- and -C(=O)-, benzene ring and cycloalkane rings (the -CH₂- group(s) in said cycloalkane ring may be substituted by one or more groups which may be the same or different and are selected from groups represented by the formulas: -O-, -S-, -N(R⁷)- and -C(=O)-), and a combination (s) of any adjacent two carbon atoms of said lower alkylene group may form one or more double or triple bonds which may be the same or different);
   Z is a saturated or unsaturated monocyclic hydrocarbon ring group, a saturated or unsaturated polycyclic hydrocarbon ring group, a saturated or unsaturated monocyclic heterocyclic group, or a saturated or unsaturated polycyclic heterocyclic group (these groups may be unsubstituted or may have a substituent(s));
   each of R¹, R², R³ and R⁴, which may be the same or different, is a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, a halogen atom, a nitro group, a cyano group, a carboxyl group, a substituted or unsubstituted alkyl group, a substituted or unsubstituted alkenyl group, a substituted or unsubstituted alkynyl group, a substituted or unsubstituted alkoxycarbonyl group, a substituted or unsubstituted acyl group, or a group represented by the formula: -OR⁹, -N(R¹⁰)R¹¹, -C(=O)N(R¹⁰)R¹¹, -S(O)₂N(R¹⁰)R¹¹ or -S(O)ₘR¹²;
   n and m are independently 0, 1 or 2;
   R⁵ (or R⁵s) and R⁷ (or R⁷s), which may be the same or different, and are a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, a substituted or unsubstituted alkyl group, a substituted or unsubstituted acyl group, a substituted or unsubstituted alkoxycarbonyl group, or a group represented by the formula: -C(=O)N(R¹⁰)R¹¹, -S(O)₂N(R¹⁰)R¹¹ or -S(O)ₘR¹², independently, when it exists more than one;
   R⁶ (or R⁶s) and R⁹ (or R⁹s), which may be the same or different, and are a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, a substituted or unsubstituted alkyl group, a substituted or unsubstituted acyl group, or a substituted or unsubstituted alkoxycarbonyl group, independently, when it exists more than one;
   R¹⁰ (or R¹⁰s) and R¹¹ (or R¹¹s), which may be the same or different, and are a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, a substituted or unsubstituted alkyl group, a substituted or unsubstituted acyl group, or a substituted or unsubstituted alkoxycarbonyl group, independently, when it exists more than one, or R¹⁰ and R¹¹, when taken together with the nitrogen atom to which they are bonded, represent a saturated 3- to 8-membered cyclic amino group which may contain other heteroatom(s) in the ring (said cyclic amino group may be substituted by one or more substituted or unsubstituted alkyl groups, -S(O)ₘR¹² groups or -OR⁹ groups); and
   R¹² is a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, or a substituted or unsubstituted alkyl group;
   said compound not including the following compounds:
   compounds in which X is a group represented by the formula: -O-, and Z is pyrrolidin-3-yl having a substituent at the 4-position, said substituent being a hydroxyl group, an alkoxy group, a benzyloxycarbonyloxy group or an alkylcarbamoyloxy group, a prodrug of said compound, or a pharmaceutically acceptable salt of said compound or prodrug.
[21] A method for treating hypertension, peripheral circulatory disorder, angina pectoris, cerebral vasospasm, premature birth, asthma, cerebrovascular accident, arteriosclerosis, fibroid lung, fibroid liver, fibroid kidney, renal glomerulosclerosis, kidney failure, prostatic hypertrophy, complications of diabetes, blood vessel restenosis, cancer, cardiac hypertrophy, heart failure, ischemic diseases, inflammation, autoimmune diseases, AIDS, osteopathy, brain functional disorder, infection of digestive tracts with bacteria, sepsis, adult respiratory distress syndrome, retinopathy, glaucoma, or erectile dysfunction, which comprises administering an effective amount of a compound, a prodrug thereof or a pharmaceutically acceptable salt of the compound or prodrug according to the above item [20] to a patient who needs the treatment.
[22] Use of a compound, a prodrug thereof or a pharmaceutically acceptable salt of the compound or prodrug according to the above item [20] in the manufacture of a medicament for the treatment of hypertension, peripheral circulatory disorder, angina pectoris, cerebral vasospasm, premature birth, asthma, cerebrovascular accident, arteriosclerosis, fibroid lung, fibroid liver, fibroid kidney, renal glomerulosclerosis, kidney failure, prostatic hypertrophy, complications of diabetes, blood vessel restenosis, cancer, cardiac hypertrophy, heart failure, ischemic diseases, inflammation, autoimmune diseases, AIDS, osteopathy, brain functional disorder, infection of digestive tracts with bacteria, sepsis, adult respiratory distress syndrome, retinopathy, glaucoma, or erectile dysfunction.

### BEST MODE FOR CARRYING OUT THE INVENTION

The various groups in the present invention are explained below. The following explanation applies also to cases where any of the groups is a portion of another group, unless otherwise specified.

Each of the terms "saturated or unsaturated monocyclic hydrocarbon ring group", "saturated or unsaturated polycyclic hydrocarbon ring group", "saturated or unsaturated monocyclic heterocyclic group" and "saturated or unsaturated polycyclic heterocyclic group" means a group formed by the conversion of one of the hydrogen atoms of a corresponding saturated or unsaturated monocyclic hydrocarbon ring, saturated or unsaturated polycyclic hydrocarbon ring, saturated or unsaturated monocyclic heterocyclic ring, or saturated or unsaturated polycyclic heterocyclic ring, respectively, examples of which are given below, to a bond.

As the saturated or unsaturated monocyclic hydrocarbon ring, there are exemplified 3- to 8-membered hydrocarbon rings such as cyclopropane, cyclobutane, cyclopentane, cyclohexane, cycloheptane, cyclooctane, cyclopentene, cyclohexene, cycloheptene, cyclooctene, benzene, etc.

As the saturated or unsaturated polycyclic hydrocarbon ring, there are exemplified polycyclic hydrocarbon rings of 16 or less carbon atoms, such as indene, naphthalene, azulene, fluorene, phenalene, phenanthrene, anthracene, acephenanthrylene, 1,2-dihydronaphthalene, 6,7-dihydro-5H-benzocycloheptene, benzocyclooctene, 1,2,3,4-tetrahydronaphthalene, decahydronaphthalene, octahydro-1H-indene, etc.; and polycyclic hydrocarbon rings of 12 or less carbon atoms having a crosslinkage, such as adamantane, bicyclo[2,2,2]octane, bicyclo[3,3,3]undecane, bicyclo[2,2,2]oct-2-ene, bicyclo[3,3,3]undec-2-ene, etc.

The saturated or unsaturated monocyclic heterocyclic ring includes, for example, 3- to 8-membered unsaturated monocyclic heterocyclic rings containing 1 to 4 nitrogen atoms, 3- to 8-membered saturated monocyclic heterocyclic rings containing 1 to 4 nitrogen atoms, 3- to 8-membered unsaturated monocyclic heterocyclic rings containing an oxygen atom, 3- to 8-membered unsaturated monocyclic heterocyclic rings containing one or two sulfur atoms, 3- to 8-membered unsaturated monocyclic heterocyclic rings containing 1 to 3 nitrogen atoms and one or two oxygen atoms, 3- to 8-membered saturated monocyclic heterocyclic rings containing 1 to 3 nitrogen atoms and one or two oxygen atoms, 3- to 8-membered unsaturated monocyclic heterocyclic rings containing 1 to 3 nitrogen atoms and one or two sulfur atoms, 3- to 8-membered saturated monocyclic heterocyclic rings containing 1 to 3 nitrogen atoms and one or two sulfur atoms, and 3- to 8-membered unsaturated monocyclic heterocyclic rings containing an oxygen atom and one or two sulfur atoms.

The 3- to 8-membered unsaturated monocyclic heterocyclic rings containing 1 to 4 nitrogen atoms include, for example, pyrrole, pyrroline, pyridine, dihydropyridine, imidazole, pyrazole, imidazoline, pyrazine, pyrimidine, pyridazine, pyrazole, triazole and tetrazole, etc.

The 3- to 8-membered saturated monocyclic heterocyclic rings containing 1 to 4 nitrogen atoms include, for example, pyrrolidine, piperidine, imidazolidine, pyrazolidine, and piperazine, etc.

The 3- to 8-membered unsaturated monocyclic heterocyclic rings containing an oxygen atom include, for example, furan and pyran, etc.

The 3- to 8-membered unsaturated monocyclic heterocyclic rings containing one or two sulfur atoms include, for example, thiophene, dihydrodithiin and dihydrodithion, etc.

The 3- to 8-membered unsaturated monocyclic heterocyclic rings containing 1 to 3 nitrogen atoms and one or two oxygen atoms include, for example, oxazole, oxadiazole and isoxazole, etc.

The 3- to 8-membered saturated monocyclic heterocyclic rings containing 1 to 3 nitrogen atoms and one or two oxygen atoms include, for example, morpholine and oxazolidine, etc.

The 3- to 8-membered unsaturated monocyclic heterocyclic rings containing 1 to 3 nitrogen atoms and one or two sulfur atoms include, for example, thiazole, isothiazole and thiadiazole, etc.

The 3- to 8-membered saturated monocyclic heterocyclic rings containing 1 to 3 nitrogen atoms and one or two sulfur atoms include, for example, thiazolidine, etc.

The 3- to 8-membered unsaturated monocyclic heterocyclic rings containing an oxygen atom and one or two sulfur atoms include, for example, dihydrooxathiin, etc.

The saturated or unsaturated polycyclic heterocyclic ring includes, for example, saturated or unsaturated fused heterocyclic rings containing 1 to 4 nitrogen atoms, unsaturated fused heterocyclic rings containing 1 to 3 nitrogen atoms and one or two oxygen atoms, unsaturated fused heterocyclic rings containing 1 to 3 nitrogen atoms and one or two sulfur atoms, unsaturated fused heterocyclic rings containing one or two oxygen atoms, unsaturated fused heterocyclic rings containing an oxygen atom and one or two sulfur atoms, and unsaturated fused heterocyclic rings containing one or two sulfur atoms.

The saturated or unsaturated fused heterocyclic rings containing 1 to 4 nitrogen atoms include, for example, indole, isoindole, indoline, quinoline, isoquinoline, quinolizine, indazole, phthalazine, naphthyridine, quinoxaline, quinazoline, cinnoline, carbazole, purine, pteridine, phenazine, carboline, phenanthridine, acridine, indoline, isoindoline, 1,2-dihydroisoquinoline, benzimidazole, imidazopyridine, benzotriazole, tetrahydroimidazopyridine, benz[b]azepine, benz[cd]indole, cyclohepta[cd]indole, pyrrolo[3,2,1-ij]quinoline, cyclohexa[b]pyridine, cyclohepta[b]pyridine, pyrrolo[1,2,3-de]quinoxaline, pyrrolo[3,2,1-hi]indole, pyrrolo[3,2,1-jk][1]benzazepine, pyrrolo[3,2,1-kl][1]benzoazocine, pyrrolo[3,2,1-kl]benzo-[e][1,4]diazocine, 1,2,3,4-tetrahydroquinoline, 1,2,3,4-tetrahydroisoquinoline, decahydroquinoline, decahydroisoquinoline, octahydroindole, quinuclidine, 1-azabicyclo[2,2,1]heptane and 1-azabicyclo[3,2,1]octane, etc.

The unsaturated fused heterocyclic rings containing 1 to 3 nitrogen atoms and one or two oxygen atoms include, for. example, benzoxazole, benzoxadiazole, phenoxazine, pyrrolo[1,2,3-de][1,4]benzoxazine, pyrrolo[2,1-c][1,4]benzoxazine and pyrrolo[3,2,1-kl)benz[e]-[4,1]oxazocine, etc. Preferable examples thereof are benzoxazole, pyrrolo[1,2,3-de][1,4]benzoxazine, pyrrolo[2,1-c][1,4]benzoxazine and pyrrolo[3,2,1-kl]benz[e][4,1]oxazocine.

The unsaturated fused heterocyclic rings containing 1 to 3 nitrogen atoms and one or two sulfur atoms include, for example, benzothiazole, benzothiadiazole, 1,4-benzothiazine and phenothiazine, etc. Preferable examples thereof are benzothiazole and 1,4-benzothiazine.

The unsaturated fused heterocyclic rings containing one or two oxygen atoms include, for example, benzofuran, dihydrobenzofuran, chromene, isobenzofuran, xanthene, isochroman, chroman and benz[b]oxepine, etc. Preferable examples thereof are benzofuran and benz[b]oxepine.

The unsaturated fused heterocyclic rings containing an oxygen atom and one or two sulfur atoms include, for example, 1,4-benzoxathiin and phenoxathiin, etc.

The unsaturated fused heterocyclic rings containing one or two sulfur atoms include, for example, benzothiophene, benzothiin, benzothiopyran, thiochroman and thianthrene, etc. Preferable examples thereof are benzothiophene, benzothiopyran and thiochroman.

The substituent(s) of each of the saturated or unsaturated monocyclic hydrocarbon ring, saturated or unsaturated polycyclic hydrocarbon ring, saturated or unsaturated monocyclic heterocyclic ring, saturated or unsaturated polycyclic heterocyclic ring, phenyl group, naphthyl group, heterocyclic group, aroyl group, saturated heterocyclic ring-carbonyl group and heteroaromatic acyl group may be present in a number of one or more and may be the same or different. The substituent(s) includes, for example, hydrogen atom, substituted or unsubstituted alkyl groups, substituted or unsubstituted alkenyl groups, substituted or unsubstituted alkynyl groups, substituted or unsubstituted cycloalkyl groups, substituted or unsubstituted cycloalkenyl groups, aralkyl groups, phenyl group, naphthyl group, heterocyclic groups, acyl groups (said aralkyl groups, phenyl group, naphthyl group, heterocyclic groups and acyl groups may be substituted by one or more substituents which may be the same or different and are selected from alkyl groups, alkoxy groups, alkylenedioxy groups and halogen atoms), substituted or unsubstituted alkoxycarbonyl groups, carboxyl group, halogen atoms, nitro group and groups represented by the formulas: -CN, -OR¹⁴, -N(R¹⁵)R¹⁶, -C(=O)N(R¹⁵)R¹⁶, -S(O)₂N(R¹⁵)R¹⁶, -S(O)ₙR¹³, 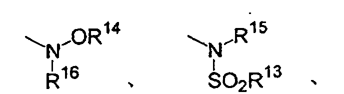 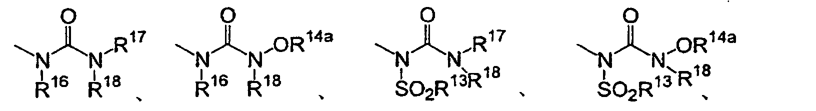 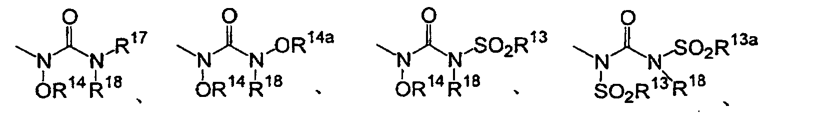 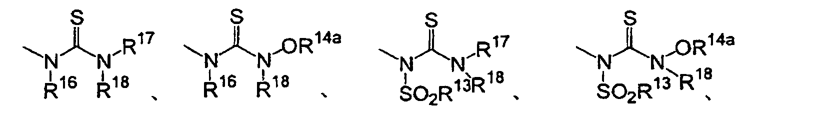 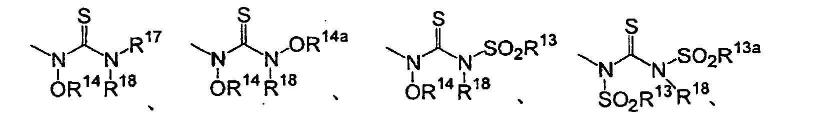 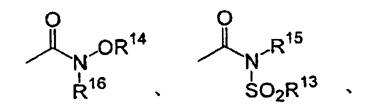 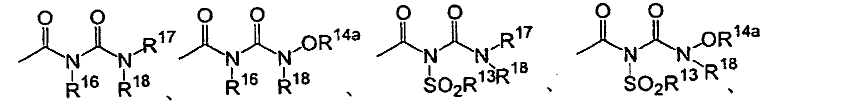 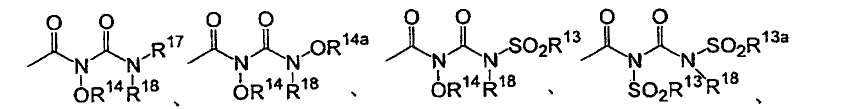 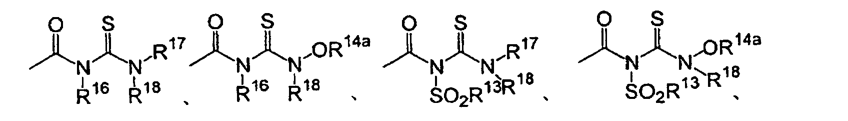 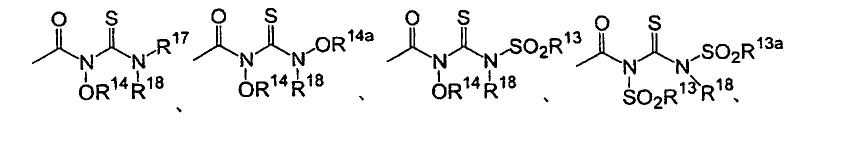 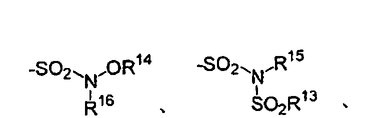 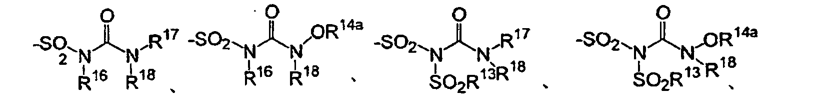 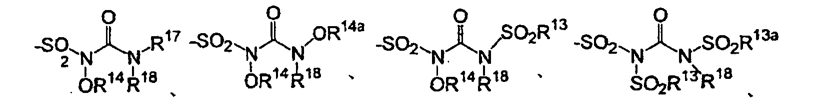 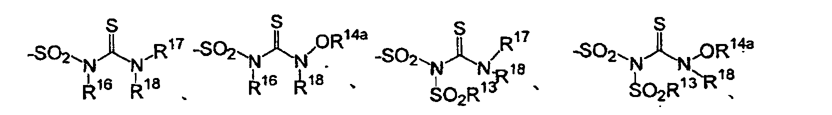 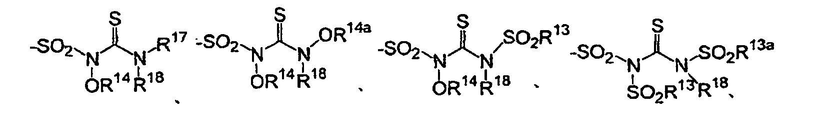 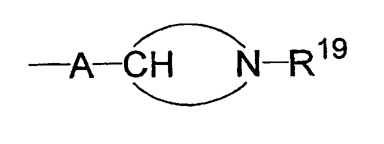 wherein A is an oxygen atom, -S(O)ᵣ- or -N(R²¹)-; r is an integer of 0, 1 or 2; R¹⁹ and R²¹ are independently a hydrogen atom, an alkyl group, an alkenyl group, an alkynyl group, a cycloalkyl group, a phenyl group, a naphthyl group, a saturated or unsaturated heterocyclic group or an acyl group; and the ring is a 3- to 8-membered saturated heterocyclic ring composed of a nitrogen atom and carbon atoms.

R¹³ and R^{13a} are independently a hydroxyl group, an alkyl group, a cycloalkyl group, a phenyl group, a naphthyl group or a saturated or unsaturated heterocyclic group.

R¹⁵, R¹⁶, R¹⁷ and R¹⁸ are independently a hydrogen atom, an alkyl group, an aralkyl group, an alkenyl group, an alkynyl group, a cycloalkyl group, a cycloalkenyl group, a phenyl group, a naphthyl group, a saturated or unsaturated heterocyclic group or an acyl group. Alternatively, R¹⁵ and R¹⁶, or R¹⁷ and R¹⁸, when taken together with the nitrogen atom to which they are bonded, represent a saturated 3- to 8-membered cyclic amino group which may contain other heteroatom(s) in the ring (said cyclic amino group may be substituted by one or more substituted or unsubstituted alkyl groups, -S(O)ₙR¹³ groups, -N(R¹⁴)R^{14a} groups, hydroxyl groups or -OR^{14b} groups).

R¹⁴, R^{14a} and R^{14b} are independently a hydrogen atom, an alkyl group, a cycloalkyl group, a cycloalkenyl group, a phenyl group, a naphthyl group or a saturated or unsaturated heterocyclic group.

The substituent(s) of each of the cycloalkyl group, cycloalkenyl group and cycloalkanecarbonyl group may be present in a number of, for example, 1 to 4 and may be the same or different. The substituent(s) includes, for example, alkyl groups, substituted alkyl groups, hydroxyl group and groups represented by the formulas: -OR¹⁴, -S(O)ₙR¹³, -N(R¹⁵)R¹⁶, -C(=O)N(R¹⁵)R¹⁶, -S(O)₂N(R¹⁵)R¹⁶ and -S(O)ₙR¹³.

The heterocyclic group includes saturated or unsaturated heterocyclic groups. The saturated or unsaturated heterocyclic groups include saturated or unsaturated monocyclic heterocyclic groups and saturated or unsaturated polycyclic heterocyclic groups.

The alkyl group includes, for example, linear or branched alkyl groups of 8 or less carbon atoms, such as methyl, ethyl, propyl, 2-propyl, butyl, 2-butyl, 2-methylpropyl, 1,1-dimethylethyl, pentyl, hexyl, heptyl, octyl, etc.

The cycloalkane ring includes, for example, 3- to 8-membered cycloalkane rings such as cyclopropane, cyclobutane, cyclopentane, cyclohexane, cycloheptane, cyclooctane, etc.

The cycloalkyl group includes, for example, 3- to 8-membered cycloalkyl groups such as cyclopropyl, cyclobutyl, cyclopentyl, cyclohexyl, cycloheptyl, etc.

The cycloalkenyl group includes, for example, 3- to 8-membered cycloalkenyl groups having a double bond, such as 1-cyclopentenyl, 2-cyclopentenyl, 3-cyclopentenyl, 1-cyclohexenyl, 2-cyclohexenyl, 3-cyclohexenyl, etc.

The alkenyl group includes, for example, alkenyl groups of 6 or less carbon atoms, such as vinyl, allyl, propenyl, 2-propenyl, butenyl, pentenyl, hexenyl, etc.

The alkynyl group includes, for example, alkynyl groups of 6 or less carbon atoms, such as ethynyl, propargyl, butynyl, pentynyl, etc.

The halogen atom includes, for example, iodine, fluorine, chlorine and bromine atoms.

The acyl group includes, for example, formyl group; alkanoyl groups of 2 to 6 carbon atoms, such as acetyl, propanoyl, etc.; cycloalkanecarbonyl groups of 4 to 7 carbon atoms, such as cyclopropanecarbonyl, cyclobutanecarbonyl, cyclopentanecarbonyl, cyclohexanecarbonyl, etc.; cycloalkenecarbonyl groups of 3 to 6 carbon atoms, such as cyclopentenecarbonyl, cyclohexenecarbonyl, etc.; aroyl groups of 6 to 10 carbon atoms, such as benzoyl, toluoyl, naphthoyl, etc.; saturated heterocyclic ring-carbonyl groups having a 5- or 6-membered saturated heterocyclic ring containing one or two heteroatoms selected from nitrogen atom, oxygen atom and sulfur atom, such as 2-piperidinecarbonyl, 3-morpholine-carbonyl, etc.; and heteroaromatic acyl groups having a 5- or 6-membered heteroaromatic ring containing one or two heteroatoms selected from nitrogen atom, oxygen atom and sulfur atom, such as furoyl, thenoyl, nicotinoyl, isonicotinoyl, etc.

The substituent(s) of the substituted alkyl group may be present in a number of one or more and may be the same or different. The substituent(s) includes halogen atoms, hydroxyl group, alkoxy groups, alkoxycarbonyl groups, cycloalkyl groups, cyano group, carboxyl group, acyl groups, phenyl group, naphthyl group, saturated or unsaturated heterocyclic groups, oxo group, thioxo group and groups represented by the formulas: -C(=O)N(R³⁵)R³⁶, -S(O)₂R³³, -S(O)₂N(R³⁵)R³⁶ and -N(R³⁵)R³⁶, and the formula: 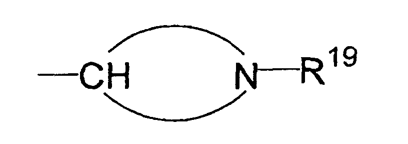 wherein R¹⁹ is as defined above, and the ring represents a 3- to 8-membered saturated heterocyclic group composed of a nitrogen atom and carbon atoms.

As R³³, R³⁵ and R³⁶, the same groups as those described above as R¹³, R¹⁵ and R¹⁶ are exemplified respectively.

Such a substituted alkyl group includes, for example, alkyl groups of 1 to 5 carbon atoms substituted by a cycloalkyl group of 3 to 6 carbon atoms, polyhaloalkyl groups of 1 to 5 carbon atoms, hydroxyalkyl groups of 1 to 6 carbon atoms, alkoxyalkyl groups of 2 to 6 carbon atoms, cyanoalkyl groups of 2 to 6 carbon atoms, carboxyalkyl groups of 2 to 6 carbon atoms, alkoxycarbonylalkyl groups of 3 to 8 carbon atoms, alkanoylalkyl groups of 3 to 8 carbon atoms, aroylalkyl groups of 16 or less carbon atoms, substituted or unsubstituted phenyl- or naphthyl-C1∼C5 alkyl groups, carbamoyl-C1∼C3 alkyl groups which may be substituted by one or two C1∼C3 alkyl groups on the nitrogen atom, amino-C1∼C5 alkyl groups which may be substituted by one or two C1∼C3 alkyl or C7∼C11 aralkyl groups on the nitrogen atom, and saturated 3- to 8-membered cyclic amino-C1∼C3 alkyl groups, etc.

Typical examples of the substituted alkyl group are polyhaloalkyl groups of 1 to 3 carbon atoms, such as trifluoromethyl, trifluoroethyl, trichloromethyl, etc.; hydroxyalkyl groups of 1 to 6 carbon atoms, such as hydroxymethyl, hydroxyethyl, 1-hydroxyethyl, etc.; aminoalkyl groups of 1 to 5 carbon atoms, such as aminomethyl, aminoethyl, 1-aminoethyl, etc.; alkoxyalkyl groups of 1 to 6 carbon atoms, such as methoxyethyl, ethoxyethyl, methoxypropyl, etc.; carboxyalkyl groups of 2 to 6 carbon atoms, such as carboxyethyl, carboxypropyl, etc.; alkoxycarbonylalkyl groups of 3 to 7 carbon atoms, such as methoxycarbonylmethyl, ethoxycarbonylmethyl, methoxycarbonylethyl, etc.; phenyl- or naphthyl-C1∼C5 alkyl groups (which may have a substituent such as a C1∼C3 alkyl group, halogen atom, nitro group, amino group, hydroxyl group, C1∼C3 alkoxy group or the like in the phenyl or naphthyl portion) such as benzyl, phenylethyl, phenylpropyl, phenylbutyl, 1- or 2-naphthylmethyl, etc.; carbamoyl-C1∼C3 alkyl groups which may be substituted by one or two C1∼C3 alkyl groups on the nitrogen atom, such as carbamoylmethyl, carbamoylethyl, dimethylcarbamoylmethyl, etc.; amino-C1∼C5 alkyl groups which may be substituted by one or two C1∼C3 alkyl or C7∼C11 aralkyl groups on the nitrogen atom, such as aminoethyl, aminopropyl, dimethylaminoethyl, dimethylaminopropyl, diethylaminoethyl, N-methyl-N-benzylaminoethyl, etc.; and saturated 3- to 8-membered cyclic amino-C1∼C3 alkyl groups such as 1-pyrrolidinylethyl, piperidinoethyl, etc.

As the substituent(s) of the substituted alkenyl group, substituted alkynyl group or substituted alkanoyl group, there are exemplified the same groups as those described above as the substituent(s) of the substituted alkyl group. The substituent(s) of each of these substituted groups may also be present in a number of one or more and may be the same or different.

As the aralkyl group, alkyl groups substituted by a phenyl group or a polycyclic hydrocarbon ring group are exemplified.

As the heteroatom(s) of the saturated 3- to 8-membered cyclic amino group which may contain other heteroatom(s) in the ring and which R¹⁰ and R¹¹; R¹⁵ and R¹⁶; R¹⁷ and R¹⁸; or R³⁵ and R³⁶ form when taken together with the nitrogen atom to which they are bonded, there are exemplified oxygen atom, nitrogen atom and sulfur atom. Specific examples of such cyclic amino group are 3- to 8-membered cyclic groups containing 1 to 3 nitrogen atoms and 3- to 8-membered cyclic groups containing a nitrogen atom and an oxygen atom. More specific examples thereof are 1-pyrrolidinyl, 1-piperidino, 1-piperazinyl, morpholino and 1-(4-methyl)piperazinyl.

As the group represented by the formula: -S(O)₂R¹², -S(O)₂R¹³ or -S(O)₂R^{13a}, there are exemplified alkylsulfonyl groups of 8 or less carbon atoms, such as methylsulfonyl group, ethylsulfonyl group, propylsulfonyl group, isopropylsulfonyl group, etc. As the group represented by the formula: -S(O)ₘR¹², there are exemplified the above-exemplified groups and corresponding alkylsulfinyl and alkylthio groups, and sulfo group.

The substituent(s) of the lower alkylene group, alkenylene group or alkynylene group may be present in a number of one or more and may be the same or different. The substituent(s) includes alkyl groups, aralkyl groups and the same groups as those described as the substituent(s) of the substituted alkyl group.

The lower alkylene group includes, for example, linear or branched alkylene groups of 10 or less carbon atoms, such as methylene, ethylene, trimethylene, tetramethylene, pentamethylene, hexamethylene, 2-methyltrimethylene, etc.

As the alkoxycarbonyl group, there are exemplified groups formed by bonding the oxygen atom side of a group represented by the formula: -C(=O)O- to the binding site of an alkyl group.

As the group represented by the formula: 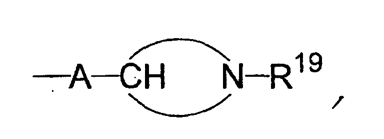 there are exemplified groups represented by

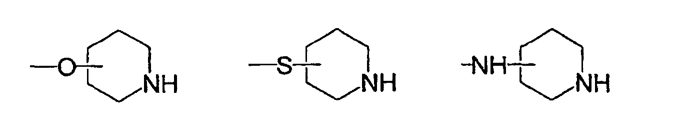

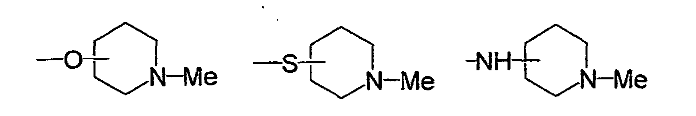

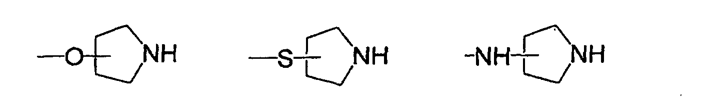

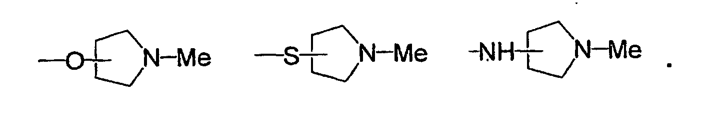

As the "prodrug", there are exemplified those which are easily hydrolyzed in a living body to regenerate the compound of formula (1). For example, when the compound of formula (1) has a carboxyl group, examples of the prodrug are compounds obtained by converting the carboxyl group to an alkoxycarbonyl group, an alkylthiocarbonyl group or an alkylaminocarbonyl group.

For example, when the compound of formula (1) has an amino group, examples of the prodrug are compounds obtained by converting the amino group to an alkanoylamino group by substitution by the alkanoyl group, compounds obtained by converting the amino group to an alkoxycarbonylamino group by substitution by the alkoxycarbonyl group, and compounds obtained by converting the amino group to an acyloxymethylamino group or hydroxylamine.

For example, when the compound of formula (1) has a hydroxyl group, examples of the prodrug are compounds obtained by converting the hydroxyl group to an acyloxy group by substitution by the above-exemplified acyl group, and compounds obtained by converting the hydroxyl group to a phosphoric ester or an acyloxymethyloxy group.

For example, when the compound of formula (1) has a sulfo group, examples of the prodrug are compounds obtained by converting the sulfo group to a sulfonic ester by substitution by an alkyl group.

For example, when the compound of formula (1) has a phosphono group, examples of the prodrug are compounds obtained by converting the phosphono group to a phosphonic monoester or a phosphonic diester by substitution by one or two alkyl groups.

Examples of the alkyl portion of the group used for such conversion to the prodrug are the above-exemplified alkyl groups. The alkyl groups as the alkyl portion may be substituted by, for example, an alkoxy group of 1 to 6 carbon atoms. Preferable examples of the alkyl portion are as follows.
(a) For example, in the case of the compounds obtained by converting the carboxyl group to an alkoxycarbonyl group, the alkoxycarbonyl group includes lower (number of carbon atoms: for example, 1 to 6) alkoxycarbonyl groups such as methoxycarbonyl, ethoxycarbonyl, etc.; and lower (number of carbon atoms: for example, 1 to 6) alkoxycarbonyl groups substituted by an alkoxy group, such as methoxymethoxycarbonyl, ethoxymethoxycarbonyl, 2-methoxyethoxycarbonyl, 2-methoxyethoxymethoxycarbonyl, pivaloyloxymethoxycarbonyl, etc.
(b) For example, in the case of the compounds obtained by converting the sulfo group to an alkoxysulfonyl group, the alkoxysulfonyl group includes lower (number of carbon atoms: for example, 1 to 6) alkoxysulfonyl groups such as methoxysulfonyl, ethoxysulfonyl, etc.; and lower (number of carbon atoms: for example, 1 to 6) alkoxysulfonyl groups substituted by an alkoxy group, such as methoxymethoxysulfonyl, ethoxymethoxysulfonyl, 2-methoxyethoxysulfonyl, 2-methoxyethoxymethoxysulfonyl, pivaloyloxymethoxysulfonyl, etc.
(c) For example, in the case of the compounds obtained by converting the phosphono group to an alkoxyphosphoryl group, the alkoxyphosphoryl group includes lower (number of carbon atoms: for example, 1 to 6) (mono- or di-)alkoxyphosphoryl groups such as methoxy(hydroxy)phosphoryl, ethoxy(hydroxy)phosphoryl, dimethoxyphosphoryl, diethoxyphosphoryl, etc.; lower (number of carbon atoms: for example, 1 to 6) (mono- or di-)alkoxyphosphoryl groups substituted by an alkoxy group(s), such as methoxymethoxy(hydroxy)phosphoryl, ethoxymethoxy(hydroxy)phosphoryl, 2-methoxyethoxy(hydroxy)phosphoryl, 2-methoxyethoxymethoxy(hydroxy)phosphoryl, pivaloyloxymethoxy(hydroxy)phosphoryl, bis(methoxymethoxy)phosphoryl, bis(ethoxymethoxy)phosphoryl, bis(2-methoxyethoxy)phosphoryl, bis(pivaloyloxymethoxy)phosphoryl, etc.

If necessary, the compound represented by formula (1) or the prodrug thereof may be converted to a pharmaceutically acceptable salt. As such a salt, there are exemplified salts with mineral acids such as hydrochloric acid, hydrobromic acid, sulfuric acid, phosphoric acid, etc.; salts with organic carboxylic acids such as formic acid, acetic acid, fumaric acid, maleic acid, oxalic acid, citric acid, malic acid, tartaric acid, aspartic acid, glutamic acid, etc.; salts with sulfonic acids such as methanesulfonic acid, benzenesulfonic acid, p-toluenesulfonic acid, hydroxybenzenesulfonic acid, dihydroxybenzenesulfonic acid, etc.; and
alkali metal salts such as sodium salt, potassium salt, etc.; alkaline earth metal salts such as calcium salt, magnesium salt, etc.; ammonium salt; and triethylamine salt, pyridine salt, picoline salt, ethanolamine salt, dicyclohexylamine salt, and salt with N,N'-dibenzylethylenediamine, etc.

Each of the compounds represented by formula (1), the prodrugs thereof and the pharmaceutically acceptable salts of the compounds or prodrugs may be in the form of an anhydride, hydrate or solvate.

When used as a pharmaceutical composition, the compounds of the present invention may be orally or parenterally administered. That is, the compounds may be orally administered in a usual dosage form such as powder, granules, tablets, capsules, syrup, suspension or the like, or the compound may be parenterally administered, for example, by injection of a solution, emulsion or suspension prepared from the compound. The compounds may be administered rectally in the form of a suppository. The compounds of the present invention may be formulated into the above-exemplified suitable dosage form by blending the compound with conventional acceptable adjuvants such as a carrier, excipient, binder, stabilizer, diluent, etc. When the compound is used in the form of an injection, the injection may contain acceptable additives such as a buffer, solubilizer, tonicity agent, etc. Although the dose and the number of administrations are varied depending on, for example, a disease to be cured, the condition of the disease, age, body weight and administration route, the compound may be administered to an adult in a dose of usually 0.1 to 2,000 mg, preferably 1 to 200 mg per day in one portion or several portions (for example, 2 to 4 portions).

The compound represented by formula (1) may be synthesized from a well-known compound by a combination of well-known synthesis processes. The compound may be synthesized, for example, by any of the processes described in the working examples described hereinafter or any of the following processes.
(A) A compound of formula (1) in which X is a group represented by the formula: -X¹-C(=O)NH- (wherein X¹ represents a portion other than the portion shown as a specific group like the formula: -C(=O)NH- in this case in the aforesaid group X, and Z is as defined above) may be synthesized, for example, as follows. 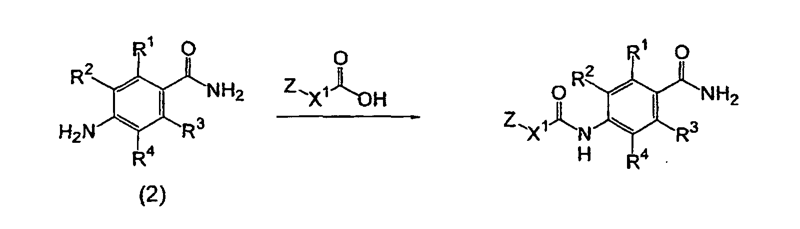 wherein Z, R¹, R², R³, R⁴ and X¹ are as defined above.
   The compound of formula (1) in which X is a group represented by the formula: -X¹-C(=O)NH- may be produced, for example, by reacting a compound represented by formula (2) with a compound represented by the formula: ZCOOH in an inert solvent in the presence of a condensing agent at room temperature or with heating, or by reacting a compound represented by formula (2) with, for example, a corresponding acid halide or acid anhydride in an inert solvent in the presence of a base at room temperature or with heating.
   As the condensing agent, there are used condensing agents such as dicyclohexylcarbodiimide (DCC), diisopropylcarbodiimide (DIPC), 1-ethyl-3-(3-dimethylaminopropyl)-carbodiimide (WSC), benzotriazol-1-yl-tris(dimethylamino)phosphonium·hexafluorophosphate (BOP), diphenylphosphonyldiamide (DPPA), N,N-carbonyldiimidazole (Angew. Chem. Int. Ed. Engl., Vol. 1, 351(1962)), etc. If necessary, there may be added additives such as N-hydroxysuccinimide (HOSu), 1-hydroxybenzotriazole (HOBt), 3-hydroxy-4-oxo-3,4-dihydro-1,2,3-benzotriazine (HOOBt), etc. As the solvent, there are exemplified aromatic hydrocarbon solvents such as benzene, toluene, xylene, etc.; ether solvents such as tetrahydrofuran, 1,4-dioxane, etc.; halogenated hydrocarbon solvents such as dichloromethane, chloroform, 1,2-dichloroethane, etc.; amide solvents such as dimethylformamide, dimethylacetamide, etc.; basic solvents such as pyridine, etc.; and mixed solvents thereof. As the base, there are exemplified inorganic bases such as sodium hydroxide, potassium hydroxide, sodium carbonate, potassium carbonate, sodium hydrogencarbonate, etc.; and organic bases such as triethylamine, pyridine, etc. As the acid halide, acid chlorides and acid bromides are exemplified.
(B) A compound of formula (1) in which the group represented by the formula: Z-X- is a group represented by the formula: R^{b}-X¹-CH(R^{a})NH- (wherein R^{a} is a hydrogen atom or a substituted or unsubstituted alkyl group, R^{b} is a saturated or unsaturated monocyclic hydrocarbon ring group, a saturated or unsaturated polycyclic hydrocarbon ring group, a saturated or unsaturated monocyclic heterocyclic group, or a saturated or unsaturated polycyclic heterocyclic group (these groups may be unsubstituted or may have a substituent(s)), and X¹ is as defined above; or X¹ is a single bond, and R^{a} and R^{b} are taken together with the nitrogen atom to which they are bonded, to represent a saturated or unsaturated monocyclic hydrocarbon ring group, a saturated or unsaturated polycyclic hydrocarbon ring group, a saturated or unsaturated monocyclic heterocyclic group, or a saturated or unsaturated polycyclic heterocyclic group (these groups may be unsubstituted or may have a substituent(s))) may be synthesized, for example, as follows. 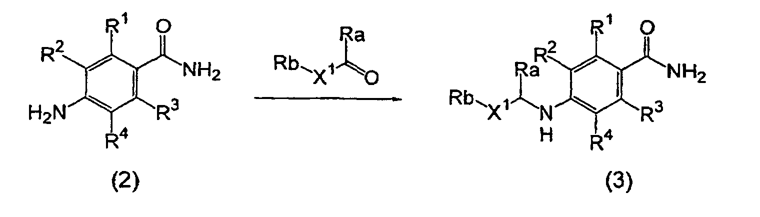 wherein R^{a}, R^{b}, X¹, R¹, R², R³ and R⁴ are as defined above.
   A compound represented by formula (3), i.e., the compound of formula (1) in which X is a group represented by the formula: R^{b}-X¹-CH(R^{a})NH- may be produced, for example, by subjecting a compound represented by formula (2) to reductive amination reaction with a compound represented by the formula: R^{a}-X¹-C(=O)R^{b} in an inert solvent in the presence of a reducing agent at room temperature or with heating.
   As the reducing agent, there may be used reducing agents including composite hydrogen compounds such as sodium triacetoxyborohydride, lithium aluminum hydride, sodium borohydride, sodium cyanoborohydride, etc.; and diborane. There may also be employed reduction with sodium, sodium amalgam or zinc-acid, and electrical reduction using lead or platinum as a cathode. As the solvent, there are exemplified alcohol solvents such as methanol, ethanol, etc.; ether solvents such as tetrahydrofuran, 1,4-dioxane, etc.; halogenated hydrocarbon solvents such as dichloromethane, chloroform, 1,2-dichloroethane, etc.; and mixed solvents thereof.
(C) A compound of formula (1) in which X is a group represented by the formula: -X¹-O- (wherein -X¹ is as defined above) may be synthesized, for example, as follows. 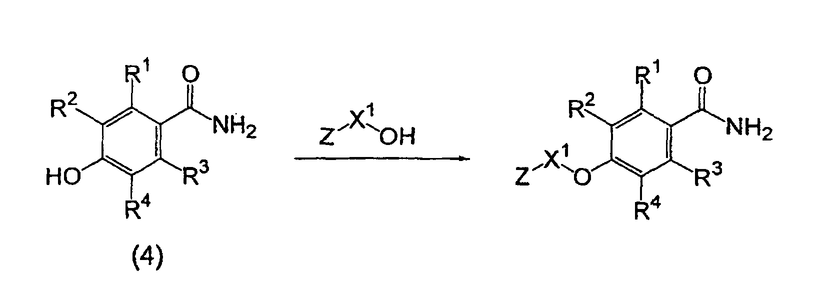 wherein Z, X¹, R¹, R², R³ and R⁴ are as defined above.
   The compound of formula (1) in which X is a group represented by the formula: -X¹-O- may be produced, for example, by reacting a compound represented by formula (4) with a compound represented by the formula: Z-X¹-OH in an inert solvent in the presence of, for example, triphenyl-phosphine and diethyl azodicarboxylate at room temperature or with heating. Like diethyl azodicarboxylate, there may also be used, for example, dialkyl azodicarboxylates such as diisopropyl azodicarboxylate, etc., and diaralkyl azodicarboxylates such as dibenzyl azodicarboxylate, etc.
(D) A compound of formula (1) in which the group represented by the formula: Z-X- is a group represented by the formula: R^{b}-X¹-CH(R^{a}) N (CH₂R^{c})-(wherein R^{a}, R^{b} and X¹ are as defined above, and R^{c} is a hydrogen atom, a substituted or unsubstituted alkyl group, a saturated or unsaturated monocyclic hydrocarbon ring group, a saturated or unsaturated polycyclic hydrocarbon ring group, a saturated or unsaturated monocyclic heterocyclic group, or a saturated or unsaturated polycyclic heterocyclic group (these groups may be unsubstituted or may have a substituent(s))) may be produced, for example, as follows. 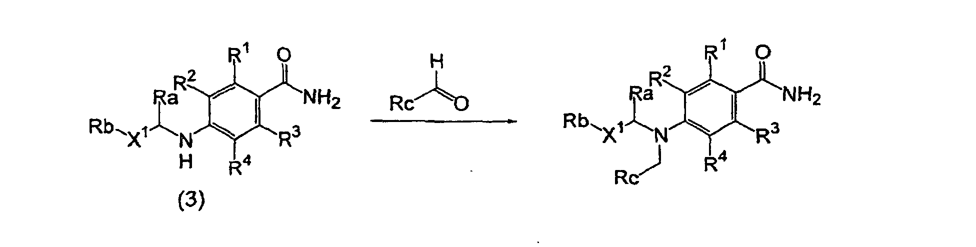 wherein R^{a}, R^{b}, R^{c}, X¹, R¹, R², R³ and R⁴ are as defined above.
   The compound of formula (1) in which the group represented by the formula: Z-X- is a group represented by the formula: R^{b}-X¹-CH(R^{a})N(CH₂R^{c})- may be produced, for example, by subjecting a compound represented by formula (3) to reductive amination reaction with a compound represented by the formula: R^{c}C(=O)H in an inert solvent in the presence of a reducing agent at room temperature or with heating.
As the reducing agent, there may be used reducing agents including composite hydrogen compounds such as sodium triacetoxyborohydride, lithium aluminum hydride, sodium borohydride, sodium cyanoborohydride, etc.; and diborane. There may also be employed reduction with sodium, sodium amalgam or zinc-acid, and electrical reduction using lead or platinum as a cathode. As the solvent, there are exemplified alcohol solvents such as methanol, ethanol, etc.; ether solvents such as tetrahydrofuran, 1,4-dioxane, etc.; halogenated hydrocarbon solvents such as dichloromethane, chloroform, 1,2-dichloroethane, etc.; and mixed solvents thereof.
(E) A compound represented by formula (1) may be synthesized also in, for example, the following manner. 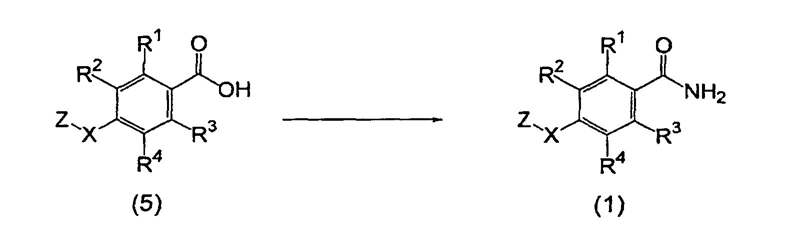 wherein R¹, R², R³, R⁴, X and Z are as defined above.

The compound represented by formula (1) may be synthesized, for example, by reacting a compound represented by formula (5) with an amine such as ammonium chloride in an inert solvent in the presence of a condensing agent at room temperature or with heating.

As the condensing agent, there are used condensing agents such as dicyclohexylcarbodiimide (DCC), diisopropylcarbodiimide (DIPC), 1-ethyl-3-(3-dimethylaminopropyl)-carbodiimide (WSC), benzotriazol-1-yl-tris(dimethylamino)phosphonium•hexafluorophosphate (BOP), diphenylphosphonyldiamide (DPPA), N,N-carbonyldiimidazole (Angew. Chem. Int. Ed. Engl., Vol. 1, 351(1962)), etc.. If necessary, there may be added additives such as N-hydroxysuccinimide (HOSu), 1-hydroxybenzotriazole (HOBt), 3-hydroxy-4-oxo-3,4-dihydro-1,2,3-benzotriazine (HOOBt), etc. As the solvent, there are exemplified aromatic hydrocarbon solvents such as benzene, toluene, xylene, etc.; ether solvents such as tetrahydrofuran, 1,4-dioxane, etc.; halogenated hydrocarbon solvents such as dichloromethane, chloroform, 1,2-dichloroethane, etc.; amide solvents such as dimethylformamide, dimethylacetamide, etc.; and mixed solvents thereof.

The compound represented by formula (1) may be synthesized also by, for example, reacting a corresponding benzonitrile compound (which refers to the same compound as the compound of formula (1) except that the amide group is replaced by a cyano group) as a starting compound in a sulfuric acid solvent with heating (for example, the process described in J. Med. Chem., Vol. 34, 281(1991)), or reacting the benzonitrile compound in an ethanol solvent with heating in the presence of potassium hydroxide (for example, the process described in J. Heterocycl. Chem., Vol. 27, 605(1990)).

In each of the above production processes, when the starting compound in the reaction has a reactive group such as hydroxyl group, amino group or carboxylic acid group, such a group at a site other than a site at which the reaction of the group is desirable is previously protected with a suitable protective group if necessary, and the protective group is removed after carrying out each reaction or several reactions, whereby a desired compound may be obtained. As the protective group for protecting the hydroxyl group, amino group, carboxyl group or the like, an ordinary protective group used in the field of organic synthetic chemistry may be used. The introduction and removal of such a protective group may be carried out according to a conventional method (for example, the method described in T.W. Greene and P.G.M. Wuts, Protective Groups in Organic Synthesis, Second Edition, John Wiley & Sons, Inc. (1991)).

For example, as a protective group for the hydroxyl group, methoxymethyl group, tetrahydropyranyl group and the like are exemplified. As a protective group for the amino group, tert-butoxycarbonyl group and the like are exemplified. The protective group for the hydroxyl group may be removed by reaction in a solvent such as aqueous methanol, aqueous ethanol or aqueous tetrahydrofuran in the presence of an acid such as hydrochloric acid, sulfuric acid or acetic acid. The protective group for the amino group may be removed by reaction in a solvent such as aqueous tetrahydrofuran, methylene chloride, chloroform or aqueous methanol in the presence of an acid such as hydrochloric acid or trifluoroacetic acid.

As a protective form for protecting the carboxyl group, there are exemplified tert-butyl esters, orthoesters, acid amides and the like. Such a protective group is removed as follows. In the case of the tert-butyl ester, the removal is carried out, for example, by reaction in an aqueous solvent in the presence of hydrochloric acid. In the case of the orthoester, the removal is carried out by treatment with an acid and then an alkali such as sodium hydroxide in a solvent such as aqueous methanol, aqueous tetrahydrofuran or aqueous 1,2-dimethoxyethane. In the case of the acid amide, the removal is carried out by reaction in a solvent such as water, aqueous methanol or aqueous tetrahydrofuran in the presence of an acid such as hydrochloric acid or sulfuric acid.

The compound represented by formula (1) includes those having an optical center of asymmetry. Therefore, the compound having an optical center of asymmetry may be obtained as a racemic modification, or it may be obtained as an optically active substance when an optically active starting material is used. If necessary, the racemic modification obtained may be physically or chemically resolved into optical antipodes by a well-known method. Preferably, diastereomers are formed from the racemic modification by a reaction using a reagent for optical resolution. The diastereomers different in form may be resolved by a well-known method such as fractional crystallization.

The present invention is more concretely illustrated below with working examples and test examples, which should not be construed as limiting the scope of the invention. The nomenclature of compounds shown in the working examples and test examples mentioned below is not always based on IUPAC.

### Example 1

### Synthesis of N-[4-(aminocarbonyl)phenyl]-3-nitrobenzamide

Thionyl chloride (1 ml) was added to a solution of 2-nitrobenzoic acid (245 mg, 1.46 mmol) in toluene (2 ml) at 0°C, and the resulting mixture was stirred at room temperature for 1 hour, at 50°C for 2 hours and then at 80°C for 2 hours. The solvent was distilled off under reduced pressure and a suspension of the resulting acid chloride in tetrahydrofuran (2 ml) was added dropwise to a suspension of p-aminobenzamide 1/4 hydrate (169 mg, 1.20 mmol) and triethylamine (0.33 ml, 2.4 mmol) in tetrahydrofuran (2 ml) at 0°C and stirred at room temperature for 1 hour. Then, water was added to the reaction mixture and the solid precipitated was filtered, and the precipitate on a filter was washed with methanol and then dried to obtain N-[4-(aminocarbonyl)phenyl]-3-nitrobenzamide (0.340 g, 99%).

MS : m/z = 286 (M + 1).

### Example 2

### Synthesis of N-[4-(aminocarbonyl)phenyl]-3-(dimethylamino)benzamide

The title compound was synthesized by carrying out reaction according to the method described in Example 1, except for using 3-dimethylaminobenzoic acid in place of 2-nitrobenzoic acid.
¹H-NMR (DMSO-d₆) δ ; 2.96 (6H, s), 6.92 (1H, br), 7.15-7.30 (3H, m), 8.32 (1H, dd, J=8.2, 8.2Hz), 7.80-7.92 (5H, m) , 10.31 (1H, s) .

### Example 3

### Synthesis of 3-amino-N-[4-(aminocarbonyl)-phenyl]benzamide hydrochloride

### (a) Synthesis of 3-amino-N-[4-(aminocarbonyl)phenyl]-benzamide

To a suspension of the N-[4-(aminocarbonyl)-phenyl]-3-nitrobenzamide obtained in Example 1 (325 mg, 1.14 mmol) in ethanol (5 ml) was added 10%-palladium/carbon (30 mg), and the resulting mixture was stirred for 4 hours under a hydrogen atmosphere at room temperature under atmospheric pressure to carry out catalytic reduction. The reaction mixture was dissolved in a tetrahydrofuran/methanol (2/1) mixture, followed by filtration using Celite. The solvent was distilled off under reduced pressure and the resulting residue was purified by a silica gel column chromatography (eluent: tetrahydrofuran/methanol/triethylamine = 100/10/1) to obtain 3-amino-N-[4-(aminocarbonyl)phenyl]benzamide (411 mg).
¹H-NMR (DMSO-d₆) δ ; 5.35 (2H, br) , 6.75-6. 78 (1H, m) , 7.05-7.11 (2H, m) , 7.16 (1H, t, J =7.8Hz), 7.25 (1H, br) , 7.79-7.94 (5H, m), 10.28 (1H, br).

### (b) Synthesis of 3-amino-N-[4-(aminocarbonyl)phenyl]-benzamide hydrochloride

To a solution of 3-amino-N-[4-(aminocarbonyl)phenyl]benzamide (411 mg) in a tetrahydrofuran (20 ml)/methanol (10 ml) mixture was added 4N-hydrochloric acid/1,4-dioxane (1 ml) at room temperature, and stirred, and the solid precipitated was collected by filtration and dried to obtain 3-amino-N-[4-(aminocarbonyl)phenyl]benzamide hydrochloride (294 mg, 89%).
¹H-NMR (DMSO-d₆) δ ; 7.28 (1H, br) , 7.42 (1H, dd, J=1.2, 7.9Hz), 7.56 (1H, t, J=7.9Hz), 7. 72-7.75 (1H, m) , 7.83 (1H, d, J=7.9Hz), 7.8 5 (2H, d, J=8.8Hz), 7.89 (2H, d, J=8.8Hz), 7. 91 (1H, br), 10.58 (1H, br).

### Example 4

### Synthesis of 4-[(3-nitrobenzyl)amino]-benzamide

To a solution of p-aminobenzamide 1/4 hydrate (489 mg, 3.47 mmol) in 1,2-dichloroethane (15 ml) were added 3-nitrobenzaldehyde (500 mg, 3.31 mmol), acetic acid (199 µl, 3.47 mmol) and sodium triacetoxyborohydride (1.12 g, 5.29 mmol), and stirred for 14 hours. Then, the reaction mixture was poured into a saturated aqueous sodium hydrogencarbonate solution and extracted with ethyl acetate. The organic layer was dried over magnesium sulfate and distilled under reduced pressure to remove the solvent, and the resulting residue was purified by a silica gel column chromatography (eluent: chloroform/methanol = 50/1) to obtain 4-[(3-nitrobenzyl)amino]benzamide (168 mg, 19%).

Melting point: 178-180°C.

### Example 5

### Synthesis of N-[4-(aminocarbonyl)phenyl]-3-(methylsulfonyl)benzamide

Thionyl chloride (729 µl, 8.03 mmol) was added to a solution of 3-methylsulfonylbenzoic acid (500 mg, 2.50 mmol) in toluene (5 ml) at room temperature and stirred at 80°C for 1 hour. Then, N,N-dimethylformamide (3 drops) was added thereto and the resulting mixture was stirred at 80°C for 1 hour. The solvent was distilled off under reduced pressure and the acid chloride thus obtained was added to a suspension of p-aminobenzamide 1/4 hydrate (351 mg, 2.50 mmol) and triethylamine (418 µl, 3.00 mmol) in methylene chloride (20 ml) at room temperature and stirred overnight. Then, the reaction mixture was poured into water and the precipitate obtained was collected by filtration and washed with ethyl acetate to obtain N-[4-(aminocarbonyl)phenyl]-3-(methylsulfonyl)benzamide (592 mg, 74%).
¹H-NMR (DMSO-d₆) δ ; 3.29 (3H, s) , 7.29 (1H, br s), 7.83 (1H, dd, J=7.9, 7.9Hz), 7.86 (2 H, d, J=7.5Hz), 7.88 (1H, d, J=7.5Hz) , 7.91 (1H, br s) , 8.16 (1H, d, J=7.9Hz) , 8.28 (1H, d, J=7.9Hz), 8.47 (1H, s), 10.71 (1H, s).

### Example 6

### Synthesis of 4-acetyl-N-[4-(aminocarbonyl)-phenyl]benzamide

The title compound was synthesized by carrying out reaction according to the method described in Example 1, except for using p-acetylbenzoic acid in place of 2-nitrobenzoic acid.

Melting point: 283-284°C (decomp.).

### Example 7

### Synthesis of N-[4-(aminocarbonyl)phenyl]-1-benzylpiperidine-4-carboxamide

### (a) Synthesis of ethyl 1-benzyl-4-piperidinecarboxylate

Benzyl chloride (13.2 ml, 115 mmol) and potassium carbonate (19.8 g, 143 mmol) were added to a solution of ethyl 4-piperidinecarboxylate (15.0 g, 95.4 mmol) in N,N-dimethylformamide (50 ml) at room temperature and stirred at 120°C for 1.5 hours. Then, the reaction suspension was filtered and the solvent of the filtrate was distilled off under reduced pressure, and the resulting residue was purified by a silica gel column chromatography (eluent: hexane/ethyl acetate = 4/1) to obtain ethyl 1-benzyl-4-piperidinecarboxylate (17.7 g, 75%).

### (b) Synthesis of 1-benzyl-4-piperidinecarboxylic acid

A 4N-aqueous sodium hydroxide solution (35 ml) was added to a solution of ethyl 1-benzyl-4-piperidinecarboxylate (16.1 g, 65.1 mmol) in a tetrahydrofuran (70 ml)/1,4-dioxane (70 ml) mixture at room temperature and stirred overnight. Then, the pH was adjusted to 7 with 2N-hydrochloric acid and the solvent was distilled off under reduced pressure. The resulting residue was suspended in ethanol and the suspension was filtered. The filtrate was concentrated under reduced pressure to obtain 1-benzyl-4-piperidinecarboxylic acid (13.6 g, 95%).

### (c) Synthesis of N-[4-(aminocarbonyl)phenyl]-1-benzylpiperidine-4-carboxamide

Thionyl chloride (7.3 ml, 100 mmol) was added to a solution of 1-benzyl-4-piperidinecarboxylic acid (2.19 g, 9.99 mmol) in toluene (17 ml) at room temperature, and the resulting mixture was stirred at room temperature for 20 minutes and then at 80°C for 3 hours. The solvent was distilled off under reduced pressure and a solution of the resulting acid chloride in methylene chloride (13 ml) was added dropwise to a suspension of p-aminobenzamide 1/4 hydrate (1.17 g, 8.32 mmol) and triethylamine (2.3 ml, 16.5 mmol) in methylene chloride (13 ml) at 0°C and stirred overnight at room temperature. Then, the reaction suspension was filtered and water was added to the solid thus obtained, and the pH was adjusted to 8 with a 1N-aqueous sodium hydroxide solution. The resulting suspension was filtered and the precipitate was dried to obtain N-[4-(aminocarbonyl)phenyl]-1-benzylpiperidine-4-carboxamide (1.05 g, 31%).
¹H-NMR (DMSO-d₆) δ : 1.62-1.72 (4H, m), 1.9 0-1. 97 (2H, m), 2.33 (1H, m) , 2.83-2.87 (2H, m), 3.45 (2H, s), 7.22-7.32 (6H, m) , 7.63-7. 65 (2H, m) , 7.75-7.81 (3H, m), 10.07 (1H, s).

### Example 8

### Synthesis of N-[4-(aminocarbonyl)phenyl]-piperidine-4-carboxamide

Ammonium formate (400 mg) and 10%-palladium/carbon (80 mg) were added to a suspension of the N-[4-(aminocarbonyl)phenyl]-1-benzylpiperidine-4-carboxamide obtained in Example 7 (400 mg, 1.19 mmol) in ethanol (20 ml), and the resulting mixture was refluxed for 5 hours. Then, ammonium formate (400 mg), 10% Pd-C (80 mg) and 1N-hydrochloric acid (several drops) were added thereto, and the resulting mixture was refluxed for 1.5 hours. The reaction mixture was filtered by the use of Celite and the filtrate was concentrated to obtain N-[4-(aminocarbonyl)-phenyl]piperidine-4-carboxamide (214 mg, 73%).
¹H-NMR (DMSO-d₆) δ ; 1.43-1.54 (2H, m), 1.6 4-1.68 (2H, m), 2.02 (1H, m) , 2.37-2.50 (2H, m), 2.93-2.98 (2H, m), 7.20 (1H, br s), 7.6 3-7.66 (2H, m), 7.74-7.81 (3H, m), 10.05 (1 H, s).

### Example 9

### Synthesis of N-[4-(aminocarbonyl)phenyl]-piperidine-4-carboxamide hydrochloride

The title compound was synthesized by carrying out reaction according to the method described in Example 3 (b), except for using the N-[4-(aminocarbonyl)phenyl]piperidine-4-carboxamide obtained in Example 8, as a starting material.
¹H-NMR (DMSO-d₆) δ ; 1.75-1.98 (4H, m), 2.6 3-2.71 (1H, m) , 2.85-2.96 (2H, m), 3.30-3. 33 (2H, m) , 7.23 (1H, br s), 7.66 (2H, d, J=8. 1Hz), 7.82 (2H, d, J=8.1Hz), 7.85 (1H, br s), 8.57 (1H, br s), 8.88 (1H, br s), 10.35 (1H, s).

### Example 10

### Synthesis of 4-[(1-benzylpiperidin-4-yl)amino]benzamide

To a solution of 1-benzyl-4-piperidone (1.89 g, 9.99 mmol) in 1,2-dichloroethane (40 ml) were added p-aminobenzamide (1.28 g, 9.10 mmol), sodium triacetoxyborohydride (3.28 g, 14.5 mmol) and acetic acid (0.6 ml, 10.5 mmol) at room temperature, and stirred overnight. After completion of the reaction, a 1N-aqueous sodium hydroxide solution was added thereto and the resulting mixture was washed and then extracted with ethyl acetate. The organic layer was washed with a saturated aqueous sodium chloride solution and dried over magnesium sulfate. The solvent was distilled off under reduced pressure and the resulting residue was suspended in ethyl acetate (26 ml). The suspension was filtered and the solid thus obtained was purified by a silica gel column chromatography (eluent: ethyl acetate/methanol = 100/1) to obtain 4-[(1-benzylpiperidin-4-yl)amino]benzamide (0.84 g, 27%).

Melting point: 192-193°C.

### Example 11

### Synthesis of 4-[(1-benzylpiperidin-4-yl)amino]benzamide hydrochloride

To a solution of the 4-[(1-benzylpiperidin-4-yl)amino]benzamide obtained in Example 10 (309 mg, 0.999 mmol) in tetrahydrofuran (12.5 ml) was added a 1N-hydrochloric acid/ether solution (2.5 ml, 2.50 mmol) at room temperature, and stirred for 30 minutes. The resulting suspension was filtered and the resulting precipitate was recrystallized from a methanol (6 ml)/water (7 drops) mixed solvent to obtain 4-[(1-benzylpiperidin-4-yl)amino]benzamide hydrochloride (117 mg, 30%).
¹H-NMR (DMSO-d₆) δ ; 1.77-1.91 (2H, m), 2.0 5-2.09 (2H, m) , 2.94-3.75 (5H, m) , 4.25-4. 29 (2H, s) , 6.58-6.67 (1H, m), 7.44-7.46 (3 H, m), 7.62-7.67 (4H, m), 10.98 (1H, m).

### Example 12

### Synthesis of 4-(piperidin-4-ylamino)benzamide hydrochloride

Ammonium formate (400 mg) and 10%-palladium/carbon (80 mg) were added to a solution of the 4-[(1-benzylpiperidin-4-yl)amino]benzamide obtained in Example 10 (400 mg, 1.19 mmol) in ethanol (15 ml) at room temperature, and the resulting mixture was refluxed for 9 hours. After completion of the reaction, the reaction mixture was filtered by the use of Celite and the filtrate was concentrated. To a solution of the resulting residue in ethanol (6 ml) was added a 1N-hydrochloric acid/ether solution (3 ml, 3.00 mmol) at room temperature, and stirred for 30 minutes. The resulting suspension was filtered and the resulting precipitate was recrystallized from a methanol (5 ml)/water (0.5 ml) mixed solvent to obtain 4-(piperidin-4-ylamino)benzamide hydrochloride (143 mg, 37%).

Melting point: 292-294°C (decomp.).

### Example 13

### Synthesis of N-[4-(aminocarbonyl)phenyl]-1-benzylpiperidine-3-carboxamide

### (a) Synthesis of ethyl 1-benzyl-3-piperidinecarboxylate

The title compound was synthesized by carrying out reaction according to the method described in Example 7 (a), except for using ethyl 3-piperidinecarboxylate as a starting material.

### (b) Synthesis of 1-benzyl-3-piperidinecarboxylic acid

The title compound was synthesized by carrying out reaction according to the method described in Example 7 (b), except for using ethyl 1-benzyl-3-piperidinecarboxylate as a starting material.

### (c) Synthesis of N-[4-(aminocarbonyl)phenyl]-1-benzylpiperidine-3-carboxamide

To a solution of 1-benzyl-3-piperidinecarboxylic acid (1.37 g, 6.23 mmol) in N,N-dimethylformamide (28 ml) were added p-aminobenzamide (0.92 g, 6.54 mmol), triethylamine (1.3 ml, 9.33 mmol), 1-ethyl-3-(3-dimethylaminopropyl)carbodiimide hydrochloride (1.79 g, 9.34 mmol) and 1-hydroxybenzotriazole monohydrate (0.93 mg, 6.88 mmol) at room temperature, and stirred overnight. Then, the reaction mixture was poured into a saturated aqueous sodium hydrogencarbonate solution and extracted with chloroform. The organic layer was washed with a saturated aqueous sodium chloride solution and then dried over magnesium sulfate. The solvent was distilled off under reduced pressure and the resulting residue was purified by a silica gel column chromatography (eluent: chloroform/methanol = 95/5) to obtain N-[4-(aminocarbonyl)phenyl]-1-benzylpiperidine-3-carboxamide (551 mg, 26%).

Melting point: 211-212°C.

### Example 14

### Synthesis of N-[4-(aminocarbonyl)phenyl]-piperidine-3-carboxamide

Ammonium formate (400 mg) and 10%-palladium/carbon (80 mg) were added to a solution of the N-[4-(aminocarbonyl)phenyl]-1-benzylpiperidine-3-carboxamide obtained in Example 13 (400 mg, 1.19 mmol) in ethanol (15 ml), and the resulting mixture was refluxed for 4 hours. After completion of the reaction, the reaction mixture was filtered by the use of Celite and the filtrate was concentrated to obtain N-[4-(aminocarbonyl)phenyl]piperidine-3-carboxamide (282 mg, 96%).
¹H-NMR (DMSO-d₆) δ ; 1.33-1.37 (2H, m), 1.5 0-1.61 (2H, m), 1.84 (1H, m), 2.39-2.43 (1H, m) , 2.55-2.62 (1H, m), 2.81-2.85 (2H, m), 2. 98-3.02 (2H, m), 7.20 (1H, br s), 7.63 (2H, d, J=8.6Hz), 7.79 (3H, m), 10.14 (1H, s).

### Example 15

Synthesis of 4-{[(4-aminocyclohexyl)-carbonyl]amino}benzamide

### (a) Synthesis of 4-[(tert-butoxycarbonyl)amino]-cyclohexanecarboxylic acid

A solution of sodium hydroxide (1.25 g, 31.3 mmol) in water (23 ml) and di-tert-butyl dicarbonate (6.82 g, 31.2 mmol) were added to a suspension of 4-aminocyclohexanecarboxylic acid (4.30 g, 30.0 mmol) in tert-butanol (53 ml) at room temperature and stirred overnight. After completion of the reaction, water (90 ml) was poured into the reaction mixture, followed by extraction with hexane. The aqueous layer was neutralized with 2N-hydrochloric acid under ice-cooling and extracted with ethyl acetate. The organic layer was washed with a saturated aqueous sodium chloride solution, dried over magnesium sulfate, and then distilled under reduced pressure to remove the solvent, whereby 4-[(tert-butoxycarbonyl)amino]cyclohexanecarboxylic acid (6.58 g, 90%) was obtained.

### (b) Synthesis of tert-butyl 4-[{[4-(aminocarbonyl)-phenyl]amino}carbonyl]cyclohexylcarbamate

To a solution of 4-[(tert-butoxycarbonyl)-amino]cyclohexanecarboxylic acid (1.46 g, 6.23 mmol) in N,N-dimethylformamide (28 ml) were added p-aminobenzamide (0.92 g, 6.54 mmol), triethylamine (1.3 ml, 9.33 mmol), 1-ethyl-3-(3-dimethylaminopropyl)-carbodiimide hydrochloride (1.79 g, 9.34 mmol) and 1-hydroxybenzotriazole monohydrate (0.93 mg, 6.88 mmol) at room temperature, and stirred overnight. After a saturated aqueous sodium hydrogencarbonate solution was added to the reaction mixture, the resulting mixture was filtered. The resulting solid was suspended in water and the suspension was filtered, and the solid thus obtained was dried to obtain tert-butyl 4-[{[4-(aminocarbonyl)phenyl]amino}carbonyl]-cyclohexylcarbamate (1.11 g, 49%, a cis/trans mixture).

### (c) Synthesis of 4-{[(4-aminocyclohexyl)carbonyl]-amino}benzamide

Trifluoroacetic acid (3.4 ml) was added to a suspension of tert-butyl 4-[{[4-(aminocarbonyl)phenyl]-amino}carbonyl]cyclohexylcarbamate (290 mg, 0.802 mmol) in methylene chloride (5 ml) at room temperature and stirred overnight. After completion of the reaction, the solvent was distilled off under-reduced pressure and the resulting residue was dissolved in water, and the resulting solution was neutralized with a saturated aqueous sodium hydrogencarbonate solution. A precipitate obtained by concentrating the neutralized solution was collected by filtration and dried to obtain 4-{[(4-aminocyclohexyl)carbonyl]amino}benzamide (29 mg, yield: 14%).
¹H-NMR (DMSO-d₆) δ ; 1.01-2.36(m) , 6.54(1 H, m), 7.20 (1H, m) , 7.63-7.95(2H, m), 7.79 (1H, d, J=8.6Hz) , 9.95-10.03 (1H, m).

### Example 16

### Synthesis of trans-4-[{[4-(aminomethyl)-cyclohexyl]carbonyl}amino]benzamide

### (a) Synthesis of trans-4-{[(tert-butoxycarbonyl)-amino]methyl}cyclohexanecarboxylic acid

The title compound was synthesized by carrying out reaction according to the method described in Example 15 (a), except for using trans-4-(aminomethyl)cyclohexanecarboxylic acid as a starting material.

### (b) Synthesis of trans-tert-butyl (4-[{[4-(aminocarbonyl)phenyl]amino}carbonyl]cyclohexyl)-methylcarbamate

The title compound was synthesized by carrying out reaction according to the method described in Example 15 (b), except for using trans-4-{[(tert-butoxycarbonyl)amino]methyl}cyclohexanecarboxylic acid as a starting material.

### (c) Synthesis of trans-4-[{[4-(aminomethyl)-cyclohexyl]carbonyl}amino]benzamide

The title compound was synthesized by carrying out reaction according to the method described in Example 15 (c), except for using trans-tert-butyl (4-[{[4-(aminocarbonyl)phenyl]amino}carbonyl]-cyclohexyl)methylcarbamate as a starting material.
¹H-NMR (DMSO-d₆) δ ; 0.82-0.95(2H, m) , 1.2 0-1.42 (3H, m) , 1.74-1.85 (4H, m) , 2.16-2. 40 (2H, m), 2.77-2.81(1H, m), 7.19(1H, m), 7. 64 (1H, d, J=8.8Hz) , 7.79(1H, d, J=8.8H z) , 7.80 (1H, m), 10.01(1H, s).

### Example 17

Synthesis of 4-[(piperidin-4-ylmethyl)amino]-benzamide

### (a) Synthesis of 1-benzylpiperidine-4-carbaldehyde

To a solution of diisopropylamine (14.8 ml, 106 mmol) in tetrahydrofuran (20 ml) was added dropwise 1.59M-n-butyllithium (7.9 ml, 12.7 mmol) at -78°C over a period of 10 minutes, and stirred for 30 minutes. Then, 2M-trimethylsilyldiazomethane (6.34 ml, 12.7 mmol) was added dropwise thereto at -78°C over a period of 5 minutes and stirred for 30 minutes. Thereafter, a solution of 1-benzyl-4-piperidone (2.0 g, 10.6 mmol) in tetrahydrofuran (20 ml) was added dropwise thereto at -78°C over a period of 30 minutes, and the resulting mixture was stirred at -78°C for 1 hour, warmed up to room temperature, stirred for 30 minutes and then refluxed for 2 hours. After completion of the reaction, the reaction mixture was poured onto ice and extracted with ethyl acetate. The organic layer was dried over magnesium sulfate and distilled under reduced pressure to remove the solvent, and to a solution of the resulting residue in ethyl acetate (200 ml) was added silica gel (10 g) at room temperature, and stirred overnight. Then, the solvent was distilled off and the resulting residue was purified by a silica gel column chromatography (eluent: chloroform/methanol = 50/1) to obtain 1-benzylpiperidine-4-carbaldehyde (1.19 g, 55%).

### (b) Synthesis of 4-{[(1-benzylpiperidin-4-yl)methyl]-amino}benzamide

The title compound was synthesized by carrying out reaction according to the method described in Example 10, except for using 1-benzylpiperidine-4-carbaldehyde in place of 1-benzyl-4-piperidone.
¹H-NMR (DMSO-d₆) δ ; 1.16-1.2 4 (2H, m) , 1.5 2 (1H, m), 1.67-1.72 (2H, m), 1.83-1.91(2H, m), 2.77-2.81(2H, m), 2.90-2.94(2H, m), 3. 42 (2H, s), 6.17 (1H, m), 6.51(2H, d, J=8.6H z), 6.80 (1H, m), 7.22-7.32(5H, m), 7.48(1 H, m) , 7.60(2H, d, J=8.6Hz).

### (c) Synthesis of 4-[(piperidin-4-ylmethyl)amino]-benzamide

The title compound was synthesized by carrying out reaction according to the method described in Example 14, except for using 4-{[(1-benzylpiperidin-4-yl)methyl]amino}benzamide as a starting material.
¹H-NMR (DMSO-d₆) δ ; 0.96-1.08 (2H, m), 1.5 8-1.66 (3H, m), 2.35-2.42 (2H, m), 2.87-2. 91(4H, m), 6.17(1H, m), 6.51 (2H, d, J=8.6H 2), 6.80 (1H, m), 7.49(1H, m), 7.60 (2H, d, J =8.6Hz).

### Example 18

### Synthesis of 4-[(1-benzylpiperidin-3-yl)amino]benzamide

The title compound was synthesized by carrying out reaction according to the method described in Example 10, except for using 1-benzyl-3-piperidone hydrochloride monohydrate in place of 1-benzyl-4-piperidone.

Melting point: 158-160°C.

### Example 19

### Synthesis of 4-(piperidin-3-ylamino)benzamide

The title compound was synthesized by carrying out reaction according to the method described in Example 14, except for using the 4-[(1-benzylpiperidin-3-yl)amino]benzamide obtained in Example 18, as a starting material.
¹H-NMR (DMSO-d₆) δ ; 1.30-1.42 (2H, m), 1.5 8 (1H, m), 1.89 (1H, m) , 2.09 (1H, m), 2.21-2. 28 (1H, m) , 2.39-2.45(1H, m) , 2.75-2.79 (1 H, m) , 3.00-3.04 (1H, m), 3.41 (1H, m), 5.94 (1H, d, J=8.6Hz), 6.53 (2H, d, J=8.6Hz), 6. 80(1H, m), 7.49(1H, m), 7.60 (2H, d, J=8.6H z).

### Example 20

### Synthesis of 4-[(1-benzylpyrrolidin-3-yl)amino]benzamide

The title compound was synthesized by carrying out reaction according to the method described in Example 10, except for using 1-benzyl-3-pyrrolidone in place of 1-benzyl-4-piperidone.

Melting point: 160-162°C.

### Example 21

### Synthesis of 4-(pyrrolidin-3-ylamino)-benzamide

The title compound was synthesized by carrying out reaction according to the method described in Example 14, except for using the 4-[(1-benzylpyrrolidin-3-yl)amino]benzamide obtained in Example 20, as a starting material.
¹H-NMR (DMSO-d₆) δ;1.50-1.56 (2H, m), 1.9 2-2.03 (1H, m), 2.54-2.60 (1H, m), 2.69-2. 89 (2H, m) , 2.96-3.01 (1H, m) , 3.78 (1H, m), 6.16 (1H, d, J=6.8Hz), 6.51 (2H, d, J=8.8H z), 6.83 (1H, m), 7.52 (1H, m), 7.61 (2H, d, J =8.8Hz).

### Example 22

### Synthesis of 4-[(4-aminocyclohexyl)amino]-benzamide trifluoroacetate

### (a) Synthesis of tert-butyl 4-hydroxycyclohexylcarbamate

An aqueous solution (52.5 ml) of sodium hydroxide (2.91 g, 72.8 mmol) was added to a suspension of trans-4-aminocyclohexanol (8.06 g, 70.0 mmol) in t-butanol (122.5 ml) at room temperature, followed by adding thereto di-t-butyl dicarbonate (15.9 g, 72.9 mmol), and the resulting mixture was stirred overnight at room temperature. Water was added to the reaction mixture, followed by extraction with n-hexane. The organic layer obtained as a suspension was filtered and the precipitate was dried to obtain tert-butyl 4-hydroxycyclohexylcarbamate (2.70 g) as a white solid. On the other hand, the aqueous layer was neutralized with 1N-hydrochloric acid and then extracted with ethyl acetate. The organic layer was washed with a saturated aqueous sodium chloride solution and dried over magnesium sulfate. The solvent was distilled off under reduced pressure and the resulting residue was purified by a silica gel column chromatography (eluent: chloroform/ethyl acetate) to obtain tert-butyl 4-hydroxycyclohexylcarbamate (11.3 g) (14.0 g in total, 93%).
¹H-NMR (DMSO-d₆) δ;1.06-1.20 (4H, m), 1.3 5 (9H, s), 1.69-1.76 (4H, m) , 3.12-3.31 (2H, m), 4.48 (1H, s), 6.64 (1H, d, J=7.5Hz).

### (b) Synthesis of tert-butyl 4-oxocyclohexylcarbamate

A solution of dimethyl sulfoxide (2.0 ml, 28.2 mmol) in methylene chloride (6 ml) was added dropwise to a solution of oxalyl chloride (1.7 ml, 19.5 mmol) in methylene chloride (30 ml) at -60°C over a period of 10 minutes and stirred at -60°C for another 10 minutes. Then, a solution of tert-butyl 4-hydroxycyclohexylcarbamate (2.56 g, 11.9 mmol) in methylene chloride (140 ml) was added dropwise thereto over a period of 35 minutes, and the resulting mixture was stirred at -60°C for 40 minutes. After triethylamine (8.4 ml, 60.3 mmol) was added thereto at -60°C, the resulting mixture got warmed to room temperature spontaneously. Water was added to the reaction mixture, followed by extraction with chloroform. The organic layer was washed with a saturated aqueous ammonium chloride solution and a saturated aqueous sodium chloride solution and then dried over magnesium sulfate. The solvent was distilled off under reduced pressure and the resulting residue was purified by a silica gel column chromatography (eluent: chloroform/ethyl acetate) to obtain tert-butyl 4-oxocyclohexylcarbamate (2.23 g, 88%).
¹H-NMR (DMSO-d₆) δ ; 1.06-1.20(4H, m), 1.3 5 (9H, s), 1.69-1.76 (4H, m) , 3.12-3.31 (2H, m), 4.48 (1H, s), 6.64 (1H, d, J=7.5Hz).

### (c) Synthesis of tert-butyl 4-([4-(aminocarbonyl)-phenyl]amino}cyclohexylcarbamate

The title compound was synthesized by carrying out reaction according to the method described in Example 10, except for using tert-butyl 4-oxocyclohexylcarbamate in place of 1-benzyl-4-piperidone.
¹H-NMR (DMSO-d₆) δ ; 1.14-1.29 (1H, m), 1.3 7 (s, 9H, Boc), 1.57-1.60 (1H, m) , 1.76 (1H, m), 1.92-1.98 (1H, m), 3.18 (1H, m), 3.36 (1 H, m) , 5.89-5.86 (1H, m) , 6.50-6.54 (2H, d, J=8.8Hz), 6.76-6.79(1H, m), 7.50 (1H, m), 7.59-7.60(2H, d, J=8.8Hz).

### (d) Synthesis of 4-[(4-aminocyclohexyl)amino]benzamide trifluoroacetate

Trifluoroacetic acid (2.2 ml) was added to a suspension of tert-butyl 4-{[4-(aminocarbonyl)phenyl]-amino}cyclohexylcarbamate (167 mg, 0.501 mmol) in methylene chloride (4 ml), and the resulting mixture was stirred overnight at room temperature. After completion of the reaction, the reaction mixture was concentrated to obtain 4-[(4-aminocyclohexyl)amino]-benzamide trifluoroacetate (230 mg, 100%).
¹H-NMR (DMSO-d₆) δ : 1.15-1.45 (2H, m), 1.6 3-1.71 (3H, m), (1H, m), 3.21 - 3.30 (1H, m) , 6.54-6.57 (2H, d, J = 8.7Hz), 6.84 (1H, m), 7. 50 (1H, m), 7.61-7.62 (2H, d, J=8.6Hz).

### Example 23

### Synthesis of 4-(tetrahydro-2H-pyran-4-ylamino)benzamide

The title compound was synthesized by carrying out reaction according to the method described in Example 10, except for using tetrahydro-4H-pyran-4-one in place of 1-benzyl-4-piperidone.

Melting point: 208-210°C.

### Example 24

### Synthesis of 4-(piperidin-4-yloxy)benzamide trifluoroacetate

### (a) Synthesis of tert-butyl 4-[4-(aminocarbonyl)-phenoxy]piperidine-1-carboxylate

To a solution of p-hydroxybenzamide (343 mg, 2.50 mmol) in tetrahydrofuran (10 ml) were added tert-butyl 4-hydroxy-1-piperidinecarboxylate (503 mg, 2.50 mmol) and triphenylphosphine (656 mg, 2.50 mmol), followed by adding thereto ethyl azodicarboxylate (1.15 ml, 2.50 mmol) under ice-cooling, and the resulting mixture was stirred overnight at room temperature. Then, the reaction mixture was concentrated and the crude product thus obtained was purified by a silica gel column chromatography (chloroform/methanol = 99/1) to obtain tert-butyl 4-[4-(aminocarbonyl)phenoxy]-piperidine-1-carboxylate (169 mg, 21%).
¹H-NMR (DMSO-d₆) δ ; 1.39(s, 9H), 1.45-1.6 0(1H, m), 1.89-1.92 (1H, m), 3.13-3.20(2H, m), 3.62 - 3.69 (2H, m) , 4.61-4.66 (1H, m), 6. 99 (2H, d, J=8.8Hz), 7.15(1H, m), 7.80 (1H, m), 7.81 (2H, d, J=8.8Hz).

### (b) Synthesis of 4-(piperidin-4-yloxy)benzamide trifluoroacetate

The title compound was synthesized by carrying out reaction according to the method described in Example 22 (d), except for using tert-butyl 4-[4-(aminocarbonyl)phenoxy]piperidine-1-carboxylate as a starting material.
¹H-NMR (DMSO-d₆) δ ; 1.74-1.84 (2H, m) , 2.0 7-2.11 (2H, m), 3.05-3.24 (4H, m), 4.65-4. 75 (1H, m), 7.03 (2H, d, J=8.8Hz), 7.19 (1H, m), 7.83 (3H, m) , 8.55 (1H, m).

### Example 25

### Synthesis of 4-[(1-benzylpiperidin-4-yl)oxy]benzamide

Benzaldehyde (0.076 ml, 0.748 mmol) and sodium triacetoxyborohydride (169 mg, 0.750 mmol) were added to a solution of the 4-(piperidin-4-yloxy)benzamide trifluoroacetate obtained in Example 24 (125 mg, 0.375 mmol) in 1,2-dichloroethane (7 ml), and the resulting mixture was stirred overnight at room temperature. Then, the reaction mixture was poured into a 1N-aqueous sodium hydroxide solution and extracted with ethyl acetate. The organic layer was washed with a saturated aqueous sodium chloride solution and dried over magnesium sulfate. The solvent was distilled off under reduced pressure and the resulting residue was purified by a silica gel column chromatography (eluent: ethyl acetate/methanol = 100/1) to obtain 4-[(1-benzylpiperidin-4-yl)oxy]benzamide (44 mg, 38%).

Melting point: 166-168°C.

### Example 26

### Synthesis of 4-[(1-benzylpiperidin-4-yl)(methyl)amino]benzamide

Acetic acid (0.29 ml, 5.07 mmol), paraformaldehyde (0.67 g, 8.00 mmol) and sodium cyanoborohydride (314 mg, 5.00 mmol) were added to a solution of the 4-[(1-benzylpiperidin-4-yl)amino]-benzamide obtained in Example 10 (309 mg, 0.999 mmol) in methanol (6 ml) under ice-cooling, and the resulting mixture was stirred overnight at room temperature. After completion of the reaction, the solvent was distilled off and the resulting residue was diluted with ethyl acetate, and the organic layer was washed with a saturated aqueous sodium chloride solution. The organic layer was dried over magnesium sulfate and distilled under reduced pressure to remove the solvent, and the resulting residue was purified by a silica gel column chromatography (eluent: chloroform/methanol = 97/3) to obtain 4-[(1-benzylpiperidin-4-yl)(methyl)-amino]benzamide (240 mg, 74%).

Melting point: 181-183°C.

### Example 27

### Synthesis of 4-[methyl(piperidin-4-yl)amino]benzamide

The title compound was synthesized by carrying out reaction according to the method described in Example 14, except for using the 4-[(1-benzylpiperidin-4-yl)(methyl)amino]benzamide obtained in Example 26, as a starting material.
¹H-NMR (DMSO-d₆) δ;1.54-1.66 (4H, m), 2.5 7-2. 66 (2H, m) , 2.76 (3H, s) , 2.99-3.03 (2H, m), 3.75-3.82 (1H, m), 6.74 (2H, d, J=9.2H z), 6.89 (1H, br s), 7.60 (1H, br s), 7.70 (2H, d, J=9.0Hz).

### Example 28

### Synthesis of 4-[(1-benzylpiperidin-4-yl)amino]-2-methoxybenzamide

### (a) Synthesis of methyl 2-methoxy-4-[(1-benzylpiperidin-4-yl)amino]benzoate

The title compound was synthesized by carrying out reaction according to the method described in Example 10, except for using methyl 4-amino-2-methoxybenzoate in place of p-aminobenzamide.
¹H-NMR (DMSO-d₆) δ ; 1.34-1.44(2H, m), 1.8 5-1.88 (2H, m), 2.04-2.11 (2H, m), 2.74-2. 78 (2H, m), 3.28-3.32 (1H, m), 3.45 (2H, s), 3.64 (3H, s), 3.71 (3H, s), 6.16(1H, d, J=8. 6Hz), 6.16(1H, s), 6.34 (1H, d, J=7.7Hz), 7. 21-7.34 (5H, m), 7.52 (1H, d, J=8.4Hz) .

### (b) Synthesis of 4-[(1-benzylpiperidin-4-yl)amino]-2-methoxybenzoic acid

A 4N-aqueous sodium hydroxide solution (2.5 ml) was added to a solution of methyl 2-methoxy-4-[(1-benzylpiperidin-4-yl)amino]benzoate (885 mg, 2.50 mmol) in a tetrahydrofuran (4 ml)/methanol (4 ml) mixture, and the resulting mixture was stirred at room temperature for 6 hours. A 4N-aqueous sodium hydroxide solution (1 ml) was added thereto and the resulting mixture was stirred overnight at room temperature. Furthermore, a 4N-aqueous sodium hydroxide solution (2 ml) was added thereto and the resulting mixture was stirred overnight again at room temperature. After completion of the reaction, the reaction mixture was neutralized with 1N-hydrochloric acid under ice-cooling and then concentrated. The residue was suspended in a chloroform/methanol mixture and the suspension was filtered. The filtrate was concentrated and the crude product thus obtained was purified by a silica gel column chromatography (eluent: chloroform/methanol = 99/1) to obtain 4-[(1-benzylpiperidin-4-yl)amino]-2-methoxybenzoic acid (695 mg, 82%).
¹H-NMR (DMSO-d₆) δ ; 1.33-1.44 (2H, m), 1.8 5-1.89 (2H, m), 2.05-2.12 (2H, m), 2.74-2. 78 (2H, m), 3.32 (1H, m), 3.46 (2H, s), 3.73 (3H, s), 6.16 (1H, d, J=8.0Hz), 6.17 (1H, s), 6.30 (1H, d, J=7.7Hz), 7.22-7.34 (5H, m), 7. 53 (1H, d, J=8.5Hz).

### (c) Synthesis of 4-[(1-benzylpiperidin-4-yl)amino]-2-methoxybenzamide

To a solution of 4-[(1-benzylpiperidin-4-yl)amino]-2-methoxybenzoic acid (221 mg, 0.649 mmol) in N,N-dimethylformamide (6 ml) were added 1-ethyl-3-(3-dimethylaminopropyl)carbodiimide hydrochloride (187 mg, 0.975 mmol), 1-hydroxybenzotriazole monohydrate (132 mg, 0.977 mmol), diisopropylethylamine (0.45 ml, 2.58 mmol) and ammonium chloride (70 mg, 1.31 mmol), and the resulting mixture was stirred overnight at room temperature. Then, the reaction mixture was poured into a saturated aqueous sodium hydrogencarbonate solution and extracted with ethyl acetate. The organic layer was washed with a saturated aqueous sodium chloride solution and dried over magnesium sulfate. The solvent was distilled off under reduced pressure and the resulting residue was purified by a silica gel column chromatography (eluent: chloroform/methanol = 200/1) and preparative TLC (developed with: chloroform/methanol = 90/10) to obtain 4-[(1-benzylpiperidin-4-yl)amino]-2-methoxybenzamide (56 mg, 25%).

Melting point: 155-157°C.

### Example 29

### Synthesis of 2-methoxy-4-(piperidin-4-ylamino)benzamide

The title compound was synthesized by carrying out reaction according to the method described in Example 14, except for using the 4-[(1-benzylpiperidin-4-yl)amino]-2-methoxybenzamide obtained in Example 28, as a starting material.
¹H-NMR (DMSO-d₆) δ ; 1.34-1.37 (2H, m), 1.8 8-1.92 (3H, m) , 2.68-2.75 (2H, m) , 2.74-2. 78 (2H, m) , 3.34 (1H, m) , 3.82 (3H, s), 6.21-6.23 (3H, m), 7.03 (1H, br s) , 7.31 (1H, br s), 7.66 (1H, d, J=9.2Hz).

### Example 30

### Synthesis of 3-methoxy-4-(piperidin-4-ylamino)benzamide

### (a) Synthesis of 4-[(1-benzylpiperidin-4-yl)amino]-3-methoxybenzoic acid

The title compound was synthesized by carrying out reaction according to the method described in Example 10, except for using 4-amino-3-methoxybenzoic acid in place of p-aminobenzamide.

¹H-NMR (DMSO-d₆) δ ; 1.33-1.44 (2H, m), 1.8 5-1.89 (2H, m), 2.05-2.12(2H, m), 2.74-2. 78 (2H, m) , 3.32 (1H, m), 3.46 (2H, s), 3.73 (3H, s), 6.16 (1H, d, J=8.0Hz), 6.17(1H, s), 6.30 (1H, d, J=7.7Hz), 7.22-7.34 (5H, m), 7. 53 (1H, d, J=8.5Hz).

### (b) Synthesis of 4-[(1-benzylpiperidin-4-yl)amino]-3-methoxybenzamide

The title compound was synthesized by carrying out reaction according to the method described in Example 28 (c), except for using 4-[(1-benzylpiperidin-4-yl)amino]-3-methoxybenzoic acid as a starting material.
¹H-NMR (DMSO-d₆) δ ; 1.43-1.53(2H, m), 1.8 4-1.88(2H, m), 2.05-2.12 (2H, m), 2.74-2. 78 (2H, m), 3.31(1H, m), 3.46 (2H, s), 3.80 (3H, s), 5.20 (1H, d, J=8.3Hz), 6.59 (1H, d, J=8.4Hz) , 7.21-7.34 (6H, m), 7.43 (1H, dd, J=1.8, 8.4Hz).

### (c) Synthesis of 3-methoxy-4-(piperidin-4-ylamino)-benzamide

The title compound was synthesized by carrying out reaction according to the method described in Example 14, except for using 4-[(1-benzylpiperidin-4-yl)amino]-3-methoxybenzamide as a starting material.
¹H-NMR (DMSO-d₆) δ ; 1.23-1.35 (2H, m), 1.8 2-1.86 (3H, m), 2.50-2.59 (2H, m), 2.91-2. 95 (2H, m), 3.34 (1H, m), 3.80 (3H, s), 4.89 (1H, d, J=8.3Hz), 6.55 (1H, d, J=8.4Hz), 6. 90 (1H, br s), 7.31 (1H, d, J=1.8Hz) , 7.38 (1H, dd, J=1.7, 8.1Hz), 7.62 (1H, br s).

### Example 31

### Synthesis of 4-[(1-benzylpiperidin-4-yl)amino]-3-methylbenzamide

### (a) Synthesis of methyl 3-methyl-4-[(1-benzylpiperidin-4-yl)amino]benzoate

The title compound was synthesized by carrying out reaction according to the method described in Example 10, except for using methyl 4-amino-3-methylbenzoate in place of p-aminobenzamide.
¹H-NMR (DMSO-d₆) δ ; 1.50-1.60 (2H, m), 1.8 5-1.88 (2H, m) , 2.03-2.09 (2H, m) , 2.09 (3H, s) , 2.78-2.82 (2H, m), 3.35 (1H, m), 3.47(2 H, s) , 3.72 (3H, s) , 5.16 (1H, d, J=7.9Hz), 6. 61 (1H, d, J=8.8Hz), 7.21-7.34 (5H, m), 7.5 5 (1H, d, J=1.5Hz), 7.52 (1H, dd, J=1.8, 8.6 Hz) .

### (b) Synthesis of 4-[(1-benzylpiperidin-4-yl)amino]-3-methylbenzoic acid

The title compound was synthesized by carrying out reaction according to the method described in Example 28 (b), except for using methyl 3-methyl-4-[(1-benzylpiperidin-4-yl)amino]benzoate as a starting material.
¹H-NMR (DMSO-d₆) δ ; 1.52-1.59 (2H, m), 1.8 5-1.89 (2H, m), 2.04-2.11 (2H, m), 2.08 (3H, s), 2.78-2.81 (2H, m), 3.32 (1H, m), 3.47 (2 H, s), 5.07 (1H, d, J=8. 3Hz), 6.58 (1H, d, J= 8. 8Hz), 7.21-7.34 (5H, m), 7.53 (1H, d), 7. 59 (1H, dd).

### (c) Synthesis of 4-[(1-benzylpiperidin-4-yl)amino]-3-methylbenzamide

The title compound was synthesized by carrying out reaction according to the method described in Example 28 (c), except for using 4-[(1-benzylpiperidin-4-yl)amino]-3-methylbenzoic acid as a starting material.

Melting point: 156-158°C.

### Example 32

### Synthesis of 3-methyl-4-(piperidin-4-ylamino)benzamide

The title compound was synthesized by carrying out reaction according to the method described in Example 14, except for using the 4-[(1-benzylpiperidin-4-yl)amino]-3-methylbenzamide obtained in Example 31, as a starting material.
¹H-NMR (DMSO-d₆) δ ; 1.44-1.55 (2H, m), 1.9 1-1.95(2H, m), 2.09 (3H, s), 2.72-2.79 (2H, m) , 3.09-3.13(2H, m), 3.46 (1H, m), 4.97 (1 H, d, J=7.5Hz), 6.58 (1H, d, J=8.4Hz), 6.84 (1H, br s), 7.53-7.57(3H, m).

### Example 33

### Synthesis of 4-[(1-benzylazepan-4-yl)amino]benzamide

### (a) Synthesis of 1-benzyl-4-azepanone

To a solution of 1-benzyl-4-piperidone (2.0 g, 10.6 mmol) in methanol (4 ml) was added dropwise N-methyl-N-nitrosourethane (1.39 ml, 10.8 mmol) over a period of 30 minutes so that the temperature might be maintained at -5°C or lower. During the dropwise addition, barium oxide (65 mg, 0.423 mmol) was added thereto in small portions. The resulting mixture was stirred overnight at -15°C and then filtered, and the resulting filtrate was distilled under reduced pressure to remove the solvent, followed by adding diethyl ether to the residue. After the insoluble material was filtered off, a saturated aqueous sodium hydrogencarbonate solution was added to the filtrate, followed by extraction with diethyl ether. The organic layer was dried over anhydrous magnesium sulfate and distilled under reduced pressure to remove the solvent, and the resulting residue was purified by a silica gel column chromatography (eluent: hexane/ethyl acetate = 2/1) to obtain 1-benzyl-4-azepanone (662 mg, 31%).
¹H-NMR (CDCl₃) δ;1.84 (2H, m) , 2.54 (2H, m), 2.60 (2H, m), 2.73 (4H, s), 3.65 (2H, s), 7.25 (5H, m) .

### (b) Synthesis of 4-[(1-benzylazepan-4-yl)amino]-benzamide

The title compound was synthesized by carrying out reaction according to the method described in Example 10, except for using 1-benzyl-4-azepanone in place of 1-benzyl-4-piperidone.
¹H-NMR (DMSO-d₆) δ ; 1.58-1.91(6H, m), 2.5 4-2.57(4H, m), 3.58-3.63 (1H, m), 3.63 (2H, s), 6.04 (1H, d, J=8.4H z), 6.47(2H, d, J=8. 6Hz), 6.81 (1H, br s), 7.22-7.32 (5H, m), 7. 49(1H, br s), 7.60(2H, d, J=8.4Hz).

### Example 34

### Synthesis of 4-(azepan-4-ylamino)benzamide

The title compound was synthesized by carrying out reaction according to the method described in Example 14, except for using the 4-[(1-benzylazepan-4-yl)amino]benzamide obtained in Example 33, as a starting material.
¹H-NMR (DMSO-d₆) δ;1.39-1.90 (6H, m), 2.6 4-2.81 (3H, m), 3.50-3.52 (1H, m), 6.03 (1H, d, J=7.7Hz) , 6.47 (2H, d, J=8.6Hz) , 6.81 (1 H, br s), 7.45 (1H, m), 7.60 (2H, d, J=8.6H z).

### Example 35

### Synthesis of ethyl 3-{[4-(aminocarbonyl)-phenyl]amino}-8-azabicyclo[3.2.1]octane-8-carboxylate

Acetic acid (2.0 ml, 34.9 mmol) and N-ethoxycarbonyl-4-tropinone (2.11 g, 10.5 mmol) were added to a solution of p-aminobenzamide (953 mg, 7.00 mmol) in methanol (42 ml) at room temperature, followed by adding thereto sodium cyanoborohydride (2.20 g, 35.0 mmol) in an ice-water bath, and the resulting mixture was stirred overnight at room temperature. Thereafter, the solvent was distilled off and the resulting residue was diluted with ethyl acetate, washed with a saturated aqueous sodium chloride solution and then dried over magnesium sulfate. The solvent was distilled off under reduced pressure and the resulting residue was purified by a silica gel column chromatography (chloroform/methanol) to obtain ethyl 3-{[4-(aminocarbonyl)phenyl]amino}-8-azabicyclo[3.2.1]octane-8-carboxylate (419 mg, 19%).
¹H-NMR (DMSO-d₆) δ : 1.18(3H, t, J=7.0Hz), 1.70-1.87 (4H, m) , 2.06-2.08 (4H, m), 3.58 -3.59 (1H, m) , 4.04 (2H, q, J=7.0Hz), 4.08-4.10(2H, m). 6.14(1H, d, J=3.8Hz) , 6.52 (2 H, d, J-8.8Hz), 6.84 (1H, br s), 7.53 (1H, m), 7.63 (2H, d, J=8.4Hz).

### Example 36

### Synthesis of 4-[(1-benzylpiperidin-4-yl)carbonyl]benzamide

### (a) Synthesis of benzyl 4-[(1-benzylpiperidin-4-yl)carbonyl]benzoate

Benzyl chloride (1.0 ml, 8.69 mmol) and potassium carbonate (1.21 g, 8.75 mmol) were added to a suspension of 4-(4-hydroxycarbonylbenzoyl)piperidine monohydrochloride (472 mg, 1.75 mmol) in N,N-dimethylformamide (9 ml), and the resulting mixture was stirred at 80°C for 17 hours. After completion of the reaction, the reaction suspension was filtered and the filtrate was concentrated, and the resulting residue was diluted with ethyl acetate. The dilution was washed with water, dried over magnesium sulfate and then filtered. The filtrate was concentrated and the crude product thus obtained was purified by a silica gel column chromatography (eluent: chloroform/ethyl acetate = 9/1) to obtain benzyl 4-[(1-benzylpiperidin-4-yl)carbonyl]benzoate (351 mg, 49%).

### (b) Synthesis of 4-[(1-benzylpiperidin-4-yl)carbonyl]-benzoic acid

A 4N-aqueous sodium hydroxide solution (2.1 ml) was added to a solution of benzyl 4-[(1-benzylpiperidin-4-yl)carbonyl]benzoate (351 mg, 0.849 mmol) in ethanol (10 ml), and the resulting mixture was stirred for 2.5 hours under refluxing conditions. After completion of the reaction, the reaction mixture was cooled, neutralized (pH 7) with 1N-hydrochloric acid, and then concentrated. The residue was suspended in methanol and the suspension was filtered. The filtrate was concentrated and the crude product thus obtained was purified by preparative TLC (developed with: chloroform/methanol = 1/1) to obtain 4-[(1-benzylpiperidin-4-yl)carbonyl]benzoic acid (262 mg, 95%).

### (c) Synthesis of 4-[(1-benzylpiperidin-4-yl)carbonyl]-benzamide

The title compound was synthesized by carrying out reaction according to the method described in Example 28 (c), except for using 4-[(1-benzylpiperidin-4-yl)carbonyl]benzoic acid as a starting material.
¹H-NMR (DMSO-d₆) δ ; 1.50-1,62(2H, m), 1.7 3-1.76 (2H, m) , 2.07-2.14 (2H, m), 2.81-2. 88 (2H, m) , 3.33-3.48 (1H, m), 3.48 (2H, s), 7.21-7.34 (5H, m) , 7.55 (1H, br s), 7.94-8. 02 (4H, m), 8.13(1H, m).

### Example 37

### Synthesis of 4-(piperidin-4-ylcarbonyl)-benzamide

Ammonium formate (60.0 mg) and 10%-palladium/carbon (15 mg) were added to a solution of the 4-[(1-benzylpiperidin-4-yl)carbonyl]benzamide obtained in Example 36 (60.0 mg, 0.186 mmol) in ethanol (14 ml), and the resulting mixture was stirred for 4 hours under refluxing conditions. After completion of the reaction, the reaction mixture was filtered by the use of Celite and the filtrate was concentrated. The crude product thus obtained was purified by preparative TLC (chloroform/methanol/aqueous ammonia = 100/100/2 (once) & 100/100/5 (once)) to obtain 4-(piperidin-4-ylcarbonyl)benzamide (6.0 mg, 14%) and 4-[hydroxy-(piperidin-4-yl)methyl]benzamide (14.8 mg, 34%), each as a white solid.
¹H-NMR (DMSO-d₆) δ ; 1.29-1.45 (2H, m), 1.5 7-1.68 (2H, m) , 2.60-2.64 (2H, m), 2.92-2. 96 (2H, m), 3.45-3.53(1H, m), 4.10-4.14(1 H, m), 7.54 (1H, br s), 7.95-8.02 (4H, m), 8. 13 (1H, br s).

### Example 38

### Synthesis of 4-[hydroxy(piperidin-4-yl)methyl]benzamide

The title compound was synthesized by the same process as in Example 37.
¹H-NMR (DMSO-d₆) δ;1.00-1.22 (2H, m), 1.4 8-1.66 (2H, m) , 2.24-2.35 (2H, m), 2.7 2-2. 90 (2H, m), 4.27 (1H, d, J=6.4Hz) , 5.19 (1H, br s), 7.28 (1H, br s) , 7. 31 (2H, d, J=8. 3H z), 7.79 (2H, d, J=8.3Hz) , 7.89 (1H, br s).

### Example 39

### Synthesis of 4-[(1-benzylpiperidin-4-yl)methyl]benzamide

### (a) Synthesis of 4-(piperidin-4-ylmethyl)benzoic acid monohydrochloride

To a solution of 4-(4-hydroxycarbonylbenzoyl)piperidine monohydrochloride (809 mg, 3.00 mmol) in a mixture of water (5.5 ml) and concentrated hydrochloric acid (0.5 ml) was added 10%-palladium/carbon (10 mg), and the resulting mixture was stirred at 70°C under a hydrogen atmosphere for 7 hours (until two equivalents of hydrogen was absorbed). After completion of the reaction, the reaction mixture was filtered by the use of Celite and the filtrate was concentrated. The solid precipitated during the concentration was collected by filtration and dried to obtain 4-(piperidin-4-ylmethyl)benzoic acid monohydrochloride (472 mg, 62%).

### (b) Synthesis of benzyl 4-[(1-benzylpiperidin-4-yl)methyl]benzoate

The title compound was synthesized by carrying out reaction according to the method described in Example 36 (a), except for using 4-(piperidin-4-ylmethyl)benzoic acid monohydrochloride as a starting material.

### (c) Synthesis of 4-[(1-benzylpiperidin-4-yl)methyl]-benzoic acid

The title compound was synthesized by carrying out reaction according to the method described in Example 36 (b), except for using benzyl 4-[(1-benzylpiperidin-4-yl)methyl]benzoate as a starting material.

### (d) Synthesis of 4-[(1-benzylpiperidin-4-yl)methyl]-benzamide

The title compound was synthesized by carrying out reaction according to the method described in Example 28 (c), except for using 4-[(1-benzylpiperidin-4-yl)methyl]benzoic acid as a starting material.
¹H-NMR (DMSO-d₆) δ;1.12-1.23 (2H, m), 1.4 7-1. 51 (3H, m), 1.79-1.86 (2H, m), 2.52-2. 54 (2H, m), 2.72-2.76 (2H, m), 3.43 (2H, s), 7.19-7.31 (8H, m), 7.76 (2H, d, J=8.3Hz), 7. 88 (1H, br s).

### Example 40

### Synthesis of 4-(piperidin-4-ylmehyl)benzamide

The title compound was synthesized by carrying out reaction according to the method described in Example 14, except for using the 4-[(1-benzylpiperidin-4-yl)methyl]benzamide obtained in Example 39, as a starting material.
¹H-NMR (DMSO-d₆) δ ; 0.94-1.07 (2H, m), 1.4 2-1.56 (3H, m), 1.98 (1H, br), 2.26-2.38 (2 H, m) , 2.50 (2H, m), 2.83-2.86 (2H, m), 7.20 (2H, d, J=8.3 Hz) , 7.26 (1H, br s), 7.76(2H, d, J=8.3Hz), 7.88 (1H, br s).

### Example 41

### Synthesis of 4-piperazin-1-ylbenzamide

### (a) Synthesis of 4-(4-benzylpiperazin-1-yl)benzonitrile

To a solution of 4-fluorobenzonitrile (2.50 g, 20.4 mmol) in N,N-dimethylformamide (25 ml) were added 1-benzylpiperazine (4.3 ml, 24.2 mmol) and potassium carbonate (5.65 g, 40.9 mmol), and the resulting mixture was stirred at 120°C for 18 hours. After completion of the reaction, potassium carbonate was filtered off and the filtrate was poured into water and extracted with ethyl acetate. The organic layer was dried over magnesium sulfate and filtered. The filtrate was concentrated and the crude product thus obtained was purified by a silica gel column chromatography (hexane/ethyl acetate) to obtain 4-(4-benzylpiperazin-1-yl)benzonitrile (4.92 g, 87%).

### (b) Synthesis of 4-(4-benzylpiperazin-1-yl)benzamide

A solution of 4-(4-benzylpiperazin-1-yl)benzonitrile (832 mg, 3.00 mmol) in concentrated sulfuric acid (6 ml) was stirred at 80°C for 1 hour. After completion of the reaction, the reaction solution was poured into ice water and made basic with a 4N-aqueous sodium hydroxide solution. The resulting suspension was filtered to obtain the desired compound as the precipitate on a filter. The desired compound was extracted also from the filtrate with ethyl acetate. Thus, 4-(4-benzylpiperazin-1-yl)benzamide (823 mg, 93%) was obtained as a white solid.

### (c) Synthesis of 4-piperazin-1-ylbenzamide

The title compound was synthesized by carrying out reaction according to the method described in Example 14, except for using 4-(4-benzylpiperazin-1-yl)benzamide as a starting material.
¹H-NMR (DMSO-d₆) δ ; 2.78-2.81(4H, m), 3.1 1-3.14 (4H, m), 6.89 (2H, d, J=9.0Hz), 6.99 (1H, br s), 7.68 (1H, br s), 7.72 (2H, d, J= 9.0Hz).

### Example 42

### Synthesis of tert-butyl 4-{[4-(aminocarbonyl)-3-chlorophenyl]amino}piperidine-1-carboxylate

### (a) Synthesis of 4-{[1-(tert-butoxycarbonyl)piperidin-4-yl]amino}-2-chlorobenzoic acid

Acetic acid (1.5 ml, 26.2 mmol) and t-butyl 4-oxo-1-piperidinecarboxylate (1.52 g, 7.48 mmol) were added to a solution of 4-amino-2-chlorobenzoic acid (858 mg, 5.00 mmol) in methanol (30 ml) at room temperature, followed by adding thereto sodium cyanoborohydride (1.57 g, 25.0 mmol) in an ice-water bath, and the resulting mixture was stirred overnight at room temperature. After completion of the reaction, the solvent was distilled off and the resulting residue was diluted with ethyl acetate, and the dilution was washed with a saturated aqueous sodium chloride solution, dried over magnesium sulfate, and then filtered. The filtrate was concentrated and the crude product thus obtained was purified by a silica gel column chromatography (eluent: chloroform/methanol = 97/3) to obtain 4-{[1-(tert-butoxycarbonyl)piperidin-4-yl]amino}-2-chlorobenzoic acid (1.70 g) containing a small amount of impurities.

### (b) Synthesis of tert-butyl 4-{[4-(aminocarbonyl)-3-chlorophenyl]amino}piperidine-1-carboxylate

To a solution of 4-{[1-(tert-butoxycarbonyl)-piperidin-4-yl]amino}-2-chlorobenzoic acid containing a small amount of impurities (1.70 g) in N,N-dimethylformamide (45 ml) were added 1-ethyl-3-(3-dimethylaminopropyl)carbodiimide hydrochloride (1.41 g, 7.36 mmol), 1-hydroxybenzotriazole monohydrate (997 mg, 7.38 mmol), diisopropylethylamine (3.45 ml, 19.8 mmol) and ammonium chloride (526 mg, 9.83 mmol), and the resulting mixture was stirred overnight at room temperature. After completion of the reaction, the reaction mixture was poured into a saturated aqueous sodium hydrogencarbonate solution and extracted with ethyl acetate. The organic layer was washed with a saturated aqueous sodium chloride solution, dried over magnesium sulfate, and then filtered. The filtrate was concentrated and the crude product thus obtained was purified by a silica gel column chromatography (eluent: chloroform/ethyl acetate = 5/1) and preparative TLC (developed with: chloroform/ethyl acetate = 5/1) to obtain tert-butyl 4-{[4-(aminocarbonyl)-3-chlorophenyl]amino}piperidine-1-carboxylate (598 mg, total yield from the two steps: 23%).
¹H-NMR (DMSO-d₆) δ;1.18-1.24 (2H, m), 1.3 9 (9H, s), 1.82-1.85 (2H, m), 2.90 (2H, m), 3. 41 - 3.45 (1H, m), 3.82-3.87 (2H, m), 6.13 (1 H, d, J=7.9Hz) , 6.19 (1H, dd, J=2.2, 8.4Hz), 6. 59 (1H, d, J=2.0Hz), 7. 20 (1H, br s) , 7. 2 6 (1H, d, J=8.4Hz, 7.4 3 (1H, m).

### Example 43

### Synthesis of 2-chloro-4-(piperidin-4-ylamino)benzamide trifluoroacetate

The title compound was synthesized by carrying out reaction according to the method described in Example 22 (d), except for using the tert-butyl 4-{[4-(aminocarbonyl)-3-chlorophenyl]amino}piperidine-1-carboxylate obtained in Example 42, as a starting material.
¹H-NMR (DMSO-d₆) δ;1.44-1.55 (2H, m), 1.9 5-2.03 (2H, m), 2.94-3.04 (2H, m), 3.27-3. 35 (2H, m), 3.51-3.60 (1H, m), 6.54 (1H, dd, J=2.2,8.4Hz), 6.62 (1H, d, J=2.0Hz), 7.18 (1H, m), 7.27 (1H, d , J=8.4Hz), 7.45 (1H, m).

### Example 44

Synthesis of 4-[(1-benzylpiperidin-4-yl)amino]-2-chlorobenzamide

### (a) Synthesis of 4-amino-2-chlorobenzamide

Ammonium chloride (1.07 g, 20.0 mmol), 1-ethyl-3-(3-dimethylaminopropyl)carbodiimide hydrochloride (2.88 g, 15.0 mmol), 1-hydroxybenzotriazole monohydrate (2.03 g, 15.0 mmol) and diisopropylethylamine (7.0 ml, 40.2 mmol) were added to a solution of 4-amino-2-chlorobenzoic acid (858 mg, 5.00 mmol) in N,N-dimethylformamide (50 ml), and the resulting mixture was stirred overnight at room temperature. After completion of the reaction, the reaction mixture was poured into a saturated aqueous sodium hydrogencarbonate solution and the precipitate formed was filtered off. Sodium chloride was added to the filtrate, followed by extraction with ethyl acetate and chloroform. The organic layer was dried over magnesium sulfate and then filtered. The filtrate was concentrated and the crude product thus obtained was purified by a silica gel column chromatography (eluent: chloroform/methanol = 50/1) to obtain 4-amino-2-chlorobenzamide (673 mg, 79%).

### (b) Synthesis of 4-[(1-benzylpiperidin-4-yl)amino]-2-chlorobenzamide

The title compound was synthesized by carrying out reaction according to the method described in Example 10, except for using 4-amino-2-chlorobenzamide in place of p-aminobenzamide.
¹H-NMR (DMSO-d₆) δ;1.30-1.41 (2H, m) , 1.8 3-1.86 (2H, m), 2.04-2.11 (2H, m), 2.73-2. 77 (2H, m) , 3.24-3.30 (1H, m), 3.46 (2H, s) , 6.11 (1H, d, J=7. 9Hz), 6.49 (1H, dd, J=2. 2, 8.7 Hz) , 6.56 (1H, d, J=2.2Hz), 7.18 (1H, br s), 7.21-7.34 (6H, m), 7.42 (1H, br s).

### Example 45

### Synthesis of 3-chloro-4-(piperidin-4-ylamino)benzamide

### (a) Synthesis of tert-butyl 4-{[4-(aminocarbonyl)-phenyl]amino}piperidine-1-carboxylate

A solution of t-butyl 4-oxo-1-piperidinecarboxylate (8.12 g, 39.9 mmol) in 1,2-dichloroethane (3 ml), sodium triacetoxyborohydride (11.3 g, 50.1 mmol) and acetic acid (2.3 ml, 40.29 mmol) were added to a solution of p-aminobenzamide (2.72 g, 20.0 mmol) in 1,2-dichloroethane (80 ml), and the resulting mixture was stirred overnight at room temperature. After completion of the reaction, the reaction mixture was poured into a 1N-aqueous sodium hydroxide solution and extracted with ethyl acetate. The organic layer was washed with a saturated aqueous sodium chloride solution, dried over magnesium sulfate and then filtered. The filtrate was concentrated and the crude product thus obtained was purified by a silica gel column chromatography (eluents: chloroform/methanol = 95/5 and chloroform/ethyl acetate = 3/2) to obtain tert-butyl 4-{[4-(aminocarbonyl)phenyl]amino}piperidine-1-carboxylate (5.73 g, 90%).

### (b) Synthesis of tert-butyl 4-{[4-(aminocarbonyl)-2-chlorophenyl]amino}piperidine-1-carboxylate

To a solution of tert-butyl 4-{[4-(aminocarbonyl)phenyl]amino}piperidine-1-carboxylate (958 mg, 3.00 mmol) in 2-propanol (25 ml) was added N-chlorosuccinimide (401 mg, 3.00 mmol), and the resulting mixture was stirred at room temperature overnight and then at 60°C for 4 hours. After completion of the reaction, the solvent was distilled off and the residue was diluted with ethyl acetate, and the dilution was washed with water, dried over magnesium sulfate and then filtered. The filtrate was concentrated and the crude product thus obtained was purified by a silica gel column chromatography (eluent: hexane/ethyl acetate = 1/2) to obtain tert-butyl 4-{[4-(aminocarbonyl)-2-chlorophenyl]amino}piperidine-1-carboxylate (790 mg, 75%).

### (c) Synthesis of 3-chloro-4-(piperidin-4-ylamino)-benzamide

Trifluoroacetic acid (9.5 ml) was added to a solution of tert-butyl 4-{[4-(aminocarbonyl)-2-chlorophenyl]amino}piperidine-1-carboxylate (850 mg, 2.40 mmol) in methylene chloride (15 ml), and the resulting mixture was stirred overnight at room temperature. After completion of the reaction, the reaction mixture was concentrated and a 1N-aqueous sodium hydroxide solution was added thereto, followed by extraction with ethyl acetate and chloroform. The organic layer was dried over magnesium sulfate and filtered, and the filtrate was concentrated to obtain 3-chloro-4-(piperidin-4-ylamino)benzamide (383 mg, 63%).
¹H-NMR (DMSO-d₆) δ;1.30-1.42 (2H, m), 1.8 0-1.84 (2H, m), 2.02 (1H, br), 2.54-2.57 (2 H, m), 2.90-2.94 (2H, m), 3. 41-3.44 (1H, m), 5.22 (1H, d, J=8.1Hz), 6.78 (1H, d, J=8.8H z), 7.06 (1H, br s), 7.66 (1H, dd, J=2.0, 8. 6Hz) , 7.72 (1H, br s), 7.80 (1H, d, J=2.0H z).

### Example 46

### Synthesis of 4-[(1-benzylpiperidin-4-yl)amino]-3-chlorobenzamide

Benzaldehyde (0.052 ml, 0.512 mmol), sodium triacetoxyborohydride (222 mg, 0.985 mmol) and acetic acid (0.045 ml, 0.786 mmol) were added to a solution of the 3-chloro-4-(piperidin-4-ylamino)benzamide obtained in Example 45 (100 mg, 0.394 mmol) in 1,2-dichloroethane (4 ml), and the resulting mixture was stirred overnight at room temperature. After completion of the reaction, the reaction mixture was poured into a 1N-aqueous sodium hydroxide solution and extracted with ethyl acetate. The organic layer was dried over magnesium sulfate and filtered. The filtrate was concentrated and the crude product thus obtained was purified by preparative TLC (eluent: chloroform/methanol = 9/1) to obtain 4-[(1-benzylpiperidin-4-yl)amino]-3-chlorobenzamide (96 mg, 71%) as a white solid.
¹H-NMR (DMSO-d₆) δ ; 1.50-1.60 (2H, m), 1.8 4-1.87 (2H, m) , 2.06-2.13(2H, m), 2.75-2. 79 (2H, m), 3.41 (1H, m), 3.47(2H, s), 5.26 (1H, d, J=8.3Hz) , 6.78 (1H, d, J=8.8Hz), 7. 05 (1H, br s), 7.21-7.34 (5H, m), 7.66 (1H, dd, J=2.0, 8.6Hz), 7.71 (1H, br s), 7.79 (1 11, d, J=2.0Hz).

### Example 47

### Synthesis of 4-[(1-benzylpiperidin-4-yl)amino]-2-methylbenzamide

### (a) Synthesis of 2-methyl-4-nitrobenzonitrile

Copper cyanide (3.76 g, 42.0 mmol) was added to a suspension of 2-bromo-5-nitrotoluene (7.56 g, 35.0 mmol) in N,N-dimethylformamide (8 ml), and the resulting mixture was stirred at 145°C for 5 hours. After completion of the reaction, the reaction mixture was poured into a solution of iron (III) chloride hexahydrate (9.0 g) in 1N-hydrochloric acid (67 ml), and the resulting mixture was stirred at 60°C for 20 minutes and then extracted with ethyl acetate. The extract solution was washed with water, dried over magnesium sulfate and then filtered. The filtrate was concentrated and the crude product thus obtained was purified by a silica gel column chromatography (eluent: hexane/chloroform = 5/5) to obtain 2-methyl-4-nitrobenzonitrile (4.92 g, 87%).

### (b) Synthesis of 4-amino-2-methylbenzonitrile

Tin chloride dihydrate (18.5 g, 82.0 mmol) was added to a solution of 2-methyl-4-nitrobenzonitrile (3.80 g, 23.4 mmol) in ethanol (140 ml), and the resulting mixture was stirred for 3 hours under refluxing conditions. After completion of the reaction, the solvent was distilled off and the resulting residue was diluted with ethyl acetate and washed with a saturated aqueous sodium hydrogencarbonate solution. After the precipitate obtained during the washing was filtered by the use of Celite, the filtrate was extracted with ethyl acetate and the extract solution was dried over magnesium sulfate and filtered. The filtrate was concentrated to obtain 4-amino-2-methylbenzonitrile (2.89 g, 94%).

### (c) Synthesis of 4-amino-2-methylbenzamide

A solution of 4-amino-2-methylbenzonitrile (793 mg, 6.00 mmol) in concentrated sulfuric acid (12 ml) was stirred at 80°C for 1 hour. After completion of the reaction, the reaction solution was poured into ice water and made basic with a 4N-aqueous sodium hydroxide solution, and the resulting suspension was filtered to obtain the desired compound as the precipitate on a filter. The desired compound was extracted also from the filtrate with ethyl acetate. Thus, 4-amino-2-methylbenzamide (882 mg, 98%) was obtained as a white solid.

### (d) Synthesis of 4-[(1-benzylpiperidin-4-yl)amino]-2-methylbenzamide

The title compound was synthesized by carrying out reaction according to the method described in Example 10, except for using 4-amino-2-methylbenzamide in place of p-aminobenzamide.
¹H-NMR (DMSO-d₆) δ;1.30-1.40 (2H, m), 1.8 3-1.87 (2H, m), 2.03-2.10 (2H, m), 2.29 (3H, s), 2.74-2.78 (2H, m), 3.21-3.23 (1H, m), 3. 45 (2H, s) , 5.71 (1H, d, J=8.1Hz), 6.33-6.3 5 (2H, m), 6.81 (1H, br s) , 7.20-7.34 (7H, m) .

### Example 48

### Synthesis of 2-methyl-4-(piperidin-4-ylamino)benzamide

The title compound was synthesized by carrying out reaction according to the method described in Example 14, except for using the 4-[(1-benzylpiperidin-4-yl)amino]-2-methylbenzamide obtained in Example 47, as a starting material.
¹H-NMR (DMSO-d₆) δ;1.13-1.26 (2H, m), 1.8 0-1.83 (2H, m), 2.30 (3H, s) , 2.52-2.56 (2H, m), 2.90-2.94 (2 H, m), 5.71 (1H, d, J=8.1H z), 6.33-6.35 (2H, m), 6.80 (1H, br s), 7.2 0-7.23 (3H, m).

### Example 49

### Synthesis of 4-[(1-benzylpiperidin-4-yl)amino]-2-hydroxybenzamide

A 1M-boron tribromide/methylene chloride solution (10.6 ml, 10.6 mmol) was added dropwise to a solution of the 4-[(1-benzylpiperidin-4-yl)amino]-2-methoxybenzamide obtained in Example 28 (1.20 g, 3.54 mmol) in methylene chloride (36 ml), and the resulting mixture was stirred overnight at room temperature. A 1M-boron tribromide/methylene chloride solution (4.3 ml, 4.3 mmol) was further added thereto, and the resulting mixture was stirred overnight at room temperature. After completion of the reaction, water was added dropwise to the reaction mixture in an ice-water bath, and the resulting mixture was stirred at room temperature for a while. The mixture was extracted with chloroform and the insoluble material formed during the extraction and the residue obtained by concentrating the aqueous layer were purified by a silica gel column chromatography (eluents: chloroform/methanol/aqueous ammonia = 90/10/0.1 and ethyl acetate/methanol = 100/1.5) and preparative TLC (developed with: chloroform/methanol/aqueous ammonia = 90/10/0.1) to obtain 4-[(1-benzylpiperidin-4-yl)amino]-2-hydroxybenzamide (438 mg, yield: 38%). ¹H-NMR (DMSO-d₆) δ ; 1.35-1.38 (2H, m) , 1.8 2-1.86 (2H, m) , 2.02-2.10(2H, m) , 2.73-2. 77(2H, m), 3.22 (1H, m) , 3.45(2H, br s), 5. 88 (1H, d, J=2.0Hz), 6.05 (1H, dd, J=2.2,8. 8Hz) , 6.15 (1H, d, J=7.7Hz) , 7.22-7.31(6H, m) , 7.47(1H, d, J=8.8Hz) , 7.82 (1H, br s), 13.31 (1H, s).

### Example 50

### Synthesis of 2-hydroxy-4-(piperidin-4-ylamino)benzamide

The title compound was synthesized by carrying out reaction according to the method described in Example 14, except for using the 4-[(1-benzylpiperidin-4-yl)amino]-2-hydroxybenzamide obtained in Example 49, as a starting material.
¹H-NMR (DMSO-d₆) δ ; 1.17-1.25 (2H, m) , 1.7 8-1.81(2H, m), 2.54(2H, m), 2.88-2.92 (2H, m), 3.22-3.24(1H, m) , 4.35(1H, m) , 5.87 (1 H, d, J=1.8Hz), 6.02-6.05 (1H, m) , 6.12 (1H, d, J=7.0Hz), 7.25 (1H, br s), 7.46 (1H, d, J =8.8Hz) , 7.87 (1H, br s).

### Example 51

### Synthesis of 4-[(1-benzylpiperidin-4-yl)amino]-3-hydroxybenzamide

The title compound was synthesized by carrying out reaction according to the method described in Example 49, except for using the 4-[(1-benzylpiperidin-4-yl)amino]-3-methoxybenzamide obtained in Example 30 (b), as a starting material.
¹H-NMR (DMSO-d₆) δ ; 1.43-1.46 (2H, m) , 1.8 7-1.90 (2H, m) , 2.14 (2H, m) , 2.76-2.79 (2 H, m), 3.28 (1H, m) , 3.50 (2H, br s) , 4.73(1H, d, J=8.3Hz), 6.48(1H, d, J=8.3Hz), 6.79 (1 H, br s) , 7.18-7.35(7H, m) , 7.49 (1H, br s), 9. 43 (1H, s).

### Example 52

### Synthesis of 3-hydroxy-4-(piperidin-4-ylamino)benzamide

The title compound was synthesized by carrying out reaction according to the method described in Example 14, except for using the 4-[(1-benzylpiperidin-4-yl)amino]-3-hydroxybenzamide obtained in Example 51, as a starting material.
¹H-NMR (DMSO-d₆) δ;1.18-1.28 (2H, m), 1.8 3-1.86 (2H, m) , 2.50-2.56 (2H, m) , 2.89-2. 93 (2H, m) , 3.32 (1H, m) , 4.75 (1H, d, J=8.3 H z) , 6.44 (1H, d, J=8.3Hz), 6.74 (1H, br s) , 7.15-7.18 (2H, m) , 7.43 (1H, br s).

### Example 53

### Synthesis of 4-[(1-benzylpiperidin-4-yl)amino]-2-propoxybenzamide

To a solution of the 4-[(1-benzylpiperidin-4-yl)amino]-2-hydroxybenzamide obtained in Example 49 (55 mg, 0.169 mmol) in N,N-dimethylformamide (4 ml) were added 1-bromopropane (16 µl, 10.6 mmol) and potassium carbonate (25 mg, 0.181 mmol), and the resulting mixture was stirred at 70°C for 5 hours. After completion of the reaction, the reaction mixture was poured into water and extracted with ethyl acetate, and the organic layer was dried over magnesium sulfate and filtered. The filtrate was concentrated and the crude product thus obtained was purified by preparative TLC (eluent: chloroform/methanol = 90/10) to obtain 4-[(1-benzylpiperidin-4-yl)amino]-2-propoxybenzamide (48 mg, 78%).
¹H-NMR (DMSO-d₆) δ ; 0.98 (3H, t, J=7.3Hz) , 1. 33-1.43 (2H, m) , 1.72-1.81(2 H, m) , 1.84 -1.88 (2H, m) , 2.05-2.12 (2H, m) , 2.74-2.7 8 (2H, m) , 3.27 (1H, m) , 3.46 (2H, s) , 3.99 (3 H, t, J=7.3Hz) , 6.11(1H, d, J=7.9Hz) , 6.18 -6.21 (2H, m) , 7.07 (1H, br s), 7.22-7.34 (6H, m) , 7.65 (1H, d, J=9.2Hz) .

### Example 54

### Synthesis of 4-(piperidin-4-ylamino)-2-propoxybenzamide

Ammonium formate (45.0 mg ) and 10%-palladium/carbon (14 mg) were added to a solution of the 4-[(1-benzylpiperidin-4-yl)amino]-2-propoxybenzamide obtained in Example 53 (45.0 mg, 0.122 mmol) in ethanol (5 ml), and the resulting mixture was stirred for 4 hours under refluxing conditions. After completion of the reaction, the reaction mixture was filtered by the use of Celite and the filtrate was concentrated. The crude product thus obtained was purified by preparative TLC (developed with: chloroform/methanol/aqueous ammonia = 90/10/0.1) to obtain 4-(piperidin-4-ylamino)-2-propoxybenzamide (19.0 mg, 56%).
¹H-NMR (DMSO-d₆) δ;0.98 (3H, t, J=7.3Hz) , 1.14-1.25 (2H, m) , 1.72-1.84 (4H, m) , 2.00 (1H, br s) , 2.50-2.55 (2H, m), 2.89-2.93 (2H, m) , 3.99 (2H, t, J=6.4Hz) , 6.10 (1H, d, J=8.1Hz), 6.18-6.20 (2H, m), 7.07 (1H, br s) , 7.28 (1H, br s) , 7.65 (1H, d, J=9.2Hz).

### Example 55

### Synthesis of 4-[(1-benzylpiperidin-4-yl)amino]-2-isopropoxybenzamide

Isopropyl iodide (0.031 ml, 0.311 mmol) and cesium carbonate (101 mg, 0.310 mmol) were added to a solution of the 4-[(1-benzylpiperidin-4-yl)amino]-2-hydroxybenzamide obtained in Example 49 (96.0 mg, 0.295 mmol) in N,N-dimethylformamide (5 ml), and the resulting mixture was stirred at 70□ for 18 hours. The reaction mixture was poured into water and extracted with a mixture of ethyl acetate and toluene (approximately 1: 1), and the organic layer was dried over magnesium sulfate and filtered. The filtrate was concentrated and the crude product thus obtained was purified by preparative TLC (developed with: chloroform/methanol = 90/10) to obtain 4-[(1-benzylpiperidin-4-yl)amino]-2-isopropoxybenzamide (71.2 mg, 66%).
¹H-NMR (DMSO-d₆) δ ; 1.31 (6H, d, J=6.1Hz), 1.30-1.39 (2H, m) , 1.85-1.89(2H, m) , 2.05 -2.12 (2H, m) , 2.74-2.78 (2H, m) , 3.28 (1H, br s), 3.46 (2H, s) , 4.62-4.69 (1H, m) , 6.0 8 (1H, d, J=8.1Hz), 6.18-6.21(2H, m) , 7.04 (1H, br s) , 7.23-7.32 (6H, m), 7.66 (1H, d, J=8.6Hz).

### Example 56

### Synthesis of 2-isopropoxy-4-(piperidin-4-ylamino)benzamide

The title compound was synthesized by carrying out reaction according to the method described in Example 54, except for using the 4-[(1-benzylpiperidin-4-yl)amino]-2-isopropoxybenzamide obtained in Example 55, as a starting material.
¹H-NMR (DMSO-d₆) δ ; 1.32 (6H, d, J=6.1Hz), 1.32-1.37 (2H, m), 1.86-1.90 (2H, m) , 2.64 -2.71(2H, m) , 3.01-3.05 (2H, m) , 3.39-3.4 6 (1H, br s) , 4.62-4.70 (1H, m) , 6.16 (1H, d, J=7.5Hz), 6.18-6.23 (2H, m) , 7.06(1H, br s), 7.34(1H, br s) , 7.67(1H, d, J=8.4Hz).

### Example 57

### Synthesis of 4-(piperidin-4-ylamino)-3-(trifluoromethyl)benzamide

### (a) Synthesis of tert-butyl 4-{[4-(aminocarbonyl)-2-(trifluoromethyl)phenyl]amino}piperidine-1-carboxylate

To a solution of the tert-butyl 4-{[4-(aminocarbonyl)phenyl]amino}piperidine-1-carboxylate obtained in Example 45 (a) (208 mg, 0.651 mmol) in N,N-dimethylformamide (2 ml) were added 4-dimethylaminopyridine (80 mg, 0.655 mmol) and S-(trifluoromethyl)-3,7-dinitrodibenzothiophenium trifluoromethanesulfonate (321 mg, 0.652 mmol), and the resulting mixture was stirred overnight at room temperature. The reaction mixture was poured into water and extracted with ethyl acetate, and the organic layer was dried over magnesium sulfate and filtered. The filtrate was concentrated and the crude product thus obtained was purified by a silica gel column chromatography (eluent: chloroform/methanol = 95/5) and preparative TLC (developed with: ethyl acetate/methanol = 100/1.5) to obtain tert-butyl 4-{[4-(aminocarbonyl)-2-(trifluoromethyl)phenyl]amino}piperidine-1-carboxylate (31.6 mg, 13%).

### (b) Synthesis of 4-(piperidin-4-ylamino)-3-(trifluoromethyl)benzamide

The title compound was synthesized by carrying out reaction according to the method described in Example 45 (c), except for using tert-butyl 4-{[4-(aminocarbonyl)-2-(trifluoromethyl)phenyl]amino}-piperidine-1-carboxylate as a starting material.
¹H-NMR (DMSO-d₆) δ ; 1.44-1.54 (2H, m), 1.8 5-1.92 (2H, m), 2.68-2.76(2H, m) , 2.66 (3H, s) , 3.03-3.08(2H, m), 3.62 (1H, m) , 5.13 (1 H, d, J=7.9Hz), 6.96 (1H, d, J=8.8Hz) , 7.15 (1H, br s) , 7.86 (1H, br s), 7.92(1H, dd, J =1.8, 9.0Hz), 7.98 (1H, d, J=1.8Hz).

### Example 58

### Synthesis of 4-[(1-benzylpiperidin-4-yl)amino]-3-(trifluoromethyl)benzamide

The title compound was synthesized by carrying out reaction according to the method described in Example 46, except for using the 4-(piperidin-4-ylamino)-3-(trifluoromethyl)benzamide obtained in Example 57, as a starting material.
¹H-NMR (DMSO-d₆) δ ; 1.51-1.61(2H, m), 1. 8 5-1.88 (2H, m), 2.09-2.16 (2H, m) , 2.72-2. 75 (2H, m) , 3.47(1H, m) , 3.47 (2H, s ) , 5.03 (1H, d, J=7.5Hz) , 6.93 (1H, d, J=8.8Hz) , 7. 14 (1H, br s), 7.21-7.34 (5H, m) , 7.84 (1H, br s), 7.91(1H, dd, J=2.0, 8.8Hz) , 7.97 (1 H, d, J=2.0Hz).

### Example 59

### Synthesis of 4-[(1-benzylpiperidin-4-yl)amino]-2-(trifluoromethoxy)benzamide

### (a) Synthesis of 4-bromo-3-(trifluoromethoxy)aniline

A solution of N-bromosuccinimide (3.78 g, 21.2 mmol) in N,N-dimethylformamide (11 ml) was added dropwise to a solution of 3-trifluoromethoxyaniline (3.84 g, 21.2 mmol) in N,N-dimethylformamide (11 ml) at room temperature over a period of 40 minutes. After completion of the dropwise addition, the resulting mixture was stirred overnight. After completion of the reaction, the reaction mixture was poured into water and extracted with ethyl acetate, and the organic layer was dried over magnesium sulfate and filtered. The filtrate was concentrated and the crude product thus obtained was purified by a silica gel column chromatography (hexane/chloroform = 6/4) to obtain 4-bromo-3-(trifluoromethoxy)aniline (4.96 g, 91%).

### (b) Synthesis of tert-butyl 4-bromo-3-(trifluoromethoxy)phenylcarbamate

A solution of sodium hydroxide (199 mg, 4.98 mmol) in water (1.5 ml) and a solution of di-tert-butyl dicarbonate (1.09 g, 4.99 mmol) in t-butanol (1 ml) were added to a solution of 4-bromo-3-(trifluoromethoxy)aniline (510 mg, 1.99 mmol) in t-butanol (2.5 ml) at room temperature, and the resulting mixture was stirred for 4 days. After completion of the reaction, the reaction mixture obtained as a suspension was filtered by the use of a small volume of water and the filtrate was concentrated to about one-half its original volume. The resulting residue was washed with water and extracted with ethyl acetate. The organic layer was washed with a saturated aqueous sodium chloride solution, dried over magnesium sulfate and then filtered. The filtrate was concentrated and the crude product thus obtained was purified by a silica gel column chromatography (eluent: hexane/ethyl acetate = 95/5) to obtain tert-butyl 4-bromo-3-(trifluoromethoxy)phenylcarbamate (666 mg, 94%).

### (c) Synthesis of 4-amino-2-(trifluoromethoxy)-benzonitrile

Copper cyanide (966 mg, 10.8 mmol) was added to a solution of tert-butyl 4-bromo-3-(trifluoromethoxy)phenylcarbamate (1.30 g, 3.60 mmol) in N,N-dimethylformamide (14 ml) at room temperature, and the resulting mixture was stirred for 4.5 hours under refluxing conditions. Then, copper cyanide (322 mg, 3.60 mmol) was added thereto, and the resulting mixture was stirred for 4 hours under refluxing conditions. Furthermore, copper cyanide (483 mg, 5.39 mmol) was added thereto, and the resulting mixture was stirred for 1 hour under refluxing conditions. After completion of the reaction, a solution of iron (III) chloride hexahydrate (4.24 g) in 1N-hydrochloric acid (32 ml) was added to the reaction mixture and the resulting mixture was stirred at 60°C for 20 minutes and then extracted with ethyl acetate. The organic layer was washed with water, dried over magnesium sulfate and then filtered. The filtrate was concentrated and the crude product thus obtained was purified by a silica gel column chromatography (eluent: hexane/ethyl acetate = 7/3) to obtain 4-amino-2-(trifluoromethoxy)benzonitrile (214 mg, 29%).

### (d) Synthesis of 4-amino-2-(trifluoromethoxy)benzamide

A solution of 4-amino-2-(trifluoromethoxy)benzonitrile (200 mg, 0.989 mmol) in concentrated sulfuric acid (4 ml) was stirred at 80°C for 2.5 hours. After completion of the reaction, the reaction solution was poured onto ice and a 10N-aqueous sodium hydroxide solution was added thereto under ice-cooling, and the resulting mixture was stirred at room temperature for a while. The resulting suspension was filtered by the use of water to obtain 4-amino-2-(trifluoromethoxy)benzamide (165 mg) containing a small amount of impurities. Then, the filtrate was extracted with ethyl acetate and the organic layer was dehydrated over magnesium sulfate and filtered. The filtrate was concentrated, and the crude product thus obtained and the above-mentioned 4-amino-2-(trifluoromethoxy)-benzamide (165 mg) containing a small amount of impurities were together purified by a silica gel column chromatography (eluent: chloroform/ethyl acetate = 3/1) to obtain 4-amino-2-(trifluoromethoxy)benzamide (150 mg, 69%).

### (e) Synthesis of 4-[(1-benzylpiperidin-4-yl)amino]-2-(trifluoromethoxy)benzamide

The title compound was synthesized by carrying out reaction according to the method described in Example 10, except for using 4-amino-2-(trifluoromethoxy)benzamide in place of p-aminobenzamide.
¹H-NMR (DMSO-d₆) δ ; 1.32-1.42 (2H, m) , 1.8 4-1.87(2H, m), 2.04-2.11 (2H, m), 2.73-2. 77 (2H, m) , 3.2 (1H, m) , 3.46 (2H, s) , 6.37 (1 H, d, J=7.5Hz), 6.47 (1H, br s) , 6.54 (1H, d d, J=2.0, 8.8Hz), 7.16(1H, br s) , 7.22-7. 34 (6H, m) , 7.41(1H, d, J=8.6Hz).

### Example 60

### Synthesis of 4-(piperidin-4-ylamino)-2-(trifluoromethoxy)benzamide

To a solution of the 4-amino-2-(trifluoromethoxy)benzamide obtained in Example 59 (d) (440 mg, 2.00 mmol) in 1,2-dichloroethane (13 ml) were added t-butyl 4-oxo-1-piperidinecarboxylate (813 mg, 4.00 mmol), sodium triacetoxyborohydride (1.13 g, 5.01 mmol) and acetic acid (0.23 ml, 4.02 mmol), and the resulting mixture was stirred overnight at room temperature. After completion of the reaction, the reaction mixture was poured into a 1N-aqueous sodium hydroxide solution under ice-cooling and extracted with ethyl acetate. The organic layer was washed with a saturated aqueous sodium chloride solution, dried over magnesium sulfate and then filtered. The filtrate was concentrated and the crude product thus obtained was purified by a silica gel column chromatography (eluents: chloroform/methanol = 98/2 and chloroform/ethyl acetate = 5/1) to obtain a mixture containing a small amount of impurities (698 mg).

Subsequently, trifluoroacetic acid (4.8 ml) was added to a solution of the mixture containing a small amount of impurities (484 mg) in methylene chloride (8 ml), and the resulting mixture was stirred overnight at room temperature. After completion of the reaction, the reaction mixture was concentrated and the resulting residue was diluted with ethyl acetate and washed with a 1N-aqueous sodium hydroxide solution. The organic layer was dried over magnesium sulfate and filtered, and the filtrate was concentrated.

To a solution of the resulting residue (241 mg) in ethanol (3 ml) was added dropwise a 1N-hydrochloric acid/diethyl ether solution (2 ml, 2 mmol), and the resulting mixture was stirred at room temperature for 5 hours. The suspension thus obtained was filtered by the use of ethanol and the precipitate was dried to obtain 4-(piperidin-4-ylamino)-2-(trifluoromethoxy)benzamide (212 mg, 28%).
¹H-NMR (DMSO-d₆) δ ; 1.53-1.63 (2H, m), 1. 9 9-2.03 (2H, m) , 2.97-2.99 (2H, m) , 3.24-3. 29 (2H, m), 3.59(1H, m) , 6.53(1H, m), 6.61 (1H, dd, J=2.2, 8.6Hz) , 7.21(1H, br s) , 7. 29(1H, br s) , 7.44 (1H, d, J=8.6Hz), 8.89 (2H, br s).

### Example 61

### Synthesis of 2-bromo-4-(piperidin-4-ylamino)-benzamide

### (a) Synthesis of 2-bromo-4-nitrobenzoic acid

Water (40 ml) was added to a solution of 2-bromo-4-nitrotoluene (4.41 g, 20.0 mmol) in pyridine (20 ml), and the resulting mixture was heated at 70°C. Potassium permanganate (19.0 g, 120 mmol) was added thereto over a period of 40 minutes and the resulting mixture was refluxed for 8 hours. After completion of the reaction, the suspension thus obtained was filtered. The filtrate was acidified with 6N-hydrochloric acid under ice-cooling. The resulting suspension was filtered to obtain 2-bromo-4-nitrobenzoic acid (1.63 g). Hydrochloric acid was added to the filtrate, followed by extraction with ethyl acetate and chloroform. The organic layer was dried over magnesium sulfate and filtered. The filtrate was concentrated to obtain 2-bromo-4-nitrobenzoic acid (1.61 g, total amount 3.24 g, 66%).

### (b) Synthesis of 2-bromo-4-nitrobenzamide

The title compound was synthesized by carrying out reaction according to the method described in Example 44 (a), except for using 2-bromo-4-nitrobenzoic acid as a starting material.

### (c) Synthesis of 4-amino-2-bromobenzamide

The title compound was synthesized by carrying out reaction according to the method described in Example 47 (b), except for using 2-bromo-4-nitrobenzamide as a starting material.

### (d) Synthesis of tert-butyl 4-{[4-(aminocarbonyl)-3-bromophenyl]amino}piperidine-1-carboxylate

The title compound was synthesized by carrying out reaction according to the method described in Example 45 (a), except for using 4-amino-2-bromobenzamide in place of p-aminobenzamide.

### (e) Synthesis of 2-bromo-4-(piperidin-4-ylamino)benzamide

The title compound was synthesized by carrying out reaction according to the method described in Example 45 (c), except for using tert-butyl 4-{[4-(aminocarbonyl)-3-bromophenyl]amino}piperidine-1-carboxylate as a starting material.

### Example 62

### Synthesis of 4-[(1-benzylpiperidin-4-yl)amino]-2-bromobenzamide

The title compound was synthesized by carrying out reaction according to the method described in Example 10, except for using the 4-amino-2-bromobenzamide obtained in Example 61 (c), in place of p-aminobenzamide.
¹H-NMR (DMSO-d₆) δ ; 1.30-1.40 (2H, m) , 1.8 2-1.86 (2H, m) , 2.04-2.11 (2H, m) , 2.73-2. 77 (2H, m), 3. 24-3.28 (1H, m) , 3.46 (2H, s), 6.05 (1H, d, J=7.9Hz), 6.53 (dd, J=2. 2,8.6 Hz) , 6.75 (d, J=2.2Hz), 7.14 (1H, br s), 7. 17-7.34 (6H, m) , 7.49-7.54 (2H, m) , 7.43 (1 H, br s).

### Example 63

### Synthesis of 3-bromo-4-(piperidin-4-ylamino)benzamide

### (a) Synthesis of tert-butyl 4-{[4-(aminocarbonyl)-2-bromophenyl]amino}piperidine-1-carboxylate

To a solution of the tert-butyl 4-{[4-(aminocarbonyl)phenyl]amino}piperidine-1-carboxylate obtained in Example 45 (a) (500 mg, 1.57 mmol) in 2-propanol (10 ml) was added N-bromosuccinimide (279 mg, 21.2 mmol) at room temperature over a period of 15 minutes (in four equal portions and at interval of five minutes). After completion of the addition, the resulting mixture was stirred for 2 hours. After completion of the reaction, the reaction mixture was concentrated and the resulting residue was washed with water and extracted with ethyl acetate. The organic layer was dried over magnesium sulfate and filtered. The filtrate was concentrated and the crude product thus obtained was purified by a silica gel column chromatography (eluent: chloroform/ethyl acetate = 7/3) to obtain tert-butyl 4-{[4-(aminocarbonyl)-2-bromophenyl]amino}piperidine-1-carboxylate (593 mg, 95%).

### (b) Synthesis of 3-bromo-4-(piperidin-4-ylamino)benzamide

The title compound was synthesized by carrying out reaction according to the method described in Example 45 (c), except for using tert-butyl 4-{[4-(aminocarbonyl)-2-bromophenyl]amino}piperidine-1-carboxylate as a starting material.
¹H-NMR (DMSO-d₆) δ;1.27-1.40 (2H, m) , 1.8 2-1.85 (2H, m), 2.49-2.58 (2H, m) , 2.89-2. 9 4 (2H, m), 3.44-3.46 (1H, m), 4.95 (1H, d, J =7.9Hz) , 6.76 (1H, d, J=8.6Hz), 7.05 (1H, b r s), 7.70 (1H, d, J=9.9Hz) , 7.71 (1H, br s), 7.96 (1H, d, J=1.5Hz).

### Example 64

### Synthesis of 4-[(1-benzylpiperidin-4-yl)amino]-3-bromobenzamide

The title compound was synthesized by carrying out reaction according to the method described in Example 46, except for using the 3-bromo-4-(piperidin-4-ylamino)benzamide obtained in Example 63, as a starting material.
¹H-NMR (DMSO-d₆) δ ; 1.48-1.59(2H, m) , 1.8 5-1.89 (2H, m) , 2.07-2.15(2H, m) , 2.73-2. 77(2H, m) , 3.41(1H, m) , 3.47 (2H, s), 5.01 (1H, d, J=8.1Hz), 6.76 (1H, d, J=8.8Hz) , 7. 06 (1H, br s) , 7.23-7.34 (5H, m) , 7.70 (1H, d, J=8.8Hz), 7.71(1H, br s), 7.96 (1H, d, J =2.0Hz).

### Example 65

### Synthesis of 2-fluoro-4-(piperidin-4-ylamino)benzamide

### (a) Synthesis of 2-fluoro-4-nitrobenzamide

The title compound was synthesized by carrying out reaction according to the method described in Example 44 (a), except for using 2-fluoro-4-nitrobenzoic acid as a starting material.

### (b) Synthesis of 4-amino-2-fluorobenzamide

Tin chloride (7.89 g, 35.0 mmol) was added to a solution of 2-fluoro-4-nitrobenzamide (1.84 g, 9.99mmol) in ethanol (60 ml), and the resulting mixture was stirred for 4 hours under refluxing conditions. After completion of the reaction, the solvent was distilled off and the resulting residue was diluted with ethyl acetate, followed by adding thereto a saturated aqueous sodium hydrogencarbonate solution. The resulting suspension was filtered by the use of Celite and the filtrate was extracted with ethyl acetate. The organic layer was washed with a saturated aqueous sodium chloride solution, dried over magnesium sulfate and then filtered. The filtrate was concentrated and the crude product thus obtained was purified by a silica gel column chromatography (eluent: chloroform/methanol = 100/1) to obtain 4-amino-2-fluorobenzamide (1.06 g, 69%).

### (c) Synthesis of tert-butyl 4-{[4-(aminocarbonyl)-3-fluorophenyl]amino}piperidine-1-carboxylate

The title compound was synthesized by carrying out reaction according to the method described in Example 45 (a), except for using 4-amino-2-fluorobenzamide in place of p-aminobenzamide.

### (d) Synthesis of 2-fluoro-4-(piperidin-4-ylamino)benzamide

The title compound was synthesized by carrying out reaction according to the method described in Example 45 (c), except for using tert-butyl 4-{[4-(aminocarbonyl)-3-fluorophenyl]amino}piperidine-1-carboxylate as a starting material.
¹H-NMR (DMSO-d₆) δ ; 1.29-1.40 (2H, m), 1.8 5-1.92 (2H, m) , 2.69-2.77 (2H, m), 3.06-3. 11 (2H, m) , 3.3 (1H, m) , 6.33(1H, dd, J=2.0, 15.0Hz), 6.43 (1H, dd, J=2.4, 8.8Hz) , 6.46 (1H, d, J=8.1Hz), 7.00(1H, br s ) , 7.18(1H, br s), 7.51 (1H, t, J=8.8Hz).

### Example 66

### Synthesis of 4-[(1-benzylpiperidin-4-yl)amino]-2-fluorobenzamide

The title compound was synthesized by carrying out reaction according to the method described in Example 10, except for using the 4-amino-2-fluorobenzamide obtained in Example 65 (b), in place of p-aminobenzamide.
¹H-NMR (DMSO-d₆) δ;1.32-1.42 (2H, m) , 1.8 4-1.87 (2H, m), 2.04-2.11 (2H, m) , 2.73-2. 78 (2H, m) , 3.28-3.36 (1H, m), 3.46 (2H, s) , 6.30 (1H, dd, J=2.0,15.2Hz), 6.38 (1H, d, J =7.9Hz), 6.38(1H, dd, J=2.0, 8.8 Hz), 6.97 (1H, br s) , 7.15 (1H, br s), 7.21-7.34 (5H, m), 7.49 (1H, t, J=8.8Hz).

### Example 67

Synthesis of 4-[(1-benzylpiperidin-4-yl)amino]-2-fluorobenzamide hydrochloride

The title compound was synthesized by carrying out reaction according to the method described in Example 11, except for using the 4-[(1-benzylpiperidin-4-yl)amino]-2-fluorobenzamide obtained in Example 66, as a starting material.

### Example 68

### Synthesis of 3-fluoro-4-(piperidin-4-ylamino)benzamide

### (a) Synthesis of 3-fluoro-4-nitrobenzoic acid

Sodium dichromate (6.50 g, 21.8 mmol) was added to a suspension of 3-fluoro-4-nitrotoluene (2.50 g, 16.0 mmol) in water (19 ml) under ice-cooling, and concentrated sulfuric acid (14.7 ml) was added dropwise thereto at 6°C over a period of 2 hours. After completion of the dropwise addition, an ice-water bath was removed and the mixture obtained was allowed to stand for 20 minutes until its temperature became room temperature. Thereafter, the mixture was heated and then stirred at 90°C for 2 hours. After completion of the reaction, water (50 ml) was poured into the reaction mixture under ice-cooling, and the resulting mixture was stirred at room temperature for a while. This mixture was extracted twice with ethyl acetate and then the organic layer was washed three times with a 2N-aqueous sodium hydroxide solution. The aqueous layer was acidified with 6N-hydrochloric acid and extracted with ethyl acetate. The organic layer was washed with a saturated aqueous sodium chloride solution, dried over magnesium sulfate and then filtered. The filtrate was concentrated and the crude product thus obtained was purified by a silica gel column chromatography (eluent: chloroform/methanol = 8/2) to obtain 3-fluoro-4-nitrobenzoic acid (2.00 g, 68%).

### (b) Synthesis of 3-fluoro-4-nitrobenzamide

The title compound was synthesized by carrying out reaction according to the method described in Example 44 (a), except for using 3-fluoro-4-nitrobenzoic acid as a starting material.

### (c) Synthesis of 4-amino-3-fluorobenzamide

The title compound was synthesized by carrying out reaction according to the method described in Example 65 (b), except for using 3-fluoro-4-nitrobenzamide as a starting material.

### (d) Synthesis of tert-butyl 4-{[4-(aminocarbonyl)-2-fluorophenyl)amino}piperidine-1-carboxylate

The title compound was synthesized by carrying out reaction according to the method described in Example 45 (a), except for using 4-amino-3-fluorobenzamide in place of p-aminobenzamide.

### (e) Synthesis of 3-fluoro-4-(piperidin-4-ylamino)benzamide

The title compound was synthesized by carrying out reaction according to the method described in Example 45 (c), except for using tert-butyl 4-{[4-(aminocarbonyl)-2-fluorophenyl]amino}piperidine-1-carboxylate as a starting material.
¹H-NMR (DMSO-d₆) δ ; 1.39-1.50 (2H, m) , 1.8 5-1.98 (2H, m), 2.67-2.74 (2H, m) , 3.06-3. 10 (2H, m), 5.78-5.80(1H, m) , 6.74-6.80(1 H, m) , 7.04 (1H, br s), 7.50-7.55 (2H, m), 7. 66 (1H, br s).

### Example 69

### Synthesis of 4-[(1-benzylpiperidin-4-yl)amino]-3-fluorobenzamide

The title compound was synthesized by carrying out reaction according to the method described in Example 10, except for using the 4-amino-3-fluorobenzamide obtained in Example 68 (c), in place of p-aminobenzamide.
¹H-NMR (DMSO-d₆) δ ; 1.45-1.55 (2H, m) . 1.8 2-1.85 (2H, m), 2.02-2.09(2H, m), 2.77-2. 81 (2H, m), 3.3 (1H, m) , 3.46 (2H, s), 5.65-5. 67 (1H, m) , 6.71-6.77 (1H, m) , 7.02 (1H, br s), 7.21-7.34(6H, m) , 7.49-7.54(2H, m) , 7. 65 (1H, br s).

### Example 70

Synthesis of 2-(methylthio)-4-(piperidin-4-ylamino)benzamide

### (a) Synthesis of 4-bromo-3-(methylthio)aniline

The title compound was synthesized by carrying out reaction according to the method described in Example 59 (a), except for using 3-methylmercaptoaniline as a starting material.

### (b) Synthesis of tert-butyl 4-bromo-3-(methylthio)-phenylcarbamate

The title compound was synthesized by carrying out reaction according to the method described in Example 59 (b), except for using 4-bromo-3-(methylthio)aniline as a starting material.

### (c) Synthesis of 4-amino-2-(methylthio)benzonitrile

The title compound was synthesized by carrying out reaction according to the method described in Example 59 (c), except for using tert-butyl 4-bromo-3-(methylthio)phenylcarbamate as a starting material.

### (d) Synthesis of 4-amino-2-(methylthio)benzamide

The title compound was synthesized by carrying out reaction according to the method described in Example 59 (d), except for using 4-amino-2-(methylthio)benzonitrile as a starting material.

### (e) Synthesis of tert-butyl 4-{[4-(aminocarbonyl)-3-(methylthio)phenyl]amino}piperidine-1-carboxylate

The title compound was synthesized by carrying out reaction according to the method described in Example 45 (a), except for using 4-amino-2-(methylthio)benzamide in place of p-aminobenzamide.

### (f) Synthesis of 2-(methylthio)-4-(piperidin-4-ylamino)benzamide

The title compound was synthesized by carrying out reaction according to the method described in Example 45 (c), except for using tert-butyl 4-{[4-(aminocarbonyl)-3-(methylthio)phenyl]amino}piperidine-1-carboxylate as a starting material.
¹H-NMR (DMSO-d₆) δ;1.26-1.36 (2H, m) , 1.8 7-1.90 (2H, m), 2.25 (3H, s), 2.65-2.72 (2H, m), 3.02-3.06 (2H, m) , 3.3 (1H, m) , 6.06 (1H, d, J=8.3Hz) , 6.29 (1H, dd, J=2.2,8.6Hz) , 6. 43 (1H, d, J=2.0Hz), 6. 8 (1H, br s) , 7.3 (1H, br s) , 7.38 (1H, d, J=8.4Hz).

### Example 71

### Synthesis of 4-[(1-benzylpiperidin-4-yl)amino]-2-(methylthio)benzamide

The title compound was synthesized by carrying out reaction according to the method described in Example 10, except for using the 4-amino-2-(methylthio)benzamide obtained in Example 70 (d), in place of p-aminobenzamide.
¹H-NMR (DMSO-d₆) δ ; 1.32-1.42 (2H, m) , 1.8 5-1.89 (2H, m), 2.04-2.11(2H, m), 2.24 (3H, s) , 2.74-2.78 (2H, m) , 3. 28-3.36 (1H, m) , 3. 46 (2H, s) , 6.00 (1H, d, J=7.9Hz), 6.27 (1H, dd, J=2.0, 8.6Hz) , 6.41 (1H, d, J=1.8Hz) , 6. 7 (1H, br s) , 7.21-7.38 (7H, m).

### Example 72

### Synthesis of 4-[(1-benzylpiperidin-4-yl)amino]-2-ethoxybenzamide

### (a) Synthesis of 4-bromo-3-ethoxyaniline

The title compound was synthesized by carrying out reaction according to the method described in Example 59 (a), except for using m-phenetidine as a starting material.

### (b) Synthesis of tert-butyl 4-bromo-3-ethoxyphenylcarbamate

The title compound was synthesized by carrying out reaction according to the method described in Example 59 (b), except for using 4-bromo-3-ethoxyaniline as a starting material.

### (c) Synthesis of 4-amino-2-ethoxybenzonitrile

The title compound was synthesized by carrying out reaction according to the method described in Example 59 (c), except for using tert-butyl 4-bromo-3-ethoxyphenylcarbamate as a starting material.

### (d) Synthesis of 4-amino-2-ethoxybenzamide

The title compound was synthesized by carrying out reaction according to the method described in Example 59 (d), except for using 4-amino-2-ethoxybenzonitrile as a starting material.

### (e) Synthesis of 4-[(1-benzylpiperidin-4-yl)amino]-2-ethoxybenzamide

The title compound was synthesized by carrying out reaction according to the method described in Example 10, except for using 4-amino-2-ethoxybenzamide in place of p-aminobenzamide.
¹H-NMR (DMSO-d₆) δ ; 1.37 (3H, t, J=7.0Hz), 1.35-1.43 (2H, m), 1.85-1.88 (2H, m) , 2.04 -2.11(2H, m) , 2.73-2.77(2H, m) , 3.3 (1H, m) , 3.46 (2H, s) , 4.08 (2H, q, J=7.0Hz), 6.1 1 (d, J=7.9Hz, 1H), 6.18-6.21(2 H, m) , 7.06 (1H, br s) , 7.21-7.34 (6H, m) , 7.65 (1H, d, J=9.0Hz).

### Example 73

### Synthesis of 2-ethoxy-4-(piperidin-4-ylamino)benzamide

The title compound was synthesized by carrying out reaction according to the method described in Example 14, except for using the 4-[(1-benzylpiperidin-4-yl)amino]-2-ethoxybenzamide obtained in Example 72, as a starting material.
¹H-NMR (DMSO-d₆) δ ; 1.25-1.40(2H, m) , 1. 3 8 (3H, t, J=7.0Hz), 1.86-1.90 (2H, m), 2.62 - 2.69 (2H, m) , 3.00-3.04 (2H, m) , 4.08 (2H, q, J=7.0Hz), 6.16-6.23 (3H, m) , 7.07 (1H, b r s), 7.30 (1H, br s) , 7.66 (1H, d, J=8.8H z) .

### Example 74

### Synthesis of 5-chloro-2-methoxy-4-(piperidin-4-ylamino)benzamide

### (a) Synthesis of 4-amino-2-methoxybenzonitrile

The title compound was synthesized by carrying out reaction according to the method described in Example 65 (b), except for using 2-methoxy-4-nitrobenzonitrile as a starting material.

### (b) Synthesis of 4-amino-2-methoxybenzamide

The title compound was synthesized by carrying out reaction according to the method described in Example 59 (d), except for using 4-amino-2-methoxybenzonitrile as a starting material.

### (c) Synthesis of tert-butyl 4-{[4-(aminocarbonyl)-3-methoxyphenyl]amino}piperidine-1-carboxylate

The title compound was synthesized by carrying out reaction according to the method described in Example 45 (a), except for using 4-amino-2-methoxybenzamide in place of p-aminobenzamide.

### (d) Synthesis of tert-butyl 4-{[4-(aminocarbonyl)-2-chloro-5-methoxyphenyl]amino}piperidine-1-carboxylate and tert-butyl 4-{[4-(aminocarbonyl)-2-chloro-3-methoxyphenyl]amino}piperidine-1-carboxylate

To a solution of tert-butyl 4-{[4-(aminocarbonyl)-3-methoxyphenyl]amino}piperidine-1-carboxylate (350 mg, 1.00 mmol) in 2-propanol (8 ml) was added N-bromosuccinimide (134 mg, 1.00 mmol) at room temperature, and stirred at 60°C for 1 hour. After completion of the reaction, the reaction mixture was concentrated and the resulting residue was washed with water and extracted with ethyl acetate. The organic layer was dried over magnesium sulfate and filtered. The filtrate was concentrated and the crude product thus obtained was purified by a silica gel column chromatography (eluent: hexane/ethyl acetate = 1/1) and preparative TLC (developed with: hexane/ethyl acetate = 1/1; two runs of developing operation) to obtain tert-butyl 4-{[4-(aminocarbonyl)-2-chloro-5-methoxyphenyl]amino}piperidine-1-carboxylate (267 mg, 70%) and tert-butyl 4-{[4-(aminocarbonyl)-2-chloro-3-methoxyphenyl]amino}piperidine-1-carboxylate (51 mg, 13%).

### (e) Synthesis of 5-chloro-2-methoxy-4-(piperidin-4-ylamino)benzamide

The title compound was synthesized by carrying out reaction according to the method described in Example 45 (c), except for using tert-butyl 4-{[4-(aminocarbonyl)-2-chloro-5-methoxyphenyl]amino}-piperidine-1-carboxylate as a starting material.
¹H-NMR (DMSO-d₆) δ ; 1.45-1.57(2H, m) , 1.9 1-1.94 (2H, m) , 2.71-2.78 (2H, m) , 3.07-3. 11(2H, m) , 3.61(1H, m) , 3.89(3H, s), 5.41 (1H, d, J=8.1Hz), 6.36 (1H, s) , 7.27 (1H, br s), 7.40(1H, br s), 7.74(1H, s).

### Example 75

### Synthesis of 5-chloro-2-methoxy-4-(piperidin-4-ylamino)benzamide dihydrochloride

A 1N-hydrochloric acid/ether solution (3.1 ml, 3.1 mmol) was added to a solution of the 5-chloro-2-methoxy-4-(piperidin-4-ylamino)benzamide obtained in Example 74 (350 mg, 1.23 mmol) in a mixture of ethanol (3 ml) and methanol (4 ml) at room temperature and stirred for 30 minutes. The resulting suspension was filtered and the precipitate obtained was dried to obtain 5-chloro-2-methoxy-4-(piperidin-4-ylamino)benzamide dihydrochloride (202 mg, 46%).

### Example 76

### Synthesis of 4-[(1-benzylpiperidin-4-yl)amino]-5-chloro-2-methoxybenzamide

The title compound was synthesized by carrying out reaction according to the method described in Example 46, except for using the 5-chloro-2-methoxy-4-(piperidin-4-ylamino)benzamide obtained in Example 74, as a starting material.
¹H-NMR (DMSO-d₆) δ ; 1.55-1.62 (2H, m), 1. 8 7-1.90 (2H, m) , 2.09-2.16(2H, m) , 2.75-2. 79 (2H, m) , 3.48(1H, m), 3.48(2H, s), 3.88 (3H, s) , 5.29 (1H, d, J=8.1Hz), 6.31 (1H, s), 7.21-7.38(6H, m) , 7.38 (1H, br s), 7.72(1 H, s).

### Example 77

### Synthesis of 4-[(1-benzylpiperidin-4-yl)amino]-5-chloro-2-methoxybenzamide dihydrochloride

The title compound was synthesized by carrying out reaction according to the method described in Example 11, except for using the 4-[(1-benzylpiperidin-4-yl)amino]-5-chloro-2-methoxybenzamide obtained in Example 76, as a starting material.

### Example 78

### Synthesis of 3-chloro-2-methoxy-4-(piperidin-4-ylamino)benzamide

The title compound was synthesized by carrying out reaction according to the method described in Example 45 (c), except for using the tert-butyl 4-{[4-(aminocarbonyl)-2-chloro-3-methoxyphenyl]amino}-piperidine-1-carboxylate obtained in Example 74 (d), as a starting material.
¹H-NMR (DMSO-d₆) δ ; 1.40-1.51(2H, m) , 1.8 5-1.89 (2H, m), 2.63-2.70 (2H, m) , 3.01 -3. 05 (2H, m) , 3.47-3.50(1H, m) , 3.76 (3H, s) , 5.40(1H, d, J=8.3Hz) , 6.65 (1H, d, J=9.2H z) , 7.31(1H, br s) , 7.41(1H, br s), 7.59 (1H, d, J=8.8Hz).

### Example 79

### Synthesis of 4-[(1-benzylpiperidin-4-yl)amino]-3-chloro-2-methoxybenzamide

The title compound was synthesized by carrying out reaction according to the method described in Example 46, except for using the 3-chloro-2-methoxy-4-(piperidin-4-ylamino)benzamide obtained in Example 78, as a starting material.
¹H-NMR (DMSO-d₆) δ;1.50-1.61 (2H, m) , 1.8 3-1.86 (2H, m) , 2.05-2.12 (2H, m) , 2.75-2. 79 (2H, m) , 3.36 (1H, m) , 3.46 (2H, s) , 3.76 (3H, s) , 5.33 (1H, d, J=8.1Hz), 6.63 (1H, d, J=9.0Hz), 7.21-7.34 (6H, m), 7.40 (1H, br s) , 7.58 (1H, d, J=8.8Hz).

### Example 80

### Synthesis of 4-[(1-methylpiperidin-4-yl)amino]benzamide

The title compound was synthesized by carrying out reaction according to the method described in Example 10, except for using 1-methyl-4-piperidone in place of 1-benzyl-4-piperidone.
¹H-NMR (DMSO-d₆) δ;1.31-1.44 (2H, m), 1.8 3-1.87 (2H, m) , 1.95-2.02 (2H, m) , 2.15 (3H, s), 2.69-2.72 (2H, m) , 3.16-3.25 (1H, m) , 5. 99 (1H, d, J=7.9Hz) , 6.52 (2H, d, J=8.8Hz) , 6.81 (1H, br s), 7.50 (1H, br s), 7.60 (2H, d, J=8.8Hz).

### Example 81

### Synthesis of 3-chloro-4-[(1-methylpiperidin-4-yl)amino]benzamide

The title compound was synthesized by carrying out reaction according to the method described in Example 45 (b), except for using the 4-[(1-methylpiperidin-4-yl)amino]benzamide obtained in Example 80, as a starting material.
¹H-NMR (DMSO-d₆) δ;1.49-1.61 (2H, m) , 1.8 2-1.85 (2H, m) , 1.97-2.04 (2H, m) , 2.15 (3H, s) , 2.69-2.73 (2H, m) , 3.32-3.45 (1H, m) , 5. 26 (1H, d, J=8.3Hz) , 6.77 (1H, d, J=8.8Hz), 7.06 (1H, br s), 7.67 (1H, d, J=1.8, 8.8Hz), 7.71 (1H, br s), 7.80 (1H, d, J=1.8Hz).

### Example 82

### Synthesis of 3-chloro-4-[(1-methylpiperidin-4-yl)amino]benzamide dihydrochloride

The title compound was synthesized by carrying out reaction according to the method described in Example 11, except for using the 3-chloro-4-[(1-methylpiperidin-4-yl)amino]benzamide obtained in Example 81, as a starting material.

### Example 83

### Synthesis of 3-bromo-4-[(1-methylpiperidin-4-yl)amino]benzamide

The title compound was synthesized by carrying out reaction according to the method described in Example 63 (a), except for using the 4-[(1-methylpiperidin-4-yl)amino]benzamide obtained in Example 80, as a starting material.
¹H-NMR (DMSO-d₆) δ ; 1.48-1.60 (2H, m) , 1.8 4-1.89 (2H, m), 2.04-2.11 (2H, m) , 2.18(3H, s), 2.69-2.73(2H, m) , 3.40 (1H, m) , 5.01(1 H, d, J=7.9Hz) , 6.76 (1H, d, J=8. 8Hz) , 7.05 (1H, br s) , 7.71(1H, dd, J=2.0, 8.6Hz) , 7. 73 (1H, br s) , 7. 97 (1H, d, J=2.0Hz).

### Example 84

### Synthesis of 2-fluoro-4-[(1-methylpiperidin-4-yl)amino]benzamide

The 4-amino-2-fluorobenzamide obtained in Example 65 (b) (308 mg, 2.00 mmol), sodium triacetoxyborohydride (1.13 g, 5.01 mmol) and acetic acid (0.23 ml, 4.02 mmol) were added to a solution of 1-methyl-4-piperidone (0.49 ml, 3.98 mmol) in 1,2-dichloroethane (14 ml), and the resulting mixture was stirred overnight at room temperature. After completion of the reaction, the reaction mixture was poured into a 1N-aqueous sodium hydroxide solution under ice-cooling and extracted with ethyl acetate. The organic layer was washed with a saturated aqueous sodium chloride solution, dried over magnesium sulfate and then filtered. The filtrate was concentrated and the crude product thus obtained was purified by a silica gel column chromatography (eluent: chloroform/methanol/aqueous ammonia = 90/10/1) to obtain 2-fluoro-4-[(1-methylpiperidin-4-yl)amino]benzamide (287 mg, 57%).
¹H-NMR (DMSO-d₆) δ;1.31-1.43 (2H, m) , 1.8 2-1.86 (2H, m), 1.92-2.03 (2H, m), 2.15 (3H, s) , 2.68-2.72 (2H, m) , 3.3 (1H, m), 6.30 (1H, dd, J=2.0,15.2Hz), 6.37 (1H, d, J=8.1Hz), 6.41 (1H, dd, J=2.2,8.1Hz), 6.99 (1H, br s) , 7.17 (1H, br s) , 7. 50 (1H, t, J=8.8Hz).

### Example 85

### Synthesis of 4-(benzylamino)-2-methoxybenzamide

### (a) Synthesis of methyl 4-(benzylamino)-2-methoxybenzoate

Benzaldehyde (6.73 ml, 66.2 mmol) and acetic acid (3.77 ml, 66.2 mmol) were added to a solution of methyl 4-amino-2-methoxybenzoate (12 g, 66.2 mmol) in methanol (650 ml), and the resulting mixture was stirred at room temperature for 30 minutes. Then, acetic acid (7.56 ml, 132 mmol) and sodium cyanoborohydride (4.9 g, 79.4 mmol) were added thereto at 0°C and stirred at room temperature for 38 hours. The reaction mixture was concentrated, poured into a 1N-aqueous sodium hydroxide solution and then extracted with ethyl acetate. The organic layer was washed with a saturated aqueous sodium chloride solution, dried over anhydrous sodium sulfate and then filtered. The filtrate was concentrated under reduced pressure and the resulting residue was purified by a silica gel column chromatography (eluent: hexane/ethyl acetate = 2/1) to obtain methyl 4-(benzylamino)-2-methoxybenzoate (16.4 g, 91%).

### (b) Synthesis of 4-(benzylamino)-2-methoxybenzamide

Formamide (11.6 ml, 294 mmol) and 28% sodium methoxide/methanol (56.7 g, 294 mmol) were added to a solution of methyl 4-(benzylamino)-2-methoxybenzoate (16 g, 58.9 mmol) in dimethylformamide (300 ml), and the resulting mixture was stirred with heating at 70°C for one and a half hours. The reaction mixture was neutralized with a 4N-hydrochloric acid solution and distilled under reduced pressure to remove the solvent. The residue was extracted with ethyl acetate and the extract solution was washed with a saturated aqueous sodium chloride solution, and the organic layer was dried over magnesium sulfate and filtered. The filtrate was concentrated under reduced pressure and the solid precipitated was washed by repulping with ether-hexane and a small volume of methanol to obtain 4-(benzylamino)-2-methoxybenzamide (11.4 g). On the other hand, the solid precipitated during the extraction was separately collected by filtration and washed by repulping with water, a diethyl ether-hexane mixture and methanol to obtain 4-(benzylamino)-2-methoxybenzamide (1.29 g, total amount 12.7 g, 84%).

Melting point: 175-176°C.

### Example 86

### Synthesis of 2-methoxy-4-[(1-methylpiperidin-4-yl)amino]benzamide

### (a) Synthesis of 4-amino-2-methoxybenzamide

Ammonium formate (4.5 g) and 10% palladium/carbon (450 mg) were added to a solution of the 4-(benzylamino)-2-methoxybenzamide obtained in Example 85 (4.5 g, 17.5 mmol) in ethanol (450 ml), and the resulting mixture was heated under reflux for 3 hours. The reaction mixture was filtered and the filtrate was concentrated under reduced pressure to obtain 4-amino-2-methoxybenzamide as a crude product. The same reaction as above was carried out once more for batch production. The combined crude product was washed with ethanol-hexane by repulping to obtain 4-amino-2-methoxybenzamide (5.67 g, 97%).

### (b) Synthesis of 2-methoxy-4-[(1-methylpiperidin-4-yl)amino]benzamide

To a solution of the 4-amino-2-methoxybenzamide obtained in Example 74 (b) or Example 86 (a) (150 mg, 0.902 mmol) in dichloroethane were added 1-methyl-4-piperidone (0.22 ml, 1.80 mmol), acetic acid (0.103 ml, 1.80 mmol) and sodium triacetoxyborohydride (477 mg, 2.26 mmol), and the resulting mixture was stirred at room temperature for fourteen and a half hours. The reaction mixture was poured into a 1N-aqueous sodium hydroxide solution and extracted with ethyl acetate. The organic layer was dried over anhydrous magnesium sulfate and filtered. The filtrate was concentrated to obtain 2-methoxy-4-[(1-methylpiperidin-4-yl)amino]benzamide as a crude product. On the other hand, the solid precipitated during the extraction was collected by filtration to obtain 2-methoxy-4-[(1-methylpiperidin-4-yl)amino]benzamide (87 mg, 37%).
IR (neat) : 3482, 3448, 3266, 3151, 2939, 16 39, 1589, 1577, 1361, 1211, 1103, 1095cm⁻¹.

### Example 87

Synthesis of 5-chloro-2-methoxy-4-[(1-methylpiperidin-4-yl)amino]benzamide

The title compound was synthesized by carrying out reaction according to the method described in Example 45 (b), except for using the 2-methoxy-4-[(1-methylpiperidin-4-yl)amino]benzamide obtained in Example 86, as a starting material.
¹H-NMR (DMSO-d₆) δ ; 1.51-1.63 (2H, m) , 1.8 4-1.88 (2H, m) , 2.01-2.08 (2H, m) , 2.16 (3H, s), 2.69-2.73 (2H, m) , 3.44-3.46 (1H, m) , 3. 89 (3H, s) , 5.30 (1H, d, J=8.3Hz), 6.31 (1H, s), 7.28(1H, br s), 7.39(1H, br s), 7.73 (1H, s).

### Example 88

Synthesis of 5-chloro-2-methoxy-4-[(1-methylpiperidin-4-yl)amino]benzamide dihydrochloride

The title compound was synthesized by carrying out reaction according to the method described in Example 11, except for using the 5-chloro-2-methoxy-4-[(1-methylpiperidin-4-yl)amino]benzamide obtained in Example 87, as a starting material.

### Example 89

### Synthesis of 4-[(1-benzylpiperidin-4-yl)(methyl)amino]-2-methoxybenzamide

The 4-[(1-benzylpiperidin-4-yl)amino]-2-methoxybenzamide obtained in Example 28 (340 mg, 1.00 mmol), sodium cyanoborohydride (315 mg, 5.01 mmol) and acetic acid (0.29 ml, 5.07 mmol) were added to a solution of paraformaldehyde (670 mg, 8.00 mmol) in methanol (6 ml), and the resulting mixture was stirred overnight at room temperature. After completion of the reaction, the solvent was distilled off and to the resulting residue was added a saturated aqueous sodium hydrogencarbonate solution, followed by extraction with ethyl acetate. The organic layer was washed with a saturated aqueous sodium chloride solution, dried over magnesium sulfate and then filtered. The filtrate was concentrated and the crude product thus obtained was purified by a silica gel column chromatography (eluent: chloroform/methanol = 99/1) and preparative TLC (developed with: chloroform/methanol/aqueous ammonia = 90/10/1) to obtain 4-[(1-benzylpiperidin-4-yl)(methyl)amino]-2-methoxybenzamide (170 mg, 49%).
¹H-NMR (DMSO-d₆) δ;1.57-1.60 (2H, m) , 1.6 9-1.80 (2H, m) , 2.07-2.14 (2H, m) , 2.80 (3H, s) , 2.85-2.89 (2H, m), 3.48 (2H, s) , 3.67-3. 75 (1H, m) , 3.88 (3H, s) , 6.26 (1H, d, J=2. 0H z), 6.43 (1H, dd, J=2. 0, 8.8Hz) , 7.10 (1H, b r s) , 7.23-7.36 (5H, m) , 7.72 (2H, d, J=8.8 Hz) .

### Example 90

### Synthesis of 2-methoxy-4-[methyl(piperidin-4-yl)amino]benzamide

The title compound was synthesized by carrying out reaction according to the method described in Example 14, except for using the 4-[(1-benzylpiperidin-4-yl)(methyl)amino]-2-methoxybenzamide obtained in Example 89, as a starting material.
¹H-NMR (DMSO-d₆) δ ; 1.62-1.80 (4H, m), 2.7 7-2.84 (2H, m), 2.79 (3H, s) , 3.13-3.17 (2H, m) , 3.85-3.96 (1H, m) , 3.89 (3H, s) , 6.30 (1 H, d, J=2.2Hz) , 6.47 (1H, dd, J=2.4, 9.0Hz), 7.12 (1H, br s), 7.37 (1H, br s), 7.73 (1H, d, J=8.8Hz).

### Example 91

### Synthesis of 2-methoxy-4-[methyl(1-methylpiperidin-4-yl)amino]benzamide

The title compound was synthesized by carrying out reaction according to the method described in Example 89, except for using the 2-methoxy-4-[methyl(piperidin-4-yl)amino]benzamide obtained in Example 89, as a starting material.
¹H-NMR (DMSO-d₆) δ;1.54-1.58 (2H, m), 1.6 9-1.80 (2H, m) , 2.00-2.07 (2H, m) , 2.17 (3H, s), 2.79 (3H, s), 2.79-2.84 (2H, m), 3.62-3. 70 (1H, m) , 3.89 (3H, s) , 6.26 (1H, d, J=2.2H z), 6.42 (1H, dd, J=2.4,9.0Hz), 7.09 (1H, b r s), 7.36 (1H, br s), 7.72 (1H, d, J=9.0H z) .

### Example 92

### Synthesis of 3-chloro-4-[methyl(piperidin-4-yl)amino]benzamide

### (a) Synthesis of tert-butyl 4-[[4-(aminocarbonyl)-2-chlorophenyl](methyl)amino]piperidine-1-carboxylate

The title compound was synthesized by carrying out reaction according to the method described in Example 89, except for using the tert-butyl 4-{[4-(aminocarbonyl)-2-chlorophenyl]amino}piperidine-1-carboxylate obtained in Example 45 (b), as a starting material.

### (b) Synthesis of 3-chloro-4-[methyl(piperidin-4-yl)amino]benzamide

The title compound was synthesized by carrying out reaction according to the method described in Example 45 (c), except for using tert-butyl 4-[[4-(aminocarbonyl)-2-chlorophenyl](methyl)amino]-piperidine-1-carboxylate as a starting material.
¹H-NMR (DMSO-d₆) δ ; 1.54-1.59 (4H, m) , 2.1 8 (1H, br s) , 2.33-2.42 (2H, m), 2.66(3H, s) , 2.93-2.97(2H, m) , 3.21-3.26 (1H, m) , 7. 16 (1H, d, J=8.6Hz) , 7.28 (1H, br s), 7.74 (1H, dd, J=2.0, 8.6Hz), 7.87 (1H, d, J=2.0H z) , 7.89 (1H, br s).

### Example 93

### Synthesis of 2-methoxy-4-[(1-propylpiperidin-4-yl)amino]benzamide

The title compound was synthesized by carrying out reaction according to the method described in Example 86 (b), except for using 1-propyl-4-piperidone in place of 1-methyl-4-piperidone.
IR (neat) :3509, 3448, 3259, 3147, 2954, 16 50, 1577, 1369, 1207, 1103, 1087cm⁻¹.

### Example 94

### Synthesis of 4-[(1-acetylpiperidin-4-yl)amino]-2-methoxybenzamide

The title compound was synthesized by carrying out reaction according to the method described in Example 86 (b), except for using N-acetyl-4-piperidone in place of 1-methyl-4-piperidone.
IR (neat) :3452, 3317, 3155, 2946, 1650, 15 85, 1573, 1423, 1334, 1207cm⁻¹.

### Example 95

### Synthesis of 4-[(1-benzoylpiperidin-4-yl)amino]-2-methoxybenzamide

To a solution of the 4-amino-2-methoxybenzamide obtained in Example 74 (b) or Example 86 (a) (150 mg, 0.902 mmol) in methanol (7.5 ml) were added 1-benzoyl-4-piperidone (183 mg, 0.902 mmol) and acetic acid (0.052 ml, 0.90 mmol), and the resulting mixture was stirred at room temperature for 30 minutes. Then, acetic acid (0.103 ml, 1.80 mmol) and sodium cyanoborohydride (130 mg, 2.06 mmol) were added thereto, followed by stirring at room temperature for 31 hours. During this stirring, 1-benzoyl-4-piperidone (183 mg, 0.902 mmol), sodium cyanoborohydride (208 mg, 3.30 mmol) and acetic acid (0.154 ml, 2.70 mmol) were further added thereto. The reaction mixture was poured into a 1N-aqueous sodium hydroxide solution and extracted with ethyl acetate. The organic layer was dried over anhydrous magnesium sulfate and filtered. The filtrate was concentrated under reduced pressure and the resulting residue was purified by a silica gel column chromatography (eluent: ethyl acetate/methanol = 100/1 to 50/1) to obtain 4-[(1-benzoylpiperidin-4-yl)amino]-2-methoxybenzamide (97 mg, 30%).
IR (neat) : 3455, 3309, 2939, 1604, 1573, 14 23, 1338, 1211cm⁻¹.

### Example 96

### Synthesis of 2-methoxy-4-{[1-(methylsulfonyl)piperidin-4-yl]amino}benzamide

The title compound was synthesized by carrying out reaction according to the method described in Example 86 (b), except for using 1-(methylsulfonyl)-piperidin-4-one in place of 1-methyl-4-piperidone.

Melting point: 210-212°C.

### Example 97

### Synthesis of 2-methoxy-4-{[1-(phenylsulfonyl)piperidin-4-yl]amino}benzamide

### (a) Synthesis of 8-(phenylsulfonyl)-1,4-dioxa-8-azaspiro[4.5]decane

Triethylamine (1.45 ml, 10.5 mmol) was added to a solution of 1,4-dioxa-8-azaspiro[4.5]-decane (1g, 6.98 mmol) in dichloromethane (5 ml), followed by adding thereto a solution of benzenesulonyl chloride (0.935 ml, 7.33 mmol) in dichloromethane (5 ml) at 0°C, and the resulting mixture was stirred at 0°C for 1 hour. The reaction mixture was poured into a saturated aqueous sodium hydrogencarbonate solution and then extracted with ethyl acetate. The organic layer was washed with a saturated aqueous sodium chloride solution, dried over anhydrous sodium sulfate and then filtered. The filtrate was concentrated under reduced pressure to obtain 8-(phenylsulfonyl)-1,4-dioxa-8-azaspiro[4.5]decane (1.97 g, quantitative yield).

### (b) Synthesis of 1-(phenylsulfonyl)piperidin-4-one

Concentrated hydrochloric acid (43 ml) was added to a solution of 8-(phenylsulfonyl)-1,4-dioxa-8-azaspiro-[4.5]decane (1.9 g, 6.70 mmol) in a water (15 ml)-acetic acid (15 ml) mixture, and the resulting mixture was stirred at room temperature for 4 hours. The reaction mixture was adjusted to pH 11 with an aqueous sodium hydroxide solution and then the solid precipitated was filtered. The precipitate on a filter was purified by a silica gel column chromatography (eluent: hexane/ethyl acetate = 3/1 to 2.5/1) to obtain 1-(phenylsulfonyl)piperidin-4-one (1.14 g, 71%).

### (c) Synthesis of 2-methoxy-4-{[1-(phenylsulfonyl)-piperidin-4-yl]amino}benzamide

The title compound was synthesized by carrying out reaction according to the method described in Example 86 (b), except for using 1-(phenylsulfonyl)piperidin-4-one in place of 1-methyl-4-piperidone.

Melting point: 166-168°C.

### Example 98

Synthesis of 4-[(1-benzylpiperidin-3-yl)amino]-2-methoxybenzamide

The title compound was synthesized by carrying out reaction according to the method described in Example 86 (b), except for using 1-benzyl-3-piperidone hydrochloride hydrate in place of 1-methyl-4-piperidone.
¹H-NMR (DMSO-d₆) δ ; 1.16-1.24 (1H, m), 1.5 2-1.88 (4H, m) , 2.00-2.06 (1H, s), 2.65-2. 69 (1H, m), 2.84-2.89 (1H, m), 3.37-3.58 (3 H, m) , 3.74 (3H, s) , 6.06-6.17(3H, m) , 7.01 (1H, brs), 7.21-7.31(6H, m), 7.61(1H, d, J =8.6Hz).

### Example 99

### Synthesis of 2-methoxy-4-(piperidin-3-ylamino)benzamide

The title compound was synthesized by carrying out reaction according to the method described in Example 14, except for using the 4-[(1-benzylpiperidin-3-yl)amino]-2-methoxybenzamide obtained in Example 98, as a starting material.
IR (neat) : 3448, 3301, 2935, 1608, 1577, 15 62, 1423, 1334, 1211, 1103cm⁻¹.

### Example 100

Synthesis of tert-butyl 4-{[4-(aminocarbonyl)-3-methoxyphenyl]amino}azepane-1-carboxylate

### (a) Synthesis of 1-benzyl-4-azepanone

N-methyl-N-nitrosourethane (1.39 ml, 10.8 mmol) was added dropwise to a solution of 1-benzyl-4-piperidone (2.0 g, 10.6 mmol) in methanol (4 ml) at -15°C over a period of 30 minutes while maintaining the temperature at -5°C or lower. During the addition, barium oxide (65 mg, 0.423 mmol) was added thereto in small portions. The resulting mixture was stirred overnight at -15°C and then filtered, and the filtrate was distilled under reduced pressure to remove the solvent and diethyl ether was added to the residue. The insoluble material was filtered off and a saturated aqueous sodium hydrogencarbonate solution was added to the filtrate, followed by extraction with diethyl ether. The organic layer was dried over anhydrous magnesium sulfate and distilled under reduced pressure to remove the solvent, and the resulting residue was purified by a silica gel column chromatography (eluent: hexane/ethyl acetate = 2/1) to obtain 1-benzyl-4-azepanone (662 mg, 31%).

### (b) Synthesis of 1-benzyl-4-azepanol

A solution of 1-benzyl-4-azepanone (610 mg, 3.00 mmol) in diethyl ether (8 ml) was added to a suspension of lithium aluminum hydride (57 mg, 1.50 mmol) in diethyl ether (5 ml) at 0°C and stirred for 1 hour. Water (0.057 ml), a 2N-aqueous sodium hydroxide solution (0.114 ml) and water (0.171 ml) were added to the reaction mixture, and the resulting mixture was dried over anhydrous magnesium sulfate and distilled under reduced pressure to remove the solvent. The resulting residue was purified by a silica gel column chromatography (eluents: hexane/ethyl acetate = 1/1 and chloroform/methanol = 30/1) to obtain 1-benzyl-4-azepanol (516 mg, 84%).

### (c) Synthesis of tert-butyl 4-hydroxyazepane-1-carboxylate

Ammonium formate (900 mg) and 10% palladium/carbon (200 mg) were added to a solution of 1-benzyl-4-azepanol (450 mg, 2.19 mmol) in ethanol (10 ml), and the resulting mixture was refluxed for 1 hour. The reaction mixture was filtered by the use of Celite and the filtrate was concentrated. To a solution of the resulting residue in dichloromethane (10 ml) was added di-tert-butyl dicarbonate (0.504 ml, 2.19 mmol) at room temperature, and stirred for 19 hours. A saturated aqueous sodium hydrogencarbonate solution and then water were added to the reaction mixture, followed by extraction with chloroform. The organic layer was dried over anhydrous magnesium sulfate and distilled under reduced pressure to remove the solvent, and the resulting residue was purified by a silica gel column chromatography (eluent: hexane/ethyl acetate = 2/1) to obtain tert-butyl 4-hydroxyazepane-1-carboxylate (333 mg, 70%).

### (d) Synthesis of tert-butyl 4-oxoazepane-1-carboxylate

Triethylamine (35 ml) and a sulfur trioxidepyridine complex (20 g, 130 mmol) were added to a solution of tert-butyl 4-hydroxyazepane-1-carboxylate (3.5 g, 16.3 mmol) in dimethyl sulfoxide (90 ml), and the resulting mixture was stirred at room temperature for three and a half hours. The reaction mixture was poured into water and extracted with ethyl acetate. The organic layer was washed with a saturated aqueous sodium chloride solution, dried over anhydrous magnesium sulfate and then filtered. The filtrate was concentrated under reduced pressure and the resulting residue was purified by a silica gel column chromatography (eluent: hexane/ethyl acetate = 4/1) to obtain tert-butyl 4-oxoazepane-1-carboxylate (2.80 g, 81%).

### (e) Synthesis of tert-butyl 4-{[4-(aminocarbonyl)-3-methoxyphenyl]amino}azepane-1-carboxylate

The title compound was synthesized by carrying out reaction according to the method described in Example 86 (b), except for using tert-butyl 4-oxoazepane-1-carboxylate in place of 1-methyl-4-piperidone.
IR (neat) : 3463, 3313, 2931, 1635, 1608, 15 81, 1415, 1160cm⁻¹.

### Example 101

### Synthesis of 4-(azepan-4-ylamino)-2-methoxybenzamide

The title compound was synthesized by carrying out reaction according to the method described in Example 45 (c), except for using the tert-butyl 4-{[4-(aminocarbonyl)-3-methoxyphenyl]amino}azepane-1-carboxylate obtained in Example 100, as a starting material.
¹H-NMR (DMSO-d₆) δ ; 1.33-1.67 (12H, m), 1. 76-1.87 (2H, m) , 1.93-2.02 (1H, m) , 3.17-3. 49 (5H, m) , 3.81 (3H, s), 6.14-6.22 (3H, m) , 7.03 (1H, d, J=2.3Hz) , 7.31(1H, d, J=2.3H z) , 7.66 (1H, d, J=9.0Hz).

### Example 102

### Synthesis of 4-[(1-benzylazepan-4-yl)amino]-2-methoxybenzamide

The title compound was synthesized by carrying out reaction according to the method described in Example 46, except for using the 4-(azepan-4-ylamino)-2-methoxybenzamide obtained in Example 101, as a starting material.
IR (neat) : 3455, 3309, 2931, 1 646, 1577, 15 58, 1338, 1211, 1103cm⁻¹.

### Example 103

### Synthesis of 4-[(8-benzyl-8-azabicyclo[3.2.1]oct-3-yl)amino]-2-methoxybenzamide

The title compound was synthesized by carrying out reaction according to the method described in Example 86 (b), except for using 8-benzyl-8-azabicyclo[3.2.1]octan-3-one in place of 1-methyl-4-piperidone.

Melting point: 138-140°C.

### Example 104

### Synthesis of 4-(8-azabicyclo[3.2.1]oct-3-ylamino)-2-methoxybenzamide

The title compound was synthesized by carrying out reaction according to the method described in Example 14, except for using the 4-[(8-benzyl-8-azabicyclo[3.2.1]oct-3-yl)amino]-2-methoxybenzamide obtained in Example 103, as a starting material.
IR (neat) : 3448, 3313, 3131, 2939, 1643, 15 85, 1342, 1211, 1099, 817cm⁻¹.

### Example 105

### Synthesis of 4-[(trans-4-aminocyclohexyl)-amino]-2-methoxybenzamide

### (a) Synthesis of trans-tert-butyl 4-[(4-cyano-3-methoxyphenyl)amino]cyclohexylcarbamate

A solution of 4-bromo-2-methoxybenzonitrile (500 mg, 2.35 mmol), trans-tert-butyl 4-aminocyclohexylcarbamate (556 mg, 2.59 mmol), sodium tert-butoxide (349 mg, 3.63 mmol), tris(dibenzylideneacetone)dipalladium(0) (21.5 mg, 0.0235 mmol) and 2,2'-bis(diphenylphosphino)-1,1'-binaphthyl (44.0 mg, 0.0707 mmol) in toluene (5 ml) was stirred at 80°C for three and a half hours. The reaction solution was concentrated under reduced pressure and the resulting residue was purified by a silica gel column chromatography (eluent: hexane/ethyl acetate = 3/1 to 2/1) and then washed by repulping to obtain trans-tert-butyl 4-[(4-cyano-3-methoxyphenyl)amino]cyclohexylcarbamate (560 mg, 69%).

### (b) Synthesis of 4-[(trans-4-aminocyclohexyl)amino]-2-methoxybenzamide

Concentrated sulfuric acid (4 ml) was added to trans-tert-butyl 4-[(4-cyano-3-methoxyphenyl)amino]-cyclohexylcarbamate (250 mg, 0.723 mmol), and the resulting mixture was stirred at 80°C for one and a half hours. The reaction mixture was poured onto ice, adjusted to pH 11 with a 10N-aqueous sodium hydroxide solution, and then extracted with ethyl acetate. The organic layer was dried over anhydrous magnesium sulfate and filtered. The filtrate was concentrated under reduced pressure and the resulting residue was purified by a silica gel column chromatography (eluent: chloroform/methanol/aqueous ammonia = 100/10/1) to obtain 4-[(trans-4-aminocyclohexyl)amino]-2-methoxybenzamide (85 mg, 45%).
IR (neat) : 3455, 3301, 2927, 1635, 1581, 15 62, 1423, 1338, 1211, 1103cm⁻¹.

### Example 106

### Synthesis of 4-[(cis-4-aminocyclohexyl)-amino]-2-methoxybenzamide

### (a) Synthesis of cis-4-[(tert-butoxycarbonyl)amino]-cyclohexanecarboxylic acid

An aqueous solution (30 ml) of sodium hydroxide (2.23 g, 55.8 mmol) and 1,4-dioxane (30 ml) were added to cis-4-aminocyclohexanecarboxylic acid (2 g, 13.9 mmol), followed by adding thereto di-tert-butyl dicarbonate (6.09 mmol, 27.9 mmol) at 0°C, and the resulting mixture was stirred at room temperature for fourteen and a half hours. The reaction mixture was poured into water and extracted with diethyl ether. The aqueous layer was adjusted to pH 4 with 5% potassium hydrogensulfate and then extracted with ethyl acetate. The ethyl acetate layer was washed with a saturated aqueous sodium chloride solution, dried over magnesium sulfate and then filtered. The filtrate was concentrated under reduced pressure to obtain cis-4-[(tert-butoxycarbonyl)amino]cyclohexanecarboxylic acid (3.17 g, 93%).

### (b) Synthesis of benzyl tert-butyl trans-cyclohexane-1,4-diylbiscarbamate

Triethylamine (2.06 ml, 14.8 mmol) and then diphenylphosphoryl azide (2.92 ml, 13.6 mmol) were added to a solution of cis-4-[(tert-butoxycarbonyl)-amino]cyclohexanecarboxylic acid (3.0 g, 12.3 mmol) in toluene (90 ml), and the resulting mixture was stirred at 80°C for one and a half hours. Benzyl alcohol (1.53 ml, 13.6 mmol) was added to the reaction mixture and the resulting mixture was heated under reflux for 19 hours. The reaction mixture was poured into water and extracted with ethyl acetate, and the combined organic layer was washed with a saturated aqueous sodium chloride solution, dried over anhydrous magnesium sulfate and then filtered. The filtrate was concentrated under reduced pressure and the resulting residue was purified by a silica gel column chromatography (eluents: chloroform/ethyl acetate = 40/1 and hexane/ethyl acetate = 4/1) to obtain benzyl tert-butyl trans-cyclohexane-1,4-diylbiscarbamate (2.6 g, 61%).

### (c) Synthesis of cis-tert-butyl 4-aminocyclohexylcarbamate

To a solution of benzyl tert-butyl transcyclohexane-1,4-diylbiscarbamate (2.6 g, 7.46 mmol) in ethanol (10 ml) was added 10% palladium/carbon (260 mg), and the resulting mixture was stirred at room temperature for 8 hours under a hydrogen atmosphere. The reaction mixture was filtered and the filtrate was concentrated to obtain cis-tert-butyl 4-aminocyclohexylcarbamate (1.6 g, quantitative yield).

### (d) Synthesis of cis-tert-butyl 4-[(4-cyano-3-methoxyphenyl)amino]cyclohexylcarbamate

The title compound was synthesized by carrying out reaction according to the method described in Example 105 (a), except for using cis-tert-butyl 4-aminocyclohexylcarbamate as a starting material in place of trans-tert-butyl 4-aminocyclohexylcarbamate.

### (e) Synthesis of 4-[(cis-4-aminocyclohexyl)amino]-2-methoxybenzamide

The title compound was synthesized by carrying out reaction according to the method described in Example 105 (b), except for using cis-tert-butyl 4-[(4-cyano-3-methoxyphenyl)amino]cyclohexylcarbamate as a starting material.
IR (neat) :3440, 3313, 3174, 2923, 1650, 15 65, 1419, 1338, 1211cm⁻¹

### Example 107

### Synthesis of 4-{[trans-4-(benzylamino)cyclohexyl]amino}-2-methoxybenzamide

Benzaldehyde (0.077 ml, 0.759 mmol) and acetic acid (0.13 ml, 2.28 mmol) were added to a solution of the 4-[(trans-4-aminocyclohexyl)amino]-2-methoxybenzamide obtained in Example 105 (200 mg, 0.759 mmol) in methanol (10 ml) and stirred for 30 minutes. Then, sodium cyanoborohydride (57 mg, 0.910 mmol) was added thereto and the resulting mixture was stirred at room temperature for 17 hours. The reaction mixture was poured into a 1N-aqueous sodium hydroxide solution and then extracted with ethyl acetate. The organic layer was extracted with 0.25N-hydrochloric acid and the aqueous layer was adjusted to pH 11 with a 1N-aqueous sodium hydroxide solution. The solid precipitated was collected by filtration and washed with diethyl ether by repulping to obtain 4-{[trans-4-(benzylamino)cyclohexyl]amino}-2-methoxybenzamide (110 mg, 41%).
IR (neat) : 3444, 3317, 3166, 2927, 1650, 15 81, 1569, 1423, 1342, 1207cm⁻¹

### Example 108

### Synthesis of 4-({2-[benzyl(methyl)amino]-ethyl}amino)-2-methoxybenzamide

### (a) Synthesis of tert-butyl 4-cyano-3-methyl-3-methoxyphenylcarbamate

A solution of sodium hydroxide (0.566 g, 14.2 mmol) in water (6 ml) was added to a solution of the 4-amino-2-methoxybenzonitrile obtained in Example 74 (2 g, 13.5 mmol) in a mixture of t-butanol (10 ml) and tetrahydrofuran (3 ml), followed by adding dropwise thereto a solution of di-tert-butyl dicarbonate (3.09 g, 14.2 mmol) in t-butanol (2 ml), and the resulting mixture was stirred at room temperature for 4 days. During the stirring, di-tert-butyl dicarbonate (11.9 g, 54.5 mmol) and sodium hydroxide (2.18 g, 54.5 mmol) were added thereto. The reaction mixture was poured into ice water and extracted with ethyl acetate. The organic layer was washed with a saturated aqueous sodium chloride solution, dried over anhydrous magnesium sulfate and then filtered. The filtrate was concentrated under reduced pressure and the resulting residue was purified by a silica gel column chromatography (eluent: hexane/ethyl acetate = 3/1 to 2/1) to obtain tert-butyl 4-cyano-3-methyl-3-methoxyphenylcarbamate (1.87 g, 56%).

### (b) Synthesis of tert-butyl 2-[benzyl(methyl)-amino]ethyl(4-cyano-3-methoxyphenyl)carbamate

Under ice-cooling, dimethylformamide (0.5 ml) was added to sodium hydride (78.6 mg, 1.96 mmol), followed by adding dropwise thereto a solution of tert-butyl 4-cyano-3-methyl-3-methoxyphenylcarbamate (470 mg, 1.8 mmol) in dimethylformamide (1.5 ml). The resulting mixture was stirred at room temperature for 1 hour, followed by adding thereto a solution of N-(2-chloroethyl)-N-methylbenzylamine (347 mg, 1.89 mmol) in dimethylformamide (0.5 ml) and sodium iodide (about 20 mg), and the resulting mixture was stirred at 60°C for 6 hours. The reaction mixture was poured into ice water and extracted with ethyl acetate. The organic layer was washed with a saturated aqueous sodium chloride solution, dried over magnesium sulfate and then filtered. The filtrate was concentrated under reduced pressure and the resulting residue was purified by a silica gel column chromatography (eluent: hexane/ethyl acetate = 2/1) to obtain tert-butyl 2-[benzyl(methyl)-amino]ethyl(4-cyano-3-methoxyphenyl)carbamate (720 mg, 96%).

### (c) Synthesis of 4-({2-[benzyl(methyl)amino]-ethyl}amino)-2-methoxybenzamide

The title compound was synthesized by carrying out reaction according to the method described in Example 105 (b), except for using tert-butyl 2-[benzyl(methyl)amino]ethyl(4-cyano-3-methoxyphenyl)carbamate as a starting material.

Melting point: 153-155°C.

### Example 109

### Synthesis of 4-({3-[benzyl(methyl)amino]-propyl}amino)-2-methoxybenzamide

### (a) Synthesis of tert-butyl 3-[benzyl(methyl)amino]-propyl(4-cyano-3-methoxyphenyl)carbamate

The title compound was synthesized by carrying out reaction according to the method described in Example 108 (b), except for using N-(3-chloropropyl)-N-methylbenzylamine in place of N-(2-chloroethyl)-N-methylbenzylamine.

### (b) Synthesis of 4-({3-[benzyl(methyl)amino]-propyl}amino)-2-methoxybenzamide

The title compound was synthesized by carrying out reaction according to the method described in Example 105 (b), except for using tert-butyl 3-[benzyl(methyl)amino]propyl(4-cyano-3-methoxyphenyl)-carbamate as a starting material.

Melting point: 116-118°C.

### Example 110

### Synthesis of 4-{[2-(dibenzylamino)ethyl]-amino}-2-methoxybenzamide

### (a) Synthesis of tert-butyl 4-cyano-3-methoxyphenyl[2-(dibenzylamino)ethyl]carbamate

The title compound was synthesized by carrying out reaction according to the method described in Example 108 (b), except for using N-(2-chloroethyl)-dibenzylamine in place of N-(2-chloroethyl)-N-methylbenzylamine.

### (b) Synthesis of 4-{[2-(dibenzylamino)ethyl]amino}-2-methoxybenzamide

The title compound was synthesized by carrying out reaction according to the method described in Example 105 (b), except for using tert-butyl 4-cyano-3-methoxyphenyl[2-(dibenzylamino)ethyl]carbamate as a starting material.

Melting point: 136-138°C.

### Example 111

### Synthesis of 4-anilino-2-methoxybenzamide

### (a) Synthesis of 4-anilino-2-methoxybenzonitrile

The title compound was synthesized by carrying out reaction according to the method described in Example 105 (a), except for using aniline in place of trans-tert-butyl 4-aminocyclohexylcarbamate.

### (b) Synthesis of 4-anilino-2-methoxybenzamide

The title compound was synthesized by carrying out reaction according to the method described in Example 105 (b), except for using 4-anilino-2-methoxybenzonitrile as a starting material.

Melting point: 192-194°C.

### Example 112

### Synthesis of 2-methoxy-4-(pyridin-4-ylamino)benzamide

### (a) Synthesis of 2-methoxy-4-(pyridin-4-ylamino)benzonitrile

The title compound was synthesized by carrying out reaction according to the method described in Example 105 (a), except for using 4-aminopyridine in place of trans-tert-butyl 4-aminocyclohexylcarbamate.

### (b) Synthesis of 2-methoxy-4-(pyridin-4-ylamino)benzamide

The title compound was synthesized by carrying out reaction according to the method described in Example 105 (b), except for using 2-methoxy-4-(pyridin-4-ylamino)benzonitrile as a starting material.
IR (neat) : 3440, 3255, 3166, 1650, 1577, 13 34, 995cm⁻¹

### Example 113

### Synthesis of 4-[(trans-4-aminocyclohexyl)amino]-5-chloro-2-methoxybenzamide

### (a) Synthesis of trans-tert-butyl 4-[(2-chloro-4-cyano-5-methoxyphenyl)amino]cyclohexylcarbamate

The title compound was synthesized by carrying out reaction according to the method described in Example 74 (d), except for using the trans-tert-butyl 4-[(4-cyano-3-methoxyphenyl)amino]cyclohexylcarbamate obtained in Example 105 (a), as a starting material.

### (b) Synthesis of 4-[(trans-4-aminocyclohexyl)amino]-5-chloro-2-methoxybenzamide

The title compound was synthesized by carrying out reaction according to the method described in Example 105 (b), except for using trans-tert-butyl 4-[(2-chloro-4-cyano-5-methoxyphenyl)amino]cyclohexylcarbamate as a starting material.

Melting point: 200-202°C.

### Example 114

### Synthesis of 4-[(cis-4-aminocyclohexyl)-amino]-5-chloro-2-methoxybenzamide

### (a) Synthesis of cis-tert-butyl 4-[(2-chloro-4-cyano-5-methoxyphenyl)amino]cyclohexylcarbamate

The title compound was synthesized by carrying out reaction according to the method described in Example 74 (d), except for using the cis-tert-butyl 4-[(4-cyano-3-methoxyphenyl)amino]cyclohexylcarbamate obtained in Example 106 (d), as a starting material.

### (b) Synthesis of 4-[(cis-4-aminocyclohexyl)amino]-5-chloro-2-methoxybenzamide

The title compound was synthesized by carrying out reaction according to the method described in Example 105 (b), except for using cis-tert-butyl 4-[(2-chloro-4-cyano-5-methoxyphenyl)amino]cyclohexylcarbamate as a starting material.
IR (neat) : 3455, 3413, 3166, 2927, 1650, 15 81, 1423, 1353, 1211, 1110cm⁻¹

### Example 115

### Synthesis of 5-chloro-2-methoxy-4-{[trans-4-(propylamino)cyclohexyl]amino}benzamide

### (a) Synthesis of 4-[(trans-4-aminocyclohexyl)amino]-5-chloro-2-methoxybenzonitrile

To a solution of the trans-tert-butyl 4-[(2-chloro-4-cyano-5-methoxyphenyl)amino]cyclohexylcarbamate obtained in Example 113 (2.0 g, 5.26 mmol) in methylene chloride (20 ml) was added 4N-hydrochloric acid/dioxane (20 ml), and the resulting mixture was stirred at room temperature for fifteen and a half hours. The solid precipitated was filtered and the precipitate on a filter was dissolved in water, and the resulting solution was adjusted to pH 10 to 11 with a 1N-aqueous sodium hydroxide solution and extracted with ethyl acetate. The organic layer was washed with a saturated aqueous sodium chloride solution, dried over anhydrous sodium sulfate and then filtered. The filtrate was concentrated under reduced pressure to obtain 4-[(trans-4-aminocyclohexyl)amino]-5-chloro-2-methoxybenzonitrile (1.45 g, 99%).

### (b) Synthesis of 5-chloro-2-methoxy-4-{[trans-4-(propylamino)cyclohexyl]amino}benzonitrile

Propionaldehyde (0.064 ml, 0.893 mmol) was added to a solution of 4-[(trans-4-aminocyclohexyl)amino]-5-chloro-2-methoxybenzonitrile (250 mg, 0.893 mmol) in methanol (10 ml) and stirred for 15 minutes. Then, acetic acid (0.153 ml, 2.67 mmol) and sodium cyanoborohydride (67 mg, 1.07 mmol) were added thereto and the resulting mixture was stirred at room temperature for 3 hours. The reaction mixture was poured into a saturated aqueous sodium hydrogencarbonate solution and extracted with ethyl acetate. The organic layer was washed with a saturated aqueous sodium chloride solution, dried over anhydrous sodium sulfate and then filtered. The filtrate was concentrated under reduced pressure and the resulting residue was purified by a silica gel column chromatography (eluent: chloroform/methanol = 15/1 ∼ chloroform/methanol/aqueous ammonia = 100/10/1) to obtain 5-chloro-2-methoxy-4-{[trans-4-(propylamino)cyclohexyl]amino}benzonitrile (124 mg, 43%).

### (c) Synthesis of 5-chloro-2-methoxy-4-{[trans-4-(propylamino)cyclohexyl]amino}benzamide

The title compound was synthesized by carrying out reaction according to the method described in Example 41 (b), except for using 5-chloro-2-methoxy-4-{[trans-4-(propylamino)cyclohexyl]amino}benzonitrile as a starting material.
IR (neat) 3455, 3409, 3313, 3147, 2931, 16 54, 1589, 1423, 1361, 1207, 1114, 809cm⁻¹

### Example 116

### Synthesis of 5-chloro-4-{[trans-4-(isopropylamino)cyclohexyl]amino}-2-methoxybenzamide

### (a) Synthesis of 5-chloro-4-{[trans-4-(isopropylamino)cyclohexyl]amino}-2-methoxybenzonitrile

The title compound was synthesized by carrying out reaction according to the method described in Example 115 (b), except for using acetone in place of propionaldehyde.

### (b) Synthesis of 5-chloro-4-{[trans-4-(isopropylamino)cyclohexyl]amino}-2-methoxybenzamide

The title compound was synthesized by carrying out reaction according to the method described in Example 41 (b), except for using 5-chloro-4-{[trans-4-(isopropylamino)cyclohexyl]amino}-2-methoxybenzonitrile as a starting material.
IR (neat) :3455, 3409, 3301, 3158, 2969, 16 50, 1592, 1423, 1365, 1211, 1110, 809cm⁻¹

### Example 117

### Synthesis of 5-chloro-4-{[trans-4-(dimehylamino)cyclohexyl]amino}-2-methoxybenzamide

### (a) Synthesis of 5-chloro-4-{[trans-4-(dimehylamino)-cyclohexyl]amino}-2-methoxybenzonitrile

A formaldehyde solution (36%, 148 mg, 1.78 mmol), acetic acid (0.255 ml, 4.47 mmol) and sodium cyanoborohydride (123 mg, 1.96 mmol) were added to a solution of the 4-[(trans-4-aminocyclohexyl)amino]-5-chloro-2-methoxybenzonitrile obtained in Example 115 (a) (250 mg, 0.893 mmol) in methanol (10 ml), and the resulting mixture was stirred at room temperature for six and a half hours. The reaction mixture was poured into a saturated aqueous sodium hydrogencarbonate solution and then extracted with ethyl acetate. The organic layer was washed with a saturated aqueous sodium chloride solution, dried over anhydrous sodium sulfate and then filtered. The filtrate was concentrated under reduced pressure and the resulting residue was purified by a silica gel column chromatography (eluent: chloroform/methanol = 10/1) to obtain 5-chloro-4-{[trans-4-(dimehylamino)cyclohexyl]-amino}-2-methoxybenzonitrile (229 mg, 83%).

### (b) Synthesis of 5-chloro-4-{[trans-4-(dimehylamino)cyclohexyl]amino}-2-methoxybenzamide

The title compound was synthesized by carrying out reaction according to the method described in Example 41 (b), except for using 5-chloro-4-{[trans-4-(dimehylamino)cyclohexyl]amino}-2-methoxybenzonitrile as a starting material.

Melting point: 202-203°C.

### Example 118

### Synthesis of 5-chloro-4-{[trans-4-(cyclopentylamino)cyclohexyl]amino}-2-methoxybenzamide

### (a) Synthesis of 5-chloro-4-{[trans-4-(cyclopentylamino)cyclohexyl]amino}-2-methoxybenzonitrile

The title compound was synthesized by carrying out reaction according to the method described in Example 115 (b), except for using cyclopentanone in place of propionaldehyde.

### (b) Synthesis of 5-chloro-4-{[trans-4-(cyclopentylamino)cyclohexyl]amino}-2-methoxybenzamide

The title compound was synthesized by carrying out reaction according to the method described in Example 41 (b), except for using 5-chloro-4-{[trans-4-(cyclopentylamino)cyclohexyl]amino}-2-methoxybenzonitrile as a starting material.

Melting point: 169-171°C.

### Example 119

### Synthesis of 5-chloro-2-methoxy-4-[(trans-4-pyrrolidin-1-ylcyclohexyl)amino]benzamide

### (a) Synthesis of 5-chloro-2-methoxy-4-[(trans-4-pyrrolidin-1-ylcyclohexyl)amino]benzonitrile

To a solution of the 4-[(trans-4-aminocyclohexyl)amino]-5-chloro-2-methoxybenzonitrile obtained in Example 115 (a) (250 mg, 0.893 mmol) in dimethylformamide (5 ml) were added 1,4-dibromobutane (0.106 ml, 0.893 mmol) and potassium carbonate (246 mg, 1.786 mmol), and the resulting mixture was stirred with heating at 60°C for 5 hours. A 1N-aqueous sodium hydroxide solution was added to the reaction mixture, followed by extraction with ethyl acetate, and the extract solution was dried over anhydrous sodium sulfate and filtered. The filtrate was concentrated under reduced pressure and the resulting residue was purified by a silica gel column chromatography (eluent: chloroform/methanol = 10/1) to obtain 5-chloro-2-methoxy-4-[(trans-4-pyrrolidin-1-ylcyclohexyl)-amino]benzonitrile (188 mg) as a crude product.

### (b) Synthesis of 5-chloro-2-methoxy-4-[(trans-4-pyrrolidin-1-ylcyclohexyl)amino]benzamide

The title compound was synthesized by carrying out reaction according to the method described in Example 41 (b), except for using 5-chloro-2-methoxy-4-[(trans-4-pyrrolidin-1-ylcyclohexyl)amino]-benzonitrile as a starting material.
IR (neat) : 3459, 3417, 3143, 2942, 1654, 15 85, 1427, 1361, 1214, 1114, 806cm⁻¹

### Example 120

### Synthesis of 5-chloro-2-methoxy-4-{[trans-4-(methylamino)cyclohexyl]amino}benzamide

### (a) Synthesis of trans-N-methylcyclohexane-1,4-diamine

A solution of trans-tert-butyl 4-aminocyclohexylcarbamate (4.50 g, 21.0 mmol) in tetrahydrofuran (30 ml) was added to a suspension of lithium aluminum hydride (3.98 g, 105 mmol) in tetrahydrofuran (135 ml) at 11 to 13°C in a water bath over a period of 10 minutes. The resulting mixture was stirred at room temperature for 30 minutes and then the reaction was carried out with heating for 6 hours under refluxing conditions. After completion of the reaction, water (4 ml), a 1N-aqueous sodium hydroxide solution (4 ml) and water (12 ml) were carefully dropped into the reaction mixture in that order under ice-cooling, and the resulting mixture was stirred at room temperature for 15 minutes. This mixture in the form of a suspension was filtered by using tetrahydrofuran (60 ml), t-butyl methyl ether (60 ml), methylene chloride (60 ml), t-butyl methyl ether (60 ml) and tetrahydrofuran (60 ml) in that order. The filtrate was concentrated to obtain trans-N-methylcyclohexane-1,4-diamine (2.46 g, 91%).

### (b) Synthesis of trans-tert-butyl 4-aminocyclohexyl(methyl)carbamate

Benzaldehyde (2.0 ml, 19.7 mmol) was added to a solution of trans-N-methylcyclohexane-1,4-diamine (2.48 g, 19.3 mmol) in toluene (25 ml) at room temperature, and the resulting mixture was stirred for 5 hours under refluxing conditions while using a Dean-Stark trap. After the mixture was cooled to room temperature, di-tert-butyl dicarbonate (4.45 ml, 19.4 mmol) was added thereto and the resulting mixture was stirred overnight. Then, a 1N-aqueous potassium hydrogensulfate solution (20 ml) was added thereto, followed by stirring at room temperature for 7 hours. After completion of the reaction, the reaction mixture was extracted twice with diethyl ether, and the aqueous layer was made basic with a 1N-aqueous sodium hydroxide solution and then extracted twice with chloroform. Sodium chloride was added to the aqueous layer, followed by extraction with chloroform. The combined chloroform layer was dried over magnesium sulfate and filtered, and the filtrate was concentrated to obtain trans-tert-butyl 4-aminocyclohexyl-(methyl)carbamate (3.89 g, 88%).

### (c) Synthesis of trans-tert-butyl 4-[(4-cyano-3-methoxyphenyl)amino]cyclohexyl(methyl)carbamate

The title compound was synthesized by carrying out reaction according to the method described in Example 105 (a), except for using trans-tert-butyl 4-aminocyclohexyl(methyl)carbamate in place of trans-tert-butyl 4-aminocyclohexylcarbamate.

### (d) Synthesis of trans-tert-butyl 4-[(2-chloro-4-cyano-5-methoxyphenyl)amino]cyclohexyl(methyl)-carbamate

The title compound was synthesized by carrying out reaction according to the method described in Example 74 (d), except for using trans-tert-butyl 4-[(4-cyano-3-methoxyphenyl)amino]cyclohexyl(methyl)-carbamate as a starting material.

### (e) Synthesis of 5-chloro-2-methoxy-4-{[trans-4-(methylamino)cyclohexyl]amino}benzamide

The title compound was synthesized by carrying out reaction according to the method described in Example 105 (b), except for using trans-tert-butyl 4-[(2-chloro-4-cyano-5-methoxyphenyl)amino]cyclohexyl(methyl)carbamate as a starting material.

Melting point: 184-186°C.

### Example 121

### Synthesis of 4-{[trans-4-(benzylamino)-cyclohexyl]amino}-5-chloro-2-methoxybenzamide

The title compound was synthesized by carrying out reaction according to the method described in Example 107, except for using the 4-[(trans-4-aminocyclohexyl)amino]-5-chloro-2-methoxybenzamide obtained in Example 113, as a starting material in place of 4-[(4-aminocyclohexyl)amino]-2-methoxybenzamide.

Melting point: 204-206°C.

### Example 122

### Synthesis of 4-(azepan-4-ylamino)-3-chlorobenzamide

### (a) Synthesis of tert-butyl 4-{[4-(aminocarbonyl)-phenyl]amino}azepane-1-carboxylate

The title compound was synthesized by carrying out reaction according to the method described in Example 45 (a), except for using tert-butyl 4-oxoazepane-1-carboxylate as a starting material.

### (b) Synthesis of tert-butyl 4-{[4-(aminocarbonyl)-2-chlorophenyl]amino}azepane-1-carboxylate

The title compound was synthesized by carrying out reaction according to the method described in Example 45 (b), except for using tert-butyl 4-{[4-(aminocarbonyl)phenyl]amino}azepane-1-carboxylate as a starting material.

### (c) Synthesis of 4-(azepan-4-ylamino)-3-chlorobenzamide

The title compound was synthesized by carrying out reaction according to the method described in Example 45 (c), except for using tert-butyl 4-{[4-(aminocarbonyl)-2-chlorophenyl]amino}azepane-1-carboxylate as a starting material.
¹H-NMR (DMSO-d₆) δ;1.45-1.61 (2H, m) , 1.6 6-1.83 (3H, m), 1.83-1.92 (1H, m) , 2.66-2. 93 (4H, m), 3.72-3.85 (1H, m) , 5.54 (1H, d, J =8.6Hz), 6.70 (1H, d, J=8.6Hz) , 7.04 (1H, s) , 7.68 (2H, d, J=10.2Hz) , 7.80 (1H, s).

### Example 123

### Synthesis of 4-[(1-benzylazepan-4-yl)amino]-3-chlorobenzamide

The title compound was synthesized by carrying out reaction according to the method described in Example 46, except for using the 4-(azepan-4-ylamino)-3-chlorobenzamide obtained in Example 122, as a starting material.

Melting point: 174-175°C.

### Example 124

### Synthesis of 4-[(1-benzylpiperidin-3-yl)amino]-3-chlorobenzamide

### (a) Synthesis of tert-butyl 3-{[4-(aminocarbonyl)-phenyl]amino}piperidine-1-carboxylate

The title compound was synthesized by carrying out reaction according to the method described in Example 45 (a), except for using tert-butyl 3-oxopiperidine-1-carboxylate as a starting material.

### (b) Synthesis of tert-butyl 3-{[4-(aminocarbonyl)-2-chlorophenyl]amino}piperidine-1-carboxylate

The title compound was synthesized by carrying out reaction according to the method described in Example 45 (b), except for using tert-butyl 3-{[4-(aminocarbonyl)phenyl]amino}piperidine-1-carboxylate as a starting material.

### (c) Synthesis of 3-chloro-4-(piperidin-3-ylamino)benzamide

The title compound was synthesized by carrying out reaction according to the method described in Example 45 (c), except for using tert-butyl 3-{[4-(aminocarbonyl)-2-chlorophenyl]amino}piperidine-1-carboxylate as a starting material.

### (d) Synthesis of 4-[(1-benzylpiperidin-3-yl)amino]-3-chlorobenzamide

The title compound was synthesized by carrying out reaction according to the method described in Example 46, except for using 3-chloro-4-(piperidin-3-ylamino)benzamide as a starting material.
¹H-NMR (DMSO-d₆) δ;1.42-1.68 (4H, m) , 2. 2 9-2.60 (4H, m) , 3.42-3.57 (2H, m) , 3.70 (1H, s) , 5.48 (1H, d, J=8.8Hz), 6.71 (1H, d, J=8. 9Hz) , 7.05 (1H, s) , 7.18-7.35 (5H, m) , 7.63 -7.76 (2H, m) , 7.81 (1H, s).

### Example 125

### Synthesis of trans-4-[(4-aminocyclohexyl)-amino]-3-chlorobenzamide

### (a) Synthesis of tert-butyl trans-4-[(4-cyanophenyl)-amino]cyclohexylcarbamate

The title compound was synthesized by carrying out reaction according to the method described in Example 105 (a), except for using tert-butyl trans-4-aminocyclohexylcarbamate and p-bromobenzonitrile as starting materials.

### (b) Synthesis of tert-butyl trans-4-[(2-chloro-4-cyanophenyl)amino]cyclohexylcarbamate

The title compound was synthesized by carrying out reaction according to the method described in Example 45 (b), except for using tert-butyl trans-4-[(4-cyanophenyl)amino]cyclohexylcarbamate as a starting material.

### (c) Synthesis of trans-4-[(4-aminocyclohexyl)amino]-3-chlorobenzamide

The title compound was synthesized by carrying out reaction according to the method described in Example 59 (d), except for using tert-butyl trans-4-[(2-chloro-4-cyanophenyl)amino]cyclohexylcarbamate as a starting material.
IR: 3329, 3178, 1601, 1392, 1145, 1037 cm⁻¹.

The following compounds of Example 126 to Example 130 were synthesized by carrying out reaction according to the method described in Example 10, except for using 4-amino-2-methoxybenzamide and an aldehyde and a ketone which correspond to each of the compounds as a starting material.

### Example 126

### 4-(Cyclohexylamino)-2-methoxybenzamide

Melting point: 156-159°C.

### Example 127

### 2-Methoxy-4-(tetrahydro-2H-thiopyran-4-ylamino)benzamide

IR: 3309, 3167, 1643, 1581, 1342, 1103, 806 cm⁻¹.

### Example 128

### 2-Methoxy-4-[(pyridin-4-ylmethyl)amino]-benzamide

Melting point: 180-183°C.

### Example 129

### 2-Methoxy-4-[(pyridin-2-ylmethyl)amino]-benzamide

IR: 3398, 3167, 1647, 1581, 1326, 1029 cm⁻¹.

### Example 130

### 2-Methoxy-4-(tetrahydro-2H-pyran-4-ylamino)benzamide

IR: 1585, 1334, 1215, 1084, 806 cm⁻¹.

### Example 131

### Synthesis of 5-chcloro-2-methoxy 4-(tetrahydro-2H-pyran-4-ylamino)benzamide

The title compound was synthesized by carrying out reaction according to the method described in Example 45 (b), except for using the 2-methoxy-4-(tetrahydro-2H-pyran-4-ylamino)benzamide obtained in Example 130, as a starting material.

Melting point: 218-220°C.

### Example 132

### Synthesis of 2-methoxy-4-(pyridin-3-ylamino)benzamide

### (a) Synthesis of methyl 2-methoxy-4-(pyridin-3-ylamino)benzoate

The title compound was synthesized by carrying out reaction according to the method described in Example 105 (a), except for using tert-butyl 4-aminocyclohexylcarbamate and 3-bromopyridine as starting materials.

### (b) Synthesis of 2-methoxy-4-(pyridin-3-ylamino)benzamide

The title compound was synthesized by carrying out reaction according to the method described in Example 85 (b), except for using methyl 2-methoxy-4-(pyridin-3-ylamino)benzoate as a starting material.

Melting point: 164-166°C.

### Example 133

### Synthesis of 2-methoxy-4-(pyridin-2-ylamino)benzamide

### (a) Synthesis of methyl 2-methoxy-4-(pyridin-2-ylamino)benzoate

The title compound was synthesized by carrying out reaction according to the method described in Example 105 (a), except for using tert-butyl 4-aminocyclohexylcarbamate and 2-bromopyridine as starting materials.

### (b) Synthesis of 2-methoxy-4-(pyridin-2-ylamino)benzamide

The title compound was synthesized by carrying out reaction according to the method described in Example 85 (b), except for using methyl 2-methoxy-4-(pyridin-2-ylamino)benzoate as a starting material.

Melting point: 226-227°C.

### Example 134

### Synthesis of 4-{[(trans-4-aminocyclohexyl)methyl]amino}-2-methoxybenzamide

### (a) Synthesis of tert-butyl trans-4-{[(4-cyano-3-methoxyphenyl)amino]methyl}cyclohexylcarbamate

The title compound was synthesized by carrying out reaction according to the method described in Example 105 (a), except for using tert-butyl trans-4-(aminomethyl)cyclohexylcarbamate as a starting material.

### (b) Synthesis of 4-{[(trans-4-aminocyclohexyl)methyl]-amino}-2-methoxybenzamide

The title compound was synthesized by carrying out reaction according to the method described in Example 59 (d), except for using tert-butyl trans-4-{[(4-cyano-3-methoxyphenyl)amino]methyl}cyclohexylcarbamate as a starting material.
IR: 1651, 1589, 1377, 1265, 1030, 810 cm⁻¹.

### Example 135

### Synthesis of 4-{[trans-4-(aminomethyl)cyclohexyl]amino}-2-methoxybenzamide

### (a) Synthesis of trans-4-({[(benzyloxy)carbonyl] amino}methyl)cyclohexanecarboxylic acid

Benzyl chloroformate (16.3 ml, 114.48 mmol) was added to a suspension of trans-4-(aminomethyl)-cyclohexanecarboxylic acid (15 g, 95.4 mmol) in dioxane (30 ml), followed by adding dropwise thereto a 2N aqueous sodium hydroxide solution (228.9 ml, 228.9 mmol) at 0°C, and the resulting mixture was stirred at room temperature for 18 hours. The reaction mixture was neutralized with 1N hydrochloric acid and then extracted with ethyl acetate. The organic layer was washed with a saturated aqueous sodium chloride solution, dried over anhydrous sodium sulfate and then filtered. The filtrate was concentrated and the resulting residue was purified by a silica gel column chromatography (hexane : ethyl acetate = 2 : 1 to 1 : 1) to obtain trans-4-({[(benzyloxy)carbonyl]amino}-methyl)cyclohexanecarboxylic acid (11.99 g, 41.15 mmol, 43%).

### (b) Synthesis of benzyl {trans-4-[(tert-butoxycarbonyl)amino]cyclohexyl}methylcarbamate

Triethylamine (6.31 ml, 45.3 mmol) and diphenylphosphoryl azide (11.4 g, 41.52 mmol) were added to a solution of trans-4-({[(benzyloxy)carbonyl]-amino}methyl)cyclohexanecarboxylic acid (11 g, 37.75 mmol) in tert-butanol (110 ml), and the resulting mixture was heated under reflux for eight and a half hours. The reaction mixture was concentrated and the white solid precipitated was removed by filtration, and ethyl acetate and water were added to the filtrate to extract. The organic layer was concentrated and the resulting residue was purified by a silica gel column chromatography (hexane : ethyl acetate = 4 : 1 to 3 : 1) and recrystallized from ethyl acetate to obtain benzyl {trans-4-[(tert-butoxycarbonyl)amino]-cyclohexyl}methylcarbamate (2.35 g, 6.58 mmol, 17%).

### (c) Synthesis of benzyl (trans-4-aminocyclohexyl)-methylcarbamate hydrochloride

To a solution of benzyl {4-[(tert-butoxycarbonyl)amino]cyclohexyl}methylcarbamate (2.5 g, 6.89 mmol) in tetrahydrofuran (25 ml) was added 4N hydrochloric acid-dioxane (25 ml), and the resulting mixture was stirred at room temperature for 20 hours. The solid precipitated was filtered to obtain benzyl (4-aminocyclohexyl)methylcarbamate hydrochloride (1.83 g, 6.15 mmol, 89%).

### (d) Synthesis of benzyl {trans-4-[(4-cyano-3-methoxyphenyl)amino]cyclohexyl}methylcarbamate

The title compound was synthesized by carrying out reaction according to the method described in Example 105 (a), except for using benzyl (trans-4-aminocyclohexyl)methylcarbamate as a starting material.

### (e) Synthesis of 4-{[trans-4-(aminomethyl)cyclohexyl]-amino}-2-methoxybenzamide

The title compound was synthesized by carrying out reaction according to the method described in Example 59 (d), except for using benzyl {4-[(4-cyano-3-methoxyphenyl)amino]cyclohexyl}methylcarbamate as a starting material.
IR: 3305, 1562, 1338, 1211, 1026 cm⁻¹.

### Example 136

### Synthesis of 3-chloro-4-[(2-pyrrolidin-1-ylethyl)amino]benzamide

### (a) Synthesis of tert-butyl 4-cyanophenylcarbamate

Di-tert-butyl carbonate (11.1 g, 50.8 mmol) was added to a solution of 4-cyanoaniline (5 g, 42.3 mmol) in dichloromethane (200 mL), followed by adding thereto dimethylaminopyridine (1.0 g, 8.5 mmol) at 0°C, and the resulting mixture was allowed to stand at room temperature for 3 days. The solvent was distilled off under reduced pressure and the resulting residue was purified by a silica gel chromatography to obtain tert-butyl 4-cyanophenylcarbamate (5.79 g, 63%).

### (b) Synthesis of tert-butyl 4-cyanophenyl(2-pyrrolidin-1-ylethyl)carbamate

Sodium hydride (121 mg, 3.02 mmol) was added to a solution (10 mL) of tert-butyl 4-cyanophenylcarbamate (300 mg, 1.37 mmol) in N,N-dimethylformamide at 0°C, and the resulting mixture was stirred at room temperature for 1 hour. To the reaction mixture was added 2-chloroethylpyrrole hydrochloride (234 mg, 1.37 mmol), and stirred for 3 hours, and the resulting mixture was heated to 60°C and stirred with heating for 4 hours. Then, sodium hydride (121 mg) and 2-chloroethylpyrrole hydrochloride (234 mg) were further added thereto, followed by stirring at 60°C for 2 hours. The reaction mixture was cooled, followed by adding thereto water, a saturated aqueous ammonium chloride solution and ethyl acetate. The aqueous layer was extracted twice with ethyl acetate and the extract solution was dried over anhydrous magnesium sulfate. The residue was purified by a silica gel chromatography to obtain tert-butyl 4-cyanophenyl(2-pyrrolidin-1-ylethyl)carbamate (259 mg).

### (c) Synthesis of 4-[(2-pyrrolidin-1-ylethyl)amino]benzamide

The title compound was synthesized by carrying out reaction according to the method described in Example 41 (b), except for using tert-butyl 4-cyanophenyl(2-pyrrolidin-1-ylethyl)carbamate as a starting material.

### (d) Synthesis of 3-chloro-4-[(2-pyrrolidin-1-ylethyl)amino]benzamide

The title compound was synthesized by carrying out reaction according to the method described in Example 45 (b), except for using 4-[(2-pyrrolidin-1-ylethyl)amino]benzamide as a starting material.
¹H-NMR (DMSO-d₆) δ ; 1.68 (4H, b r) , 2. 65 (2H, m) , 3.25 (2H, m) , 5.73(1H, t) , 6.71(1H, d, J =8.6Hz), 7.07 (1H, br), 7.69 (2H, brt, J=8. 8Hz) , 7.80 (1H, d, J=1.8Hz).

### Example 137

### Synthesis of cis-4-[(2-aminocyclohexyl)-amino]-3-chlorobenzamide

### (a) Synthesis of cis-4-[(2-aminocyclohexyl)-amino]benzonitrile

A solution of 4-bromobenzonitrile (1 g, 5.56 mmol), tris(dibenzylideneacetone)dipalladium(0) (50.9 mg, 0.06 mmol), (+)-2,2'-bis(diphenylphosphino)-1,1'-binaphthyl (104 mg, 0.17 mmol), cis-1,2-diaminocyclohexane (1.9 g, 16.7 mmol) and sodium tert-butoxide (747 mg, 7.78 mmol) in toluene was stirred at 90°C for 4 hours. The reaction mixture was cooled, followed by adding thereto ethyl acetate, water and a saturated aqueous ammonium chloride solution. The aqueous layer was extracted with ethyl acetate and the extract solution was dried over anhydrous magnesium sulfate. The residue was purified by a silica gel chromatography to obtain cis-4-[(2-aminocyclohexyl)amino]benzonitrile (702 mg, 59%).

### (b) Synthesis of cis-4-[(2-aminocyclohexyl)-amino]benzamide

The title compound was synthesized by carrying out reaction according to the method described in Example 41 (b), except for using cis-4-[(2-aminocyclohexyl)amino]benzonitrile as a starting material.

### (c) Synthesis of cis-4-[(2-aminocyclohexyl)amino]-3-chlorobenzamide

The title compound was synthesized by carrying out reaction according to the method described in Example 45 (b), except for using cis-4-[(2-aminocyclohexyl)amino]benzamide as a starting material.
¹H-NMR (DMSO-d₆) δ ; 1.31∼1.41(3H, m) , 1. 5 2∼1.62 (5H, m), 2.98 (1H, brd) , 5.65 (1H, d, J=7.9Hz) , 6.74 (1H, d, J=8.8Hz), 7.04 (1H, br), 7.66 (1H, dd, J=2.0, 8.6Hz) , 7.70 (1H, br), 7.80 (1H, d, J=2.0Hz).

### Example 138

### Synthesis of trans-4-[(2-aminocyclohexyl)-amino]-3-chlorobenzamide monohydrochloride

Reaction was carried out according to the method described in Example 137, except for using trans-1,2-diaminocyclohexane in place of cis-1,2-diaminocyclohexane, to obtain a compound, and the title compound was synthesized by converting the obtained compound to its hydrochloride according to the method described in Example 11.
¹H-NMR (DMSO-d₆) δ;1.19∼1.51 (4H, m) , 1.6 4∼1.73 (2H, m) , 1.87∼1.90 (1H, m) , 2.06∼2. 09 (1H, m) , 3.27 (1H, br) , 5.62 (1H, d, J=9.9 Hz), 6.92 (1H, d, J=8.8Hz) , 7.11 (1H, br) , 7. 70 (1H, dd, J=1.8, 8.6Hz), 7.82 (1H, d, J=2. 0Hz), 8.00 (br, 2H).

### Example 139

### Synthesis of trans-4-{(3-aminocyclohexyl)-amino}-3-chlorobenzamide

### (a) Synthesis of trans-2-(3-aminocyclohexyl)-1H-isoindole-1,3(2H)-dione

Triethylamine (5.7 mL, 40.8 mmol) and then methanesulfonyl chloride (2.4 mL, 30.6 mmol) were added to a solution of cis-2-(3-hydroxycyclohexyl)-1H-isoindole-1,3(2H)-dione (5 g, 20.4 mmol) in tetrahydrofuran at 0°C, and the resulting mixture was stirred at the same temperature for 2 hours. After water and ethyl acetate were added to the reaction mixture, the aqueous layer was extracted with ethyl acetate and then the mixed organic layer was dried over anhydrous magnesium sulfate.

Sodium azide (4.0 g, 61.2 mmol) was added to a solution of the concentration residue in N-methylpyrrolidinone, and the resulting mixture was stirred at 90°C for 2 hours. After the reaction mixture was cooled, water and diethyl ether were added thereto and the aqueous layer was extracted with diethyl ether, and the combined organic layer was dried over anhydrous magnesium sulfate.

Palladium/carbon (2.5 g) was added to a solution of the concentration residue in methanol, and the resulting mixture was stirred for 4 hours under a hydrogen atmosphere. The reaction mixture was filtered by the use of Celite and the filtrate was concentrated to obtain trans-2-(3-aminocyclohexyl)-1H-isoindole-1,3(2H)-dione.

### (b) Synthesis of trans-tert-butyl 3-aminocyclohexylcarbamate

Di-tert-butyl carbonate (8.9 g, 40.8 mmol) was added to a solution of trans-2-(3-aminocyclohexyl)-1H-isoindole-1,3(2H)-dione in dichloromethane and stirred overnight, and the reaction mixture was concentrated. A white solid obtained by purifying the concentrate by a silica gel chromatography was dissolved in a 40% methylamine-methanol solution (100 mL) and the resulting solution was stirred at 70°C for 2 hours. The reaction solution was concentrated, followed by adding thereto ethyl acetate, and the white solid precipitated was filtered off and the filtrate was concentrated. The residue was purified by a silica gel chromatography to obtain trans-tert-butyl 3-aminocyclohexylcarbamate (2.0 g, 48% through 5 steps from cis-2-(3-hydroxycyclohexyl)-1H-isoindole-1,3(2H)-dione).

### (c) Synthesis of trans-tert-butyl 3-[(4-cyanophenyl)amino]cyclohexylcarbamate

A solution of 4-bromobenzonitrile (500 mg, 2.78 mmol), tris(dibenzylideneacetone)dipalladium(0) (25.4 mg, 0.03 mmol), (+)-2,2'-bis(diphenylphosphino)-1,1'-binaphthyl (51.9 mg, 0.08 mmol), trans-tert-butyl 3-aminocyclohexylcarbamate (655 mg, 3.06 mmol) and sodium tert-butoxide (374 mg, 3.89 mmol) in toluene was stirred at 90°C for 4 hours. The reaction solution was cooled, followed by adding thereto ethyl acetate, water and a saturated aqueous ammonium chloride solution. The aqueous layer was extracted with ethyl acetate and the organic layer was dried over anhydrous magnesium sulfate. The residue was purified by a silica gel chromatography to obtain trans-tert-butyl 3-[(4-cyanophenyl)amino]cyclohexylcarbamate (354 mg, 41%).

### (d) Synthesis of trans-tert-butyl 3-[(2-chloro-4-cyanophenyl)amino]cyclohexylcarbamate

The title compound was synthesized by carrying out reaction according to the method described in Example 45 (b), except for using trans-tert-butyl 3-[(4-cyanophenyl)amino]cyclohexylcarbamate as a starting material.

### (e) Synthesis of trans-4-{(3-aminocyclohexyl)amino}-3-chlorobenzamide

The title compound was synthesized by carrying out reaction according to the method described in Example 41 (b), except for using trans-tert-butyl 3-[(2-chloro-4-cyanophenyl)amino]cyclohexylcarbamate as a starting material.
¹H-NMR (DMSO-d₆) δ ; 1.32∼1.72 (10H, m) , 3. 04 (1H, m) , 3.84 (1H, m) , 5.05 (1H, d), 6.77 (1H, d) , 7.07(1H, br), 7.68 (1H, dd), 7.72 (1H, br) , 7.80 (1H, d).

### Example 140

### Synthesis of 4-[trans-(4-aminocyclohexyl)-amino]-2-fluorobenzamide

### (a) Synthesis of tert-butyl trans-4-[(4-cyano-3-fluorophenyl)amino]cyclohexylcarbamate

The title compound was synthesized by carrying out reaction according to the method described in Example 105 (a), except for using 4-bromo-2-fluorobenzonitrile and tert-butyl trans-4-aminocyclohexylcarbamate as starting materials.

### (b) Synthesis of 4-[trans-(4-aminocyclohexyl)amino]-2-fluorobenzamide

The title compound was synthesized by carrying out reaction according to the method described in Example 59 (d), except for using tert-butyl trans-4-[(4-cyano-3-fluorophenyl)amino]cyclohexylcarbamate as a starting material.

Melting point: 166-167°C.

### Example 141

### Synthesis of 4-[trans-(4-aminocyclohexyl)-amino]-5-chloro-2-fluorobenzamide

### (a) Synthesis of tert-butyl trans-4-[(2-chloro-4-cyano-5-fluorophenyl)amino]cyclohexylcarbamate

The title compound was synthesized by carrying out reaction according to the method described in Example 45 (b), except for using the tert-butyl trans-4-[(4-cyano-3-fluorophenyl)amino]cyclohexylcarbamate obtained in Example 140 (a), as a starting material.

### (b) Synthesis of 4-[trans-(4-aminocyclohexyl)amino]-5-chloro-2-fluorobenzamide

The title compound was synthesized by carrying out reaction according to the method described in Example 59 (d), except for using tert-butyl trans-4-[(2-chloro-4-cyano-5-fluorophenyl)amino]cyclohexylcarbamate as a starting material.

Melting point: 160-162°C.

### Example 142

### Synthesis of 4-[(1-benzylpiperidin-4-yl)amino]-2-methoxy-5-methylbenzamide

### (a) Synthesis of 2-(5-methoxy-2-methyl-4-nitrophenyl)-1H-isoindole-1,3(2H)-dione

Phthalic anhydride (2.7 g, 18.1 mmol) was added to a solution of 5-methoxy-2-methyl-4-nitrophenylamine (3.0 g, 16.5 mmol) in acetic acid (30 mL), and the resulting mixture was stirred at 110°C for 3 hours. The mixture was allowed to cool and then was distilled under reduced pressure to remove the solvent. The resulting residue was dissolved in acetic anhydride (30 mL) and the resulting solution was stirred at 100°C for 5 hours. This solution was allowed to cool and then was distilled under reduced pressure to remove the acetic anhydride, and the resulting residue was washed with diethyl ether to obtain 2-(5-methoxy-2-methyl-4-nitrophenyl)-1H-isoindole-1,3(2H)-dione (4.2 g, 82%) as pale-brown powder.

### (b) Synthesis of 2-(4-amino-5-methoxy-2-methylphenyl)-1H-isoindole-1,3(2H)-dione

The title compound was synthesized by carrying out reaction according to the method described in Example 3 (a), except for using 2-(5-methoxy-2-methyl-4-nitrophenyl)-1H-isoindole-1,3(2H)-dione as a starting material.

### (c) Synthesis of 4-(1,3-dioxo-1,3-dihydro-2H-isoindol-2-yl)-2-methoxy-5-methylbenzonitrile

Concentrated hydrochloric acid (0.4 mL) and water (5 mL) were added to 2-(4-amino-5-methoxy-2-methylphenyl)-1H-isoindole-1,3(2H)-dione (280 mg, 1.0 mmol), followed by adding dropwise thereto an aqueous solution (2 mL) of sodium nitrite (1.2 mmol, 83 mg) under ice-cooling, and the resulting mixture was stirred for 10 minutes to prepare a diazonium salt solution. The prepared diazonium salt solution was added dropwise to an aqueous solution (5 mL) of potassium cyanide (325 mg, 5.0 mmol) and copper (I) cyanide (360 mg, 4.0 mmol) at room temperature and stirred at 50°C for 2 hours. A saturated aqueous sodium chloride solution and a saturated aqueous sodium hydrogencarbonate solution were added to the reaction solution, followed by extraction with ethyl acetate (twice), and the organic layer was washed with a saturated aqueous sodium chloride solution. The washed organic layer was dried over magnesium sulfate and distilled under reduced pressure to remove the solvent, and the resulting residue was purified by a silica gel chromatography (hexane : ethyl acetate = 2 : 1) to obtain 4-(1,3-dioxo-1,3-dihydro-2H-isoindol-2-yl)-2-methoxy-5-methylbenzonitrile (112 mg, 38%) as pale-brown powder.

### (d) Synthesis of 4-amino-2-methoxy-5-methylbenzonitrile

Tetrahydrofuran (5 mL) and a 35% aqueous hydrazine solution (155 µL, 1.7 mmol) were added to a solution of 4-(1,3-dioxo-1,3-dihydro-2H-isoindol-2-yl)-2-methoxy-5-methylbenzonitrile (100 mg, 0.34 mmol) in ethanol (10 mL), and the resulting mixture was stirred at room temperature for 5 hours and then at 50°C for 1 hour. A saturated aqueous sodium chloride solution and a saturated aqueous sodium hydrogencarbonate solution were added to the reaction mixture, followed by extraction with ethyl acetate (three times), and the organic layer was washed with a saturated aqueous sodium chloride solution. The washed organic layer was dried over magnesium sulfate and distilled under reduced pressure to remove the solvent, and the resulting residue was purified by a silica gel chromatography and then washed with diethyl ether to obtain 4-amino-2-methoxy-5-methylbenzonitrile (40 mg, 73%) as pale-brown powder.

### (e) Synthesis of 4-bromo-2-methoxy-5-methylbenzonitrile

A 48% aqueous HBr solution (140 µL, 1.2 mmol) and water (1 mL) were added to 4-amino-2-methoxy-5-methylbenzonitrile (40 mg, 0.25 mmol), followed by adding dropwise thereto an aqueous solution (0.5 mL) of sodium nitrite (20 mg, 0.30 mmol) under ice-cooling, and the resulting mixture was stirred for 10 minutes to prepare a diazonium salt solution. Copper (I) bromide (177 mg, 1.2 mmol) was dissolved in a 48% aqueous HBr solution (5 mL), and the prepared diazonium salt solution was added dropwise thereto at room temperature and stirred at 50°C for 1 hour. A saturated aqueous sodium chloride solution was added to the reaction mixture, followed by extraction with ethyl acetate (twice), and the organic layer was washed with a saturated aqueous sodium chloride solution and a saturated aqueous sodium hydrogencarbonate solution. The washed organic layer was dried over magnesium sulfate and distilled under reduced pressure to remove the solvent, and the resulting residue was washed with 50% diethyl ether-hexane to obtain 4-bromo-2-methoxy-5-methylbenzonitrile (35 mg, 63%) as white powder.

### (f) Synthesis of 4-[(1-benzylpiperidin-4-yl)amino]-2-methoxy-5-methylbenzonitrile

The title compound was synthesized by carrying out reaction according to the method described in Example 105 (a), except for using 4-bromo-2-methoxy-5-methylbenzonitrile as a starting material.

### (g) Synthesis of 4-[(1-benzylpiperidin-4-yl)amino]-2-methoxy-5-methylbenzamide

The title compound was synthesized by carrying out reaction according to the method described in Example 41 (b), except for using 4-[(1-benzyl-piperidin-4-yl)amino]-2-methoxy-5-methylbenzonitrile as a starting material.

Melting point: 153-156°C.
¹H-NMR (CDCl₃) δ;1.50-1.70 (2H, m) , 2.00 -2.14 (5H, m) , 2.22 (2H, m) , 2.78-2.95 (2H, m) , 3.40 (1H, m) , 3.55 (2H, s), 3.82 (1H, d, J =7. 5Hz), 3.92 (3H, s) , 5.53 (1H, br s) , 6.1 1 (1H, s), 7.25-7.40 (5H, m), 7.57 (1 H, br s) , 7.87 (1H, s).

### Example 143

### Synthesis of 2-methoxy-5-methyl-4-(piperidin-4-ylamino)benzamide

The title compound was synthesized by carrying out reaction according to the method described in Example 14, except for using the 4-[(1-benzylpiperidin-4-yl)amino]-2-methoxy-5-methylbenzamide obtained in Example 141, as a starting material.

Melting point: 194-196°C.
¹H-NMR (DMSO-d₆) δ;1.35 (2H, m) , 1.76-1. 90 (2H, m), 2.50-2.70 (2H, m), 2.85-3.00 (2 H, m) , 2.30-3.50 (1H, m) , 3.84 (3H, s), 4.91 (1H, d, J=7.9Hz), 6.16 (1H, s) , 7.02 (1H, b r s) , 7.33 (1H, br s), 7.52 (1H, s).

### Example 144

### Synthesis of 5-chloro-2-methoxy-4-(piperidin-4-yloxy)benzamide

### (a) Synthesis of methyl 5-chloro-4-hydroxy-2-methoxybenzoate

A 48% aqueous HBF₄ solution (1.6 mL, 12.4 mmol) and water (5 mL) were added to 4-amino-5-chloro-2-methoxybenzoic acid (1.0 g, 5.0 mmol), followed by adding dropwise thereto an aqueous solution (3 mL) of sodium nitrite (345 mg, 5.5 mmol) under ice-cooling, and the resulting mixture was stirred for 20 minutes. The white precipitate formed was collected by filtration to isolate a diazonium salt (wet weight: 1.3 g). The diazonium salt was suspended in acetic acid (50 mL) and the resulting suspension was stirred at 100°C for 1 hour. After the reaction mixture was allowed to stand at room temperature for 24 hours, a saturated aqueous sodium chloride solution was added thereto, followed by extraction with ethyl acetate (three times), and the organic layer was washed with a saturated aqueous sodium chloride solution. The washed organic layer was dried over magnesium sulfate and distilled under reduced pressure to remove the solvent. The resulting residue was dissolved in methanol (20 mL), followed by adding dropwise thereto thionyl chloride (1 mL) under ice-cooling, and the resulting mixture was warmed up to room temperature and then stirred overnight. The reaction mixture was concentrated under reduced pressure and the residue was purified by a silica gel chromatography. The crystals thus obtained were washed with 50% diethyl ether-hexane to obtain methyl 5-chloro-4-hydroxy-2-methoxybenzoate (518 mg, 48%) as dark-brown powder.

### (b) Synthesis of 5-chloro-2-methoxy-4-(piperidin-4-yloxy)benzamide

Toluene (20 mL), 1-tert-butoxycarbonyl-4-hydroxypiperidine (409 mg, 2.03 mmol) and cyanomethylenetri-n-butylphosphorane (490 mg, 2.03 mmol) were added to methyl 5-chloro-4-hydroxy-2-methoxybenzoate (220 mg, 1.01 mmol), and the resulting mixture was stirred at 100°C for 4 hours. After the reaction mixture was allowed to cool, a saturated aqueous sodium chloride solution was poured thereinto, followed by extraction with ethyl acetate (twice), and the organic layer was washed with a saturated aqueous sodium chloride solution. The washed organic layer was dried over magnesium sulfate and distilled under reduced pressure to remove the solvent, and the resulting residue was purified by a silica gel chromatography to obtain tert-butyl 4-[2-chloro-5-methoxy-4-(methoxycarbonyl)phenoxy]piperidine-1-carboxylate (510 mg) as a pale-brown oil. Trifluoroacetic acid (10 mL) was added to a solution of the oil in dichloromethane (15 mL) at room temperature and stirred for 1 hour. Then, the solvent of the reaction mixture was distilled off under reduced pressure to obtain crude methyl 5-chloro-2-methoxy-4-(piperidin-4-yloxy)benzoate trifluoroacetate as a reddish-brown oil. This oil was dissolved in N,N-dimethylformamide (10 mL), followed by adding thereto formamide (200 µL, 5.0 mmol) and a 25% sodium methoxide-methanol solution (950 µL, 6.0 mmol), and the resulting mixture was stirred at 80°C for 3 hours. Then, ammonium formate (20 g) was added to the reaction mixture and the resulting mixture was stirred with heating at 170 to 180°C for 6 hours. The solvent was distilled off under reduced pressure and the resulting residue was purified by a silica gel chromatography (chloroform : methanol = 99 : 1) to obtain 5-chloro-4-[(1-formylpiperidin-4-yl)oxy]-2-methoxybenzamide (112 mg) as a colorless oil. Thionyl chloride (130 µL, 1.76 mmol) was added dropwise to a solution of the colorless oil (110 mg, 0.35 mmol) in methanol (10 mL) under ice-cooling, and the resulting mixture was heated to 60°C and then stirred for 2 hours. The solvent of the reaction mixture was distilled off under reduced pressure and the resulting residue was purified by a silica gel chromatography (chloroform : methanol : 28% aqueous ammonia = 90 : 10 : 0.5) to obtain 5-chloro-2-methoxy-4-(piperidin-4-yloxy)benzamide (26 mg, 26%) as white powder.
¹H-NMR (DMSO-d₆) δ ; 1.45-1.65(2H, m), 1. 78-2.00 (2H, m), 2.55-2.68 (2H, m), 2 90-3. 00(2H, m), 3.92 (3H, s), 4.74 (1H, m), 6.85 (1H, s), 7.45-7.60 (2H, brm), 7.82 (1H, s).

### Example 145

### Synthesis of 5-chloro-2-fluoro-4-(piperidin-4-ylamino)benzamide

### (a) Synthesis of tert-butyl 4-{[4-(aminocarbonyl)-2-chloro-5-fluorophenyl]amino}piperidine-1-carboxylate and tert-butyl 4-{[4-(aminocarbonyl)-2-chloro-3-fluorophenyl]amino}piperidine-1-carboxylate

The tert-butyl 4-{[4-(aminocarbonyl)-3-fluorophenyl]amino}piperidine-1-carboxylate (290 mg, 0.86 mmol) obtained in Example 65 (c) and N-chlorosuccinimide (115 mg, 0.86 mmol) were added to isopropanol (20 mL), and the resulting mixture was stirred at 60°C for 1 hour. After cooling, the solution of the reaction mixture was distilled off under reduced pressure and the residue was separated and purified by preparative TLC. Then, the crude crystals thus obtained were washed with diethyl ether to obtain tert-butyl 4-{[4-(aminocarbonyl)-2-chloro-5-fluorophenyl]amino}piperidine-1-carboxylate (102 mg, 32%) and tert-butyl 4-{[4-(aminocarbonyl)-2-chloro-3-fluorophenyl]amino}piperidine-1-carboxylate (55 mg, 17%) as white crystalline powder.

### (b) Synthesis of 5-chloro-2-fluoro-4-(piperidin-4-ylamino)benzamide

The title compound was synthesized by carrying out reaction according to the method described in Example 45 (c), except for using tert-butyl 4-{[4-(aminocarbonyl)-2-chloro-5-fluorophenyl]amino}-piperidine-1-carboxylate as a starting material.
¹H-NMR (DMSO-d₆) δ;1.30-1.45 (2H, m) , 1. 72-1.85 (2H, m) , 2.50-2.60 (2H, m) , 2.85-2. 95 (2H, m) , 3.30-3.50 (1H, m) , 5.55 (1H, d, J =7. 9Hz), 6.63 (1H, d, J=14.2Hz) , 7.22 (1H, br s), 7.37 (1H, br s), 7.61 (1H, d, J=7.9Hz).

### Example 146

### Synthesis of 3-chloro-2-fluoro-4-(piperidin-4-ylamino)benzamide

The title compound was synthesized by carrying out reaction according to the method described in Example 45 (c), except for using the tert-butyl 4-{[4-(aminocarbonyl)-2-chloro-3-fluorophenyl]-amino}piperidine-1-carboxylate obtained in Example 145 (a), as a starting material.
¹H-NMR (DMSO-d₆) δ ; 1.32-1.50 (2H, m) , 1. 74-1.88 (2H, m) , 2.50-2.62 (2H, m) , 2.90-3. 00(2H, m), 3.35-3.55 (1H, m) , 5.58 (1H, d, J =8.2H z) , 6.66 (1H, d, J=8.6Hz) , 7.25-7.40 (2H, br m), 7.51(1H, t, J=8.8Hz).

### Example 147

### Synthesis of 4-[(1-benzylpiperidin-4-yl)amino]-2,6-difluorobenzamide

### (a) Synthesis of 4-[(1-benzylpiperidin-4-yl)amino]-2,6-difluorobenzonitrile

Potassium carbonate (2.63 g, 19.1 mmol) was added to a solution of 2,4,6-trifluorobenzonitrile (1.0 g, 6.37 mmol) in N,N-dimethylformamide (5 mL), followed by quickly dropping thereinto 4-amino-1-benzylpiperidine (1.33 mL, 6.37 mmol) with stirring at room temperature, and the resulting mixture was stirred for another 1 hour. A saturated aqueous sodium chloride solution and a saturated aqueous sodium hydrogencarbonate solution were added to the reaction mixture, followed by extraction with ethyl acetate (twice), and the organic layer was washed with a saturated aqueous sodium chloride solution. The washed organic layer was dried over magnesium sulfate and distilled under reduced pressure to remove the solvent, and the resulting residue was purified by a silica gel chromatography (20 ∼ 50% ethyl acetate-hexane) to obtain 4-[(1-benzylpiperidin-4-yl)amino]-2,6-difluorobenzonitrile (505 mg, 24%) (colorless needles).

### (b) Synthesis of 4-[(1-benzylpiperidin-4-yl)amino]-2,6-difluorobenzamide

The title compound was synthesized by carrying out reaction according to the method described in Example 41 (b), except for using 4-[(1-benzylpiperidin-4-yl)amino]-2,6-difluorobenzonitrile as a starting material.

Melting point: 201-203°C.
¹H-NMR (DMSO-d₆) δ;1.25-1.45 (2H, m) , 1.7 5-1.95 (2H, m) , 2.00-2.15 (2H, m) , 2.68-2. 80 (2H, m) , 3.16-3.30 (1H, m) , 3.45 (2H, s) , 6.20 (2H, d, J=11.7Hz), 6.38 (1H, d, J=7.9H z) , 7.20-7. 35 (5H, m) , 7.40 (1H, br s), 7.60 (1H, s).

### Example 148

### Synthesis of 2,6-difluoro-4-(piperidin-4-ylamino)benzamide

The title compound was synthesized by carrying out reaction according to the method described in Example 14, except for using the 4-[(1-benzylpiperidin-4-yl)amino]-2,6-difluorobenzamide obtained in Example 147 (b), as a starting material.

Melting point: 166-168°C.
¹H-NMR (DMSO-d₆) δ;1.10-1.30 (2H, m) , 1. 7 0-1.90 (2H, m) , 2.51-2.60 (2H, m) , 2.85-3. 00 (2H, m), 3.16 - 3.40 (1H, m) , 6.20 (2H, d, J =11.7Hz) , 6.38 (1H, d, J=8.0Hz), 7.40 (1H, b r s) , 7. 59 (1H, s).

### Example 149

### Synthesis of 2-fluoro-4-{[trans-4-(isopropylamino)cyclohexyl]amino}benzamide

Acetone (0.052 ml, 0.716 mmol), acetic acid (0.122 ml, 2.14 mmol) and sodium cyanoborohydride (54 mg, 0.859 mmol) were added to a solution of the 4-[trans-(4-aminocyclohexyl)amino]-2-fluorobenzamide obtained in Example 140 (180 mg, 0.716 mmol) in methanol (8 ml), and the resulting mixture was stirred at room temperature for 20 hours. During the stirring, acetone (0.052 ml, 0.716 mmol) and sodium cyanoborohydride (45 mg, 0.716 mmol) were added thereto. The reaction mixture was poured into a saturated aqueous sodium hydrogencarbonate solution and extracted with ethyl acetate. After the organic layer was extracted with 0.25N-hydrochloric acid, the aqueous layer was adjusted to pH 11 with a 1N-aqueous sodium hydroxide solution and extracted with ethyl acetate. The organic layer was dried over anhydrous sodium sulfate and filtered, and the filtrate was concentrated under reduced pressure. The resulting residue was purified by a silica gel column chromatography (eluent: chloroform/methanol/aqueous ammonia = 100/10/1) to obtain 2-fluoro-4-{[trans-4-(isopropylamino)-cyclohexyl]amino}benzamide (160 mg, 76%).

Melting point: 174-176°C.

### Example 150

### Synthesis of 2-fluoro-4-[(4-nitrophenyl)-amino]benzamide

### (a) Synthesis of 2-fluoro-4-[(4-nitrophenyl)-amino]benzonitrile

The title compound was synthesized by carrying out reaction according to the method described in Example 105 (a), except for using 4-bromo-2-fluorobenzonitrile as a starting material in place of 4-bromo-2-methoxybenzonitrile and using 4-nitroaniline in place of trans-tert-butyl 4-aminocyclohexylcarbamate.

### (b) Synthesis of 2-fluoro-4-[(4-nitrophenyl)-amino]benzamide

A mixture of 2-fluoro-4-[(4-nitrophenyl)-amino]benzonitrile (298 mg, 1.16 mmol), 1N-aqueous sodium hydroxide solution (3.5 mL) and ethanol (20 mL) was stirred at 90°C for 8.5 hours. The solvent was distilled off and a 1N-aqueous sodium hydroxide solution (30 mL) was added to the residue, followed by three runs of extraction with ethyl acetate (30 mL). The organic layer was dried over magnesium sulfate and distilled to remove the solvent, and the solid residue was purified by a silica gel column chromatography (eluent: hexane/ethyl acetate = 3 : 2 to 1 : 2) to obtain 45.8 mg (a brown solid, 0.166 mmol, 14%) of 2-fluoro-4-[(4-nitrophenyl)amino]benzamide.
IR (neat) : 1585, 1304, 1265, 1111, 829 cm⁻¹.

### Example 151

### Synthesis of 2-fluoro-4-[trans-(4-hydroxycyclohexyl)amino]benzamide

### (a) Synthesis of 2-fluoro-4-[trans-(4-hydroxycyclohexyl)amino]benzonitrile

A mixture of 2,4-difluorobenzonitrile (417 mg, 3.00 mmol), trans-4-aminocyclohexanol (380 mg, 3.30 mmol) and pyridine (8 mL) was stirred at 50°C for 5 hours. The solvent was distilled off and water (30 mL) was added to the residue, followed by three runs of extraction with ethyl acetate (30 mL). The organic layer was dried over magnesium sulfate and distilled to remove the solvent, and the solid residue was purified by a silica gel column chromatography (eluent: hexane/ethyl acetate = 2 : 1 to 1 : 2) to obtain 84.3 mg (a brown solid, 0.360 mmol, 12%) of 2-fluoro-4-[trans-(4-hydroxycyclohexyl)amino]benzonitrile.

### (b) Synthesis of 2-fluoro-4-[trans-(4-hydroxycyclohexyl)amino]benzamide

A 30%-aqueous hydrogen peroxide solution (0.30 mL) and then water (0.50 mL) were added dropwise to a mixture of 2-fluoro-4-[trans-(4-hydroxycyclohexyl)amino]benzonitrile (105 mg, 0.448 mmol), lithium hydroxide (32.2 mg, 1.34 mmol) and methanol (2.5 mL) at room temperature and stirred at room temperature for 4 hours. The reaction was stopped by the addition of a 10%-aqueous sodium thiosulfate solution, and the solvent was distilled off. A 1N-aqueous sodium hydroxide solution (20 mL) was added to the residue, followed by two runs of extraction with ethyl acetate (20 mL). The organic layer was dried over magnesium sulfate and distilled to remove the solvent, and the solid residue was purified by a silica gel column chromatography (eluent: chloroform/methanol = 30 : 1) to obtain 77.8 mg (a white solid, 0.308 mmol, 62%) of 2-fluoro-4-[trans-(4-hydroxycyclohexyl)amino]benzamide. IR (neat) : 3321, 1601, 1361, 1098, 829, 771 cm⁻¹.

### Example 152

### Synthesis of 4-(1-azabicyclo[2.2.2]oct-3-ylamino)-2-fluorobenzamide

The title compound was synthesized by carrying out reaction according to the method described in Example 10, except for using the 4-amino-2-fluorobenzamide obtained in Example 65 (b), as a starting material in place of p-aminobenzamide and using quinuclidin-3-one in place of 1-benzyl-4-piperidone.
IR (neat) : 3166, 1604, 1381, 1095, 818 cm⁻¹.

### Example 153

### Synthesis of 4-[(cis-4-aminocyclohexyl)-amino]-2-fluorobenzamide

### (a) Synthesis of cis-tert-butyl 4-[(4-cyano-3-fluorophenyl)amino]cyclohexylcarbamate

The title compound was synthesized by carrying out reaction according to the method described in Example 105 (a), except for using 4-bromo-2-fluorobenzonitrile as a starting material in place of 4-bromo-2-methoxybenzonitrile and using cis-tert-butyl 4-aminocyclohexylcarbamate in place of trans-tert-butyl 4-aminocyclohexylcarbamate.

### (b) Synthesis of 4-[(cis-4-aminocyclohexyl)amino]-2-fluorobenzamide

The title compound was synthesized by carrying out reaction according to the method described in Example 59 (d), except for using cis-tert-butyl 4-[(4-cyano-3-fluorophenyl)amino]cyclohexylcarbamate as a starting material.
IR (neat) : 2931, 1620, 1377, 1091, 825 cm⁻¹.

### Example 154

### Synthesis of 4-[(cis-4-aminocyclohexyl)-amino]-5-chloro-2-fluorobenzamide

### (a) Synthesis of cis-tert-butyl 4-[(2-chloro-4-cyano-5-fluorophenyl)amino]cyclohexylcarbamate

The title compound was synthesized by carrying out reaction according to the method described in Example 45 (b), except for using the cis-tert-butyl 4-[(4-cyano-3-fluorophenyl)amino]cyclohexylcarbamate obtained in Example 153 (a), as a starting material.

### (b) Synthesis of 4-[(cis-4-aminocyclohexyl)amino]-5-chloro-2-fluorobenzamide

The title compound was synthesized by carrying out reaction according to the method described in Example 59 (d), except for using cis-tert-butyl 4-[(2-chloro-4-cyano-5-fluorophenyl)amino]cyclohexylcarbamate as a starting material.
IR (neat) : 2916, 1612, 1369, 1111, 922 cm⁻¹.

### Example 155

### Synthesis of 3-chloro-4-(pyrrolidin-3-ylamino)benzamide

### (a) Synthesis of tert-butyl 3-hydroxypyrrolidine-1-carboxylate

A solution of di-tert-butyl carbonate (13.8 g, 63.1 mmol) in dichloromethane was added to a solution of 3-pyrrolidinol (5 g, 57.4 mmol) in dichloromethane (50 mL), and the resulting mixture was allowed to stand overnight. After the solvent was distilled off under reduced pressure, the residue was purified by a silica gel chromatography to obtain tert-butyl 3-hydroxypyrrolidine-1-carboxylate (9.23 g, 86%).

### (b) Synthesis of tert-butyl 3-aminopyrrolidine-1-carboxylate

Triethylamine (0.75 mL, 5.34 mmol) and methanesulfonyl chloride (0.31 mL, 4.00 mmol) were added to a solution of tert-butyl 3-hydroxypyrrolidine-1-carboxylate (500 mg, 2.67 mmol) in tetrahydrofuran (20 mL) at 0°C and stirred at the same temperature for 1.5 hours. Water and ethyl acetate were added to the reaction mixture, followed by extraction with ethyl acetate, and the extract solution was dried over anhydrous magnesium sulfate. The concentration residue was dissolved in N-methylpyrrolidinone (20 mL), followed by adding thereto sodium azide (520.8 mg, 8.01 mmol) at 0°C, and the resulting mixture was stirred with heating at 80°C for 3 hours. After the reaction mixture was cooled to 0°C, water and diethyl ether were added thereto, followed by two runs of extraction with diethyl ether, and the extract solution was dried over anhydrous magnesium sulfate. The solvent was distilled off under reduced pressure to obtain an azide.

To a solution of this azide in methanol (50 mL) was added palladium-carbon (250 mg) under a nitrogen atmosphere, and the resulting mixture was stirred for 3 hours under a hydrogen atmosphere. The reaction mixture was filtered by the use of Celite and the filtrate was distilled under reduced pressure to remove the solvent. The residue was purified by a silica gel chromatography to obtain tert-butyl 3-aminopyrrolidine-1-carboxylate (365.6 mg, 73%).

### (c) Synthesis of tert-butyl 3-[(4-cyanophenyl)amino]-pyrrolidine-1-carboxylate

A solution of tert-butyl 3-aminopyrrolidine-1-carboxylate (360 mg, 2.01 mmol) in toluene (5 mL) was added to a mixture of 4-bromobenzonitrile (330 mg, 1.83 mmol), tris(dibenzylideneacetone)dipalladium (16.8 mg, 0.03 mmol), (+)-2,2'-bis(diphenylphosphino)-1,1'-binaphthyl (34.2 mg, 0.08 mmol) and sodium tert-butoxide (246.6 mg, 2.57 mmol), and the resulting mixture was stirred at 80°C for 4 hours. The reaction mixture was distilled under reduced pressure to remove the solvent, and the residue was washed with 2-propanol by repulping to obtain tert-butyl 3-[(4-cyanophenyl)amino]pyrrolidine-1-carboxylate (274.4 mg, 52%).

### (d) Synthesis of tert-butyl 3-[(2-chloro-4-cyanophenyl)amino]pyrrolidine-1-carboxylate

The title compound was synthesized by carrying out reaction according to the method described in Example 45 (b), except for using tert-butyl 3-[(4-cyanophenyl)amino]pyrrolidine-1-carboxylate as a starting material.

### (e) Synthesis of 3-chloro-4-(pyrrolidin-3-ylamino)benzamide

The title compound was synthesized by carrying out reaction according to the method described in Example 41 (b), except for using tert-butyl 3-[(2-chloro-4-cyanophenyl)amino]pyrrolidine-1-carboxylate as a starting material.
¹H-NMR (DMSO-d₆) δ ; 1.58(1H, m) , 2.05 (1H, m) , 2.71 (2H, m) , 2.90 (1H, m) , 2.97(2H, m) , 3.92 (1H, m) , 5.45 (1H, d, J=6.78Hz ) , 6.74 (1H, d, J=8.80Hz), 7.08 (1H, br) , 7.68(1H, dd, J=2.01, 8.06Hz) , 7.73(1H, br) , 7.79(1 H, d, J=2.01Hz).

### Example 156

### Synthesis of 4-(azepan-3-ylamino)-3-chlorobenzamide

### (a) Synthesis of tert-butyl 3-aminoazepane-1-carboxylate

The title compound was synthesized by carrying out reaction according to the method described in Example 155 (b), except for using tert-butyl 3-hydroxyazepane-1-carboxylate as a starting material.

### (b) Synthesis of tert-butyl 3-[(4-cyanophenyl)amino]-azepane-1-carboxylate

The title compound was synthesized by carrying out reaction according to the method described in Example 155 (c), except for using tert-butyl 3-aminoazepane-1-carboxylate as a starting material.

### (c) Synthesis of tert-butyl 3-[(2-chloro-4-cyanophenyl)amino]azepane-1-carboxylate

The title compound was synthesized by carrying out reaction according to the method described in Example 45 (b), except for using tert-butyl 3-[(4-cyanophenyl)amino]azepane-1-carboxylate as a starting material.

### (d) Synthesis of 4-(azepan-3-ylamino)-3-chlorobenzamide

The title compound was synthesized by carrying out reaction according to the method described in Example 41 (b), except for using tert-butyl 3-[(2-chloro-4-cyanophenyl)amino]azepane-1-carboxylate as a starting material.
¹H-NMR (DMSO-d₆) δ ; 1.52 (1H, m) , 1.64-1.8 7 (5H, m) , 2.88-3.05 (4H, m) , 3.89(1H, br), 5.67 (1H, d, J=8.80Hz) , 6.81 (1H, d, J=8.62 Hz) , 7.10 (1H, br) , 7.70 (1H, d, J=8.62Hz) , 7.76 (1H, br) , 7.82 (1H, d, J=1.29Hz).

### Example 157

### Synthesis of 3-chloro-5-fluoro-4-(piperidin-4-ylamino)benzamide

### (a) Synthesis of tert-butyl 4-{[4-(aminocarbonyl)-2-chloro-6-fluorophenyl]amino}piperidine-1-carboxylate

The title compound was synthesized by carrying out reaction according to the method described in Example 45 (b), except for using the tert-butyl 4-{[4-(aminocarbonyl)-2-fluorophenyl]amino}piperidine-1-carboxylate obtained in Example 68 (d), as a starting material.

### (b) Synthesis of 3-chloro-5-fluoro-4-(piperidin-4-ylamino)benzamide

Hydrochloric acid-dioxane (25 mL) was added to tert-butyl 4-{[4-(aminocarbonyl)-2-chloro-6-fluorophenyl]amino}piperidine-1-carboxylate and stirred overnight. The precipitate was collected by filtration and then purified by a silica gel chromatography to obtain 3-chloro-5-fluoro-4-(piperidin-4-ylamino)benzamide (505.5 mg, 34% from 4-amino-3-fluorobenzamide).
¹H-NMR (DMSO-d₆) δ ; 1.36 (2H, m) , 1.80 (2H, m), 2.94(2H, m), 3.59 (1H, m) , 4.77 (1H, dd, J=1.84, 8.99 H z), 7.33 (1H, br) , 7.58 (1H, d d, J=2.02, 14.02Hz) , 7.72 (d, 1H, J=2.02H z), 7.88 (1H, br).

### Example 158

### Synthesis of 4-[(trans-4-aminocyclohexyl)amino]-5-fluoro-2-methoxybenzamide

### (a) Synthesis of tert-butyl (trans-4-[(4-cyano-2,5-difluorophenyl)amino]cyclohexyl)carbamate

Potassium carbonate (2.63 g, 19.1 mmol) was added to a solution of 2,4,5-trifluorobenzonitrile (1.0 g, 6.37 mmol) in dimethylformamide (20 mL), and a solution of trans-tert-butyl 4-aminocyclohexylcarbamate (1.50 g, 7.0 mmol) in dimethylformamide (10 mL) was quickly dropped thereinto with stirring under ice-cooling. The resulting mixture was warmed up to room temperature and then stirred for another 3 hours. A saturated aqueous sodium chloride solution and a saturated aqueous sodium hydrogencarbonate solution were added to the reaction solution, followed by extraction with ethyl acetate (twice), and the organic layer was washed with a saturated aqueous sodium chloride solution. The washed organic layer was dried over magnesium sulfate and distilled under reduced pressure to remove the solvent, and the resulting residue was washed with diethyl ether-hexane to obtain tert-butyl (trans-4-[(4-cyano-2,5-difluorophenyl)amino]cyclohexyl)carbamate (1.1 g, 50%) as white powder.

### (b) Synthesis of 4-[(trans-4-aminocyclohexyl)amino]-5-fluoro-2-methoxybenzonitrile

Trifluoroacetic acid (5 mL) was added to a solution of tert-butyl (trans-4-[(4-cyano-2,5-difluorophenyl)amino]cyclohexyl)carbamate (351 mg, 1.0 mmol) in dichloromethane (5 mL) under ice-cooling, and the resulting mixture was warmed up to room temperature and then stirred for 2 hours. The reaction mixture was concentrated under reduced pressure, followed by adding thereto tetrahydrofuran (5 mL) and a solution of sodium methoxide in methanol (574 µL, 10 mmol), and the resulting mixture was heated under reflux for 12 hours. A saturated aqueous sodium chloride solution was added to the reaction mixture, followed by extraction with ethyl acetate (twice), and the organic layer was washed with a saturated aqueous sodium chloride solution. The washed organic layer was dried over magnesium sulfate and distilled under reduced pressure to remove the solvent, and the resulting residue was purified by a silica gel chromatography to obtain 4-[(trans-4-aminocyclohexyl)amino]-5-fluoro-2-methoxybenzonitrile (187 mg, 71%) as a colorless oil.

### (c) Synthesis of 4-[(trans-4-aminocyclohexyl)amino]-5-fluoro-2-methoxybenzamide

The title compound was synthesized by carrying out reaction according to the method described in Example 41 (b), except for using 4-[(trans-4-aminocyclohexyl)amino]-5-fluoro-2-methoxybenzonitrile as a starting material.
¹H-NMR (DMSO-d₆) δ;1.10-1.40 (4H, m) , 1.7 0-2.00 (6H, m) , 2.53 (1H, m) , 3.33 (1H, m) , 3. 86 (3H, s) , 5.72 (1H, m) , 6.30 (1H, d, J=7.3H z) , 7.24 (1H, br s), 7.35-7.50 (2H, m).

### Example 159

### Synthesis of 4-[(trans-4-aminocyclohexyl)-oxy]-5-chloro-2-methoxybenzamide

### (a) Synthesis of 4-([4-(1,3-dioxo-1,3-dihydro-2H-isoindol-2-yl)cyclohexyl]oxy)-2-fluorobenzonitrile

To a solution of 2-fluoro-4-hydroxybenzonitrile (4.0 g, 29 mmol) in tetrahydrofuran (150 mL) were added cis-2-(4-hydroxycyclohexyl)-lH-isoindole-1,3(2H)-dione (7.2 g, 29 mmol) and triphenylphosphine (9.2 g, 35 mmol), followed by adding dropwise thereto diisopropyl azodicarboxylate (8.3 mL, 35 mmol) under ice-cooling, and the resulting mixture was warmed up to room temperature and then stirred overnight. A saturated aqueous sodium chloride solution and a saturated aqueous sodium hydrogencarbonate solution were added to the reaction mixture, followed by extraction with ethyl acetate (twice), and the organic layer was washed with a saturated aqueous sodium chloride solution. The washed organic layer was dried over magnesium sulfate and distilled under reduced pressure to remove the solvent, and the resulting residue was purified by a silica gel chromatography and then washed with diethyl ether to obtain 4-([4-(1,3-dioxo-1,3-dihydro-2H-isoindol-2-yl)cyclohexyl]oxy)-2-fluorobenzonitrile (3.4 g, 32%) as white powder.

### (b) Synthesis of tert-butyl 4-(4-cyano-3-fluorophenoxy)cyclohexylcarbamate

A solution of methylamine in ethanol (80 mL) was added to a solution of 4-([4-(1,3-dioxo-1,3-dihydro-2H-isoindol-2-yl)cyclohexyl]oxy)-2-fluorobenzonitrile (3.3 g, 9.1 mmol) in tetrahydrofuran (20 mL), and the resulting mixture was stirred at room temperature for 1 hour and then at 60°C for 2 hours. After cooling, the solvent of the reaction mixture was distilled off under reduced pressure and the residue was dissolved in tetrahydrofuran (100 mL). Then, di-tert-butyl dicarbonate (2.2 g, 10 mmol) was added thereto with stirring under ice-cooling. The resulting mixture was warmed up to room temperature and stirred for 1 hour. Thereafter, the reaction solution was concentrated under reduced pressure and the resulting residue was purified by a silica gel chromatography (eluent: 3 ∼ 12% ethyl acetate-hexane). The resulting residue was washed with diethyl ether-hexane to obtain tert-butyl 4-(4-cyano-3-fluorophenoxy)cyclohexylcarbamate (2.1 g, 68%) as colorless needles.

### (c) Synthesis of tert-butyl 4-(4-cyano-3-methoxyphenoxy)cyclohexylcarbamate

A 28%-sodium methoxide-methanol solution (1.3 mL, 22.4 mmol) was added to a solution of tert-butyl 4-(4-cyano-3-fluorophenoxy)cyclohexylcarbamate (1.5 g, 4.5 mmol) in dimethylformamide (60 mL) under ice-cooling and stirred for 3 hours. The reaction mixture was poured into a cold aqueous ammonium chloride solution and extracted twice with ethyl acetate, and the organic layer was washed with a saturated aqueous sodium chloride solution. The washed organic layer was dried over magnesium sulfate and distilled under reduced pressure to remove the solvent, and the resulting residue was washed with diethyl ether-hexane to obtain tert-butyl 4-(4-cyano-3-methoxyphenoxy)-cyclohexylcarbamate (1.3 g, 84%) as colorless needles.

### (d) Synthesis of 4-[(trans-4-aminocyclohexyl)oxy]-5-chloro-2-methoxybenzonitrile

N-chlorosuccinimide (1.93 g, 14.4 mmol) was added to a solution of tert-butyl 4-(4-cyano-3-methoxyphenoxy)cyclohexylcarbamate (1.0 g, 2.9 mmol) in 2-propanol (50 mL) at room temperature and stirred at 50°C for 40 minutes. The reaction mixture was allowed to cool to room temperature, followed by adding thereto N-chlorosuccinimide (580 mg, 4.4 mmol), and the resulting mixture was stirred with heating at 50°C for another 1 hour. The reaction mixture was allowed to cool and then concentrated to dryness under reduced pressure, and the residue was purified by a silica gel chromatography (eluent: chloroform : methanol : aqueous ammonia = 100 : 10 : 1). The resulting residue was washed with chloroform- hexane to obtain 4-[(trans-4-aminocyclohexyl)oxy]-5-chloro-2-methoxybenzonitrile (635 mg, 78%) as colorless needles.

### (e) Synthesis of 4-[(trans-4-aminocyclohexyl)oxy]-5-chloro-2-methoxybenzamide

Lithium hydroxide (51 mg, 2.1 mmol), a 30% aqueous hydrogen peroxide solution (404 mg, 3.5 mmol) and water (2 mL) were added to a solution of 4-[(trans-4-aminocyclohexyl)oxy]-5-chloro-2-methoxybenzonitrile (200 mg, 0.71 mmol) in methanol (8 mL), and the resulting mixture was stirred at 50°C for 3 hours. The reaction mixture was poured into a saturated aqueous sodium chloride solution and aqueous ammonia was added thereto, followed by extraction with ethyl acetate (twice). The organic layer was washed with a saturated aqueous sodium chloride solution and a 5% aqueous sodium thiosulfate solution, dried over magnesium sulfate and then distilled under reduced pressure to remove the solvent, and the resulting residue was washed with methanol-diethyl ether to obtain 4-[(trans-4-aminocyclohexyl)oxy]-5-chloro-2-methoxybenzamide (88 mg, 41%) as white powder.
¹H-NMR (DMSO-d₆) δ ; 1.10-1.30 (2H, m) , 1.3 5-1.55 (2H, m) , 1.70-1.85 (2H, m) , 1.95-2. 10(2H, m) , 2.66 (1H, m) , 3.92 (3H,s ) , 4.55 (1H, m), 6.83 (1H, s), 7.52 (1H, brm) , 7.81 (1H, s).

### Example 160

### Synthesis of 4-[(trans-4-aminocyclohexyl)-oxy]-2-fluorobenzamide

### (a) Synthesis of tert-butyl (trans-4-[4-(aminocarbonyl)-3-fluorophenoxy]cyclohexyl)carbamate

Lithium hydroxide (45 mg, 1.9 mmol), a 30% aqueous hydrogen peroxide solution (356 mg, 3.1 mmol) and water (2 mL) were added to a solution of the tert-butyl 4-(4-cyano-3-fluorophenoxy)cyclohexylcarbamate obtained in Example 159 (b) (210 mg, 0.63 mmol) in methanol (5 mL), and the resulting mixture was stirred at room temperature for 8 hours. A saturated aqueous sodium chloride solution was poured into the reaction mixture, followed by extraction with ethyl acetate (twice). The organic layer was washed with a saturated aqueous sodium chloride solution and a 5% aqueous sodium thiosulfate solution, dried over magnesium sulfate and then distilled under reduced pressure to remove the solvent, and the resulting residue was washed with diethyl ether-hexane to obtain tert-butyl (trans-4-[4-(aminocarbonyl)-3-fluorophenoxy]-cyclohexyl)carbamate (201 mg, 91%) as white powder.

### (b) Synthesis of 4-[(trans-4-aminocyclohexyl)oxy]-2-fluorobenzamide

Trifluoroacetic acid (8 mL) was added to a solution of tert-butyl (trans-4-[4-(aminocarbonyl)-3-fluorophenoxy]cyclohexyl)carbamate (195 mg, 0.55 mmol) in dichloromethane (8 mL), and the resulting mixture was stirred at room temperature for 1 hour. The reaction mixture was concentrated under reduced pressure and the resulting residue was purified by a silica gel chromatography (eluent: chloroform : methanol : aqueous ammonia = 200 : 20 : 1) to obtain 4-[(trans-4-aminocyclohexyl)oxy]-2-fluorobenzamide (101 mg, 72%) as white powder.
¹H-NMR (DMSO-d₆) δ;1.10-1.45 (4H, m) , 1. 5 5 (2H, br s) , 1.70-1.80 (2H, m) , 1.90-2.05 (2H, m) , 2.60 (1H, m) , 4.35 (1H, m) , 6.70-6. 95 (2H, m) , 7.41 (1H, br s) , 7.47 (1H, br s), 7.62 (1H, t, J=8. 6Hz).

### Example 161

### Synthesis of 4-[(trans-4-aminocyclohexyl)oxy]-5-chloro-2-fluorobenzamide

### (a) Synthesis of 4-[(trans-4-aminocyclohexyl)oxy]-5-chloro-2-fluorobenzonitrile

Sulfuryl chloride (288 µL, 3.6 mmol) was added to a solution of the tert-butyl 4-(4-cyano-3-fluorophenoxy)cyclohexylcarbamate obtained in Example 159 (b) (240 mg, 0.72 mmol) in acetic acid (3 mL), and the resulting mixture was stirred at 50°C for 10 hours. The reaction mixture was concentrated under reduced pressure and into the resulting residue was poured a 10% aqueous sodium carbonate solution, followed by extraction with ethyl acetate (three times). The organic layer was washed with a saturated aqueous sodium chloride solution, dried over magnesium sulfate and then distilled under reduced pressure to remove the solvent, and the resulting residue was purified by a silica gel chromatography. The crystals thus obtained were washed with diethyl ether to obtain 4-[(trans-4-aminocyclohexyl)oxy]-5-chloro-2-fluorobenzonitrile (114 mg, 59%) as white powder.

### (b) Synthesis of 4-[(trans-4-aminocyclohexyl)oxy]-5-chloro-2-fluorobenzamide

The title compound was synthesized by carrying out reaction according to the method described in Example 159 (e), except for using 4-[(trans-4-aminocyclohexyl)oxy]-5-chloro-2-fluorobenzonitrile as a starting material.
¹H-NMR (DMSO-d₆) δ ; 1.10-1.50(4H, m) , 1.5 0-1.90 (4H, m) , 1.90-2.20 (2H, m) , 2.65 (1H, m) , 4.48 (1H, m) , 7.26 (1H, d, J=12.8Hz), 7. 50-7.65(2H, brm) , 7.69(1H, d, J=7.7Hz).

### Example 162

### Synthesis of 4-[trans-(4-aminocyclohexyl)-amino]-2,5-difluorobenzamide

The title compound was synthesized by carrying out reaction according to the method described in Example 59 (d), except for using the tert-butyl trans-4-[(4-cyano-2,5-difluorophenyl)amino]cyclohexylcarbamate obtained in Example 158 (a), as a starting material.

Melting point: 209-210°C.

### Example 163

### Synthesis of 4-[trans-(4-aminocyclohexyl)-amino]-5-bromo-2-fluorobenzamide

### (a) Synthesis of tert-butyl trans-4-[(2-bromo-4-cyano-5-fluorophenyl)amino]cyclohexylcarbamate

The title compound was synthesized by carrying out reaction according to the method described in Example 63 (a), except for using the tert-butyl trans-4-[(4-cyano-3-fluorophenyl)amino]cyclohexylcarbamate obtained in Example 140 (a), as a starting material.

### (b) Synthesis of 4-[trans-(4-aminocyclohexyl)amino]-5-bromo-2-fluorobenzamide

The title compound was synthesized by carrying out reaction according to the method described in Example 59 (d), except for using tert-butyl trans-4-[(2-bromo-4-cyano-5-fluorophenyl)amino]cyclohexylcarbamate as a starting material.

Melting point: 171-173°C.

### Example 164

### Synthesis of 4-[(1-benzylpiperidin-4-yl)amino]-2,5-difluorobenzamide

### (a) Synthesis of 4-[(1-benzylpiperidin-4-yl)amino]-2,5-difluorobenzonitrile

Potassium carbonate (2.64 g, 19.1 mmol) was added to a solution of 2,4,5-trifluorobenzonitrile (1.00 g, 6.4 mmol) in N,N-dimethylformamide (30 ml), and the resulting mixture was ice-cooled, followed by adding thereto 4-amino-1-benzylpiperidine (1.36 ml, 6.7 mmol). The resulting mixture was slowly warmed up to room temperature and stirred overnight. The reaction solution was added to a saturated aqueous sodium hydrogencarbonate solution and extracted three times with toluene/ethyl acetate = 1/1, and the combined organic layer was washed once with a saturated aqueous sodium chloride solution and dried over anhydrous magnesium sulfate. The solvent was distilled off and the solid precipitated was suspended in hexane/diethyl ether to be washed. The washed solid was collected by filtration and dried under reduced pressure to obtain 4-[(1-benzylpiperidin-4-yl)amino]-2,5-difluorobenzonitrile (1.749 g, 83%).

### (b) Synthesis of 4-[(1-benzylpiperidin-4-yl)amino]-2,5-difluorobenzamide

The title compound was synthesized by carrying out reaction according to the method described in Example 59 (d), except for using 4-[(1-benzylpiperidin-4-yl)amino]-2,5-difluorobenzonitrile as a starting material.

Melting point: 195-197°C.

### Example 165

### Synthesis of 2,5-difluoro-4-(piperidin-4-ylamino)benzamide

The title compound was synthesized by carrying out reaction according to the method described in Example 14, except for using the 4-[(1-benzylpiperidin-4-yl)amino]-2,5-difluorobenzamide obtained in Example 164, as a starting material.

Melting point: 187.5-189.5°C.

### Example 166

### Synthesis of 2-fluoro-4-(1,2,3,6-tetrahydropyridin-4-ylmethyl)benzamide

### (a) Synthesis of diethyl (4-cyano-3-fluorobenzyl)phosphonate

Triethyl phosphite (1 ml) was added to 4-cyano-2-fluorobenzyl bromide (100 mg, 0.467 mmol), a compound known in literature, at room temperature under a nitrogen atmosphere, and the resulting mixture was adjusted to 120°C. After 1 hour, the reaction solution was concentrated under reduced pressure and the resulting residue was purified by a silica gel column chromatography (eluents: hexane/ethyl acetate and chloroform/ethyl acetate) to obtain diethyl (4-cyano-3-fluorobenzyl)phosphonate (120.6 mg, 95%).

### (b) Synthesis of tert-butyl 4-(4-cyano-3-fluorobenzylidene)piperidine-1-carboxylate

To a solution of diethyl (4-cyano-3-fluorobenzyl)phosphonate (50 mg, 0.184 mmol) in tetrahydrofuran (1 ml) was added 60%-sodium hydride (8.1 mg, 0.202 mmol) at 0°C under a nitrogen atmosphere. After 5 minutes, tert-butyl 4-oxo-1-piperidinecarboxylate (40 mg, 0.202 mmol) was added thereto. After 30 minutes, the reaction mixture was warmed up to room temperature. After another 2.5 hours, the reaction solution was poured into water and extracted with ethyl acetate. The organic layer was dried over anhydrous magnesium sulfate and concentrated under reduced pressure, and the resulting residue was purified by a silica gel column chromatography (eluent: hexane/ethyl acetate) to obtain tert-butyl 4-(4-cyano-3-fluorobenzylidene)piperidine-1-carboxylate (52.7 mg, 90%).

### (c) Synthesis of 2-fluoro-4-(1,2,3,6-tetrahydropyridin-4-ylmethyl)benzamide

Concentrated sulfuric acid (1 ml) was added to tert-butyl 4-(4-cyano-3-fluorobenzylidene)-piperidine-1-carboxylate (47 mg, 0.149 mmol) at room temperature under a nitrogen atmosphere, and the resulting mixture was adjusted to 60°C. After 1 hour, the reaction solution was poured onto ice, adjusted to pH 12 with a 10N-aqueous sodium hydroxide solution and then extracted with ethyl acetate. The organic layer was dried over anhydrous magnesium sulfate and concentrated under reduced pressure, and the resulting residue was purified by a silica gel column chromatography (eluents: chloroform/methanol and chloroform/methanol (1%-aqueous ammonia)) to obtain 2-fluoro-4-(1,2,3,6-tetrahydropyridin-4-ylmethyl)-benzamide (2.7 mg, 7.8%).
¹H-NMR (CDCl₃) δ;1.92 (2H, m) , 2.93 (2H, t, J=5.7Hz) , 3.32 (2H, m), 3.36 (2H, m) , 5.51 (1H, m) , 5.82 (1H, br s. ), 6.67 (1H, br s. ), 6. 99 (1H, d, J = 12.8Hz) , 7.11 (1H, d, J=8.2Hz) , 8.04 (1H, dd, J=8.1, 8.1Hz).

### Example 167

### Synthesis of 5-chloro-2-fluoro-4-({4-trans-[(3,3,3-trifluoropropyl)amino]cyclohexyl}-amino)benzamide

Diisopropylethylamine (238 µl, 1.37 mmol), 3,3,3-trifluoropropylamide (48 µl, 0.453 mmol) and potassium iodide (15 mg, 0.0904 mmol) were added to a solution of the 4-[trans-(4-aminocyclohexyl)amino]-5-chloro-2-fluorobenzamide obtained in Example 141 (130 mg, 0.455 mmol) in dimethylformamide (3 ml), and the resulting mixture was stirred at room temperature for 13 hours. The solvent was distilled off under reduced pressure, followed by five runs of azeotropy operation with toluene. Then, chloroform-methanol was added to the residue and the solid was removed, followed by concentration. The concentrate was dissolved in methanol and purified by a preparative thin-layer silica gel chromatography (0.5 mm x 20 cm x 20 cm; 4 plates; developed with: chloroform/methanol/aqueous ammonia = 20 : 1 : 0.1) to obtain 5-chloro-2-fluoro-4-({4-trans-[(3,3,3-trifluoropropyl)amino]cyclohexyl)amino)benzamide (23 mg, 13%) as a white solid.

Melting point: 161-161°C.

### Example 168

### Synthesis of 5-chloro-2-fluoro-4-({4-trans-[(2,2,2-trifluoroethyl)amino]cyclohexyl}amino)benzamide

Diisopropylethylamine (37 µl, 0.212 mmol) and 2,2,2-trifluoroethyl trifluoromethanesulfonate (10 µl, 0.0694 mmol) were added to a solution of the 4-[trans-(4-aminocyclohexyl)amino]-5-chloro-2-fluorobenzamide obtained in Example 141 (20 mg, 0.070 mmol) in dimethylformamide (1 ml), and the resulting mixture was stirred at room temperature for 20 hours. The solvent was distilled off under reduced pressure, followed by five runs of azeotropy operation with toluene. Then, the residue was dissolved in methanol and purified by a preparative thin-layer silica gel chromatography (0.5 mm x 20 cm x 20 cm; 2 plates; developed with: chloroform/methanol/aqueous ammonia = 20 : 1 : 0.1) to obtain 5-chloro-2-fluoro-4-({4-trans-[(2,2,2-trifluoroethyl)amino]cyclohexyl}amino)benzamide (15 mg, 58%) as a white solid.

Melting point: 157-158°C.

### Example 169

Synthesis of 5-chloro-2-fluoro-4-{[1-(3,3,3-trifluoropropyl)piperidin-4-yl]amino}benzamide

The title compound was synthesized by carrying out reaction according to the method described in Example 167, except for using the 5-chloro-2-fluoro-4-(piperidin-4-ylamino)benzamide obtained in Example 145, as a starting material.

Melting point: 130-132°C (decomp.).

### Example 170

### Synthesis of 5-chloro-2-fluoro-4-{[1-(2,2,2-trifluoroethyl)piperidin-4-yl]amino}benzamide

The title compound was synthesized by carrying out reaction according to the method described in Example 168, except for using the 5-chloro-2-fluoro-4-(piperidin-4-ylamino)benzamide obtained in Example 145, as a starting material.

Melting point: 172-173°C.

### Test Example 1

### Assay of the inhibition of phosphorylation by Rho kinase

A bovine brain extract fraction was prepared as follows. That is, a piece of gray matter was minced from bovine brain and suspended in a buffer solution for immunoprecipitation (10 mM tris(hydroxymethyl)aminomethane (Tris) (pH 7.5), 1% Triton X-100, 0.5% NP-40, 150 mM NaCl, 20 mM sodium fluoride, 1 mM ethylenediaminetetraacetic acid (EDTA), 1mM ethylene glycol bis(β-aminoethyl ether)-N,N,N',N'-tetraacetic acid (EGTA) and 0.2 mM phenylmethylsulfonyl fluoride (PMSF)) in two volumes of the gray matter. The tissue was homogenized by the use of a Potter type homogenizer made of polytetrafluoroethylene. After the homogenate was centrifuged at 20,000 g for 15 minutes, the supernatant was collected and then subjected to ultracentrifugation at 100,000 g for 60 minutes. The resulting supernatant was used as a bovine brain extract fraction.

An anti-Rho kinase antibody immobilized plate was prepared as follows. That is, a secondary antibody (anti-goat IgG (Fc) antibody) was diluted 300-fold with phosphate-buffered saline (PBS) (1 µl antibody/300 µl PBS), and 100 µl of the resulting dilution was added to each well of a 96-well ELISA plate. After coating at room temperature for 2 hours, the supernatant was removed. Thereafter, 100 µl of PBS was added and then the supernatant was removed (this washing operation was carried out twice). After the washing, 100 µl of a blocking buffer solution (0.05% Tween 20 and 0.25% bovine serum albumin (fatty acid free)/PBS) was added, followed by blocking at room temperature for 1 hour. After the blocking, each well was washed twice with 100 µl of the blocking buffer solution, and 100 µl of a primary antibody (anti-ROKII (Rho kinase) peptide antibody) diluted 200-fold (0.5 µl (0.1 µg)/100 µl) with PBS was added, followed by coating at room temperature for 2 hours. After the coating, each well was washed once with 100 µl of the blocking buffer solution. In addition, 100 µl of the blocking buffer solution was added to obtain an. anti-Rho kinase antibody immobilized plate.

Using the aforesaid plate, Rho kinase was selectively immobilized from the bovine brain extract fraction, and phosphorylation by Rho kinase was assayed. To each well of the antibody-immobilized plate was added 100 µl of a bovine brain extract solution prepared so as to have a concentration of 1.5 mg/ml, and the reactions were incubated at 4°C for 1 hour to immobilize Rho kinase on the plate. After completion of the reaction, the supernatant was removed and each well was washed three times with 100 µl of the buffer solution for immunoprecipitation. In addition, each well was washed three times with 100 µl of buffer solution A (50 mM Tris (pH 7.5), 10 mM MgCl₂ and 150 mM NaCl). To each well of the plate freed from the supernatant was added 40 µl of the aforesaid buffer solution for reaction (50 mM Tris-HCl (pH 7.5), 2 mM EDTA and 10 mM MgCl₂). Further, adenosine 5'-triphosphate (ATP) buffer (0.1 µM ATP (containing 6 nM [ν-³²P]ATP) and 10 µg histone (HF2A)) containing each compound was prepared, and 10 µl of this solution was added to each well of the plate to initiate the reaction. The reactions were carried out at room temperature for 4 hours. The reaction was terminated by the addition of a phosphoric acid solution of a final concentration of 75 mM and 50 µM ATP. After completion of the reaction, 50 µl of the reaction solutions were spotted onto a phosphocellulose filter for Beta Plate 1205 (Wallac) only. After completion of the spot, the filter was washed with 150 ml of a 75 mM phosphoric acid solution for 10 minutes. This washing operation was repeated three times. After completion of the washing, the filter was dried and then wrapped in a cellophane bag, and 10 ml of a liquid scintillation cocktail was added. The amount of energy (β-ray radioactivity count) trapped in the filter was measured with Beta Plate 1205.

A count measured for a sample containing no Rho kinase was taken as a background count (= activity 0%), and a count (the phosphorylating-activity of Rho kinase) measured for a sample containing no compound was taken as activity of 100%. A compound concentration at which the phosphorylation reaction was inhibited by 50% was taken as an IC₅₀ value for Rho kinase.

IC₅₀ values for Rho kinase obtained by this test were as follows; the compound of Example 10: 0.62 µg/ml, the compound of Example 113: 0.0065 µg/ml, the compound of Example 141: 0.0082 µg/ml, the compound of Example 158: 0.0080 µg/ml, the compound of Example 159: 0.0073 µg/ml, the compound of Example 162: 0.0071 µg/ml, and the compound of Example 163: 0.0082 µg/ml.

### INDUSTRIAL APPLICABILITY

The compounds of the present invention have inhibitory effect on Rho kinase and hence are useful as a therapeutic agent for diseases which are such that morbidity due to them is expected to be improved by inhibition of Rho kinase and secondary effects such as inhibition of the Na⁺/H⁺ exchange transport system caused by the Rho kinase inhibition, for example, hypertension, peripheral circulatory disorder, angina pectoris, cerebral vasospasm, premature birth and asthma which are improved by smooth muscle relaxing effect, and diseases (chronic arterial obstruction and cerebrovascular accident) caused by hyperaggregability of platelet; diseases such as arteriosclerosis, fibroid lung, fibroid liver, liver failure, fibroid kidney, renal glomerulosclerosis, kidney failure, organ hypertrophy, prostatic hypertrophy, complications of diabetes, blood vessel restenosis, and cancer, which diseases are improved by inhibitory effect on cell over-proliferation·emigration·fibrosing (e.g. fibroblast proliferation, smooth muscle cell proliferation mesangium cell proliferation and hemoendothelial cell proliferation); cardiac hypertrophy; heart failure; ischemic diseases; inflammation; autoimmune diseases; AIDS; osteopathy such as osteoporosis; brain functional disorder; infection of digestive tracts with bacteria; sepsis; adult respiratory distress syndrome; retinopathy; glaucoma; and erectile dysfunction.

## Claims

1. A compound represented by formula (1): 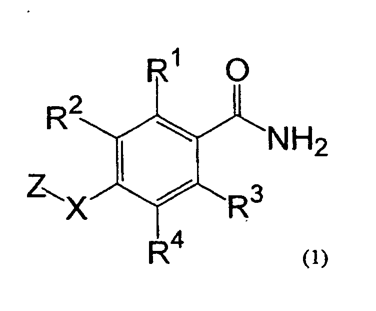 wherein X is a single bond or a substituted or unsubstituted lower alkylene group (the -CH₂- group(s) of said lower alkylene group may be substituted by one or more groups which may be the same or different and are selected from groups represented by the formulas: -O-, -S(O)ₙ-, -N(R⁵)-, -N(R⁶)C(=O)-, -C(=O)N(R⁶)-, -N(R⁶)S(O)₂-, -S(O)₂N(R⁶)- and -C(=O)-, benzene ring and cycloalkane rings (the -CH₂- group(s) in said cycloalkane ring may be substituted by one or more groups which may be the same or different and are selected from groups represented by the formulas: -O-, -S-, -N(R⁷)- and -C(=O)-), and a combination (s) of any adjacent two carbon atoms of said lower alkylene group may form one or more double or triple bonds which may be the same or different);
Z is a saturated or unsaturated monocyclic hydrocarbon ring group, a saturated or unsaturated polycyclic hydrocarbon ring group, a saturated or unsaturated monocyclic heterocyclic group, or a saturated or unsaturated polycyclic heterocyclic group (these groups may be unsubstituted or may have a substituent(s));
each of R¹, R², R³ and R⁴, which may be the same or different, is a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, a halogen atom, a nitro group, a cyano group, a carboxyl group, a substituted or unsubstituted alkyl group, a substituted or unsubstituted alkenyl group, a substituted or unsubstituted alkynyl group, a substituted or unsubstituted alkoxycarbonyl group, a substituted or unsubstituted acyl group, or a group represented by the formula: -OR⁹, -N(R¹⁰)R¹¹, -C(=O)N(R¹⁰)R¹¹, -S(O)₂N(R¹⁰)R¹¹ or -S(O)ₘR¹²;
n and m are independently 0, 1 or 2;
R⁵ (or R⁵s) and R⁷ (or R⁷s), which may be the same or different, and are a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, a substituted or unsubstituted alkyl group, a substituted or unsubstituted acyl group, a substituted or unsubstituted alkoxycarbonyl group, or a group represented by the formula: -C (=O)N(R¹⁰)R¹¹, -S(O)₂N(R¹⁰)R¹¹ or -S(O)ₘR¹², independently, when it exists more than one;
R⁶ (or R⁶s) and R⁹ (or R⁹s), which may be the same or different, and are a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, a substituted or unsubstituted alkyl group, a substituted or unsubstituted acyl group, or a substituted or unsubstituted alkoxycarbonyl group, independently, when it exists more than one;
R¹⁰ (or R¹⁰s) and R¹¹ (or R¹¹s), which may be the same or different, and are a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, a substituted or unsubstituted alkyl group, a substituted or unsubstituted acyl group, or a substituted or unsubstituted alkoxycarbonyl group, independently, when it exists more than one, or R¹⁰ and R¹¹, when taken together with the nitrogen atom to which they are bonded, represent a saturated 3- to 8-membered cyclic amino group which may contain other heteroatom(s) in the ring (said cyclic amino group may be substituted by one or more substituted or unsubstituted alkyl groups, -S(O)ₘR¹² groups or -OR⁹ groups) ; and
R¹² is a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, or a substituted or unsubstituted alkyl group;
said compound not including the following compounds:
(i) compounds in which X is a group represented by the formula: -O- or -S-, and Z is pyrrolidin-3-yl having a substituent at the 4-position, piperidin-3-yl having a substituent at the 4-position, piperidin-4-yl having a substituent at the 3-position, homopiperidin-3-yl having a substituent at the 4-position, homopiperidin-4-yl having a substituent at the 5-position, or homopiperidin-4-yl having a substituent at the 3-position, said substituent of each of them being an oxo group, a hydroxyl group, a substituted or unsubstituted alkoxy group, a substituted or unsubstituted alkenyloxy group, a substituted or unsubstituted alkynyloxy group, a substituted or unsubstituted aralkyloxy group, an alkenyloxy group substituted by a substituted or unsubstituted aryl group, an alkynyloxy group substituted by a substituted or unsubstituted aryl group, a substituted or unsubstituted aryloxy group, a substituted or unsubstituted acyloxy group, a benzyloxycarbonyloxy group, or an alkylcarbamoyloxy group, and
(ii) compounds in which X is a group represented by the formula: -O-, and Z is a group represented by the formula: 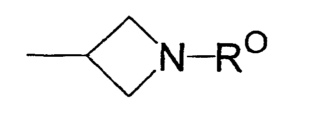
wherein R⁰ is a hydrogen atom, a substituted or unsubstituted carbamoyl group, or a substituted or unsubstituted thiocarbamoyl group,
a prodrug of said compound, or a pharmaceutically acceptable salt of said compound or prodrug.

2. A compound, a prodrug thereof or a pharmaceutically acceptable salt of the compound or prodrug according to claim 1, wherein X is a group represented by the formula: -N(R⁵)-, -C(=O)N(R⁶)-, -CH₂N(R⁵)-, -CH₂- or -O-.

3. A compound, a prodrug thereof or a pharmaceutically acceptable salt of the compound or prodrug according to claim 1, wherein X is a group represented by the formula: -N(R⁵)-.

4. A compound, a prodrug thereof or a pharmaceutically acceptable salt of the compound or prodrug according to claim 1, wherein X is a group represented by the formula: -O-.

5. A compound, a prodrug thereof or a pharmaceutically acceptable salt of the compound or prodrug according to claim 1, 2, 3 or 4, wherein at least one of R¹ and R³ is a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a halogen atom, a nitro group, a cyano group, a carboxyl group, a substituted or unsubstituted alkyl group, a substituted or unsubstituted alkenyl group, a substituted or unsubstituted alkynyl group, a substituted or unsubstituted alkoxycarbonyl group, a substituted or unsubstituted acyl group, or a group represented by the formula: -OR⁹, -N(R¹⁰)R¹¹, -C(=O)N(R¹⁰)R¹¹, -S(O)₂N(R¹⁰)R¹¹ or -S(O)ₘR¹².

6. A compound, a prodrug thereof or a pharmaceutically acceptable salt of the compound or prodrug according to claim 1, 2, 3 or 4, wherein at least one of R² and R⁴ is a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a halogen atom, a nitro group, a cyano group, a carboxyl group, a substituted or unsubstituted alkyl group, a substituted or unsubstituted alkenyl group, a substituted or unsubstituted alkynyl group, a substituted or unsubstituted alkoxycarbonyl group, a substituted or unsubstituted acyl group, or a group represented by the formula: -OR⁹, -N(R¹⁰)R¹¹, -C(=O)N(R¹⁰)R¹¹, -S(O)₂N(R¹⁰)R¹¹ or -S(O)ₘR¹².

7. A compound, a prodrug thereof or a pharmaceutically acceptable salt of the compound or prodrug according to claim 1, 2, 3 or 4, wherein each of R¹ and R⁴, which may be the same or different, or each of R³ and R², which may be the same or different, is a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a halogen atom, a nitro group, a cyano group, a carboxyl group, a substituted or unsubstituted alkyl group, a substituted or unsubstituted alkenyl group, a substituted or unsubstituted alkynyl group, a substituted or unsubstituted alkoxycarbonyl group, a substituted or unsubstituted acyl group, or a group represented by the formula: -OR⁹, -N(R¹⁰)R¹¹, -C(=O)N(R¹⁰)R¹¹, -S(O)₂N(R¹⁰)R¹¹ or -S(O)ₘR¹².

8. A compound, a prodrug thereof or a pharmaceutically acceptable salt of the compound or prodrug according to claim 1, 2, 3, 4, 5 or 7, wherein at least one of R¹ and R³ is a group represented by the formula: -OR⁹ or a halogen atom.

9. A compound, a prodrug thereof or a pharmaceutically acceptable salt of the compound or prodrug according to claim 1, 2, 3, 4, 5 or 7, wherein at least one of R¹ and R³ is a methoxy group.

10. A compound, a prodrug thereof or a pharmaceutically acceptable salt of the compound or prodrug according to claim 1, 2, 3, 4, 5 or 7, wherein at least one of R¹ and R³ is a fluorine atom.

11. A compound, a prodrug thereof or a pharmaceutically acceptable salt of the compound or prodrug according to claim 1, 2, 3, 4, 6 or 7, wherein at least one of R² and R⁴ is a halogen atom.

12. A compound, a prodrug thereof or a pharmaceutically acceptable salt of the compound or prodrug according to claim 1, 2, 3, 4, 6 or 7, wherein at least one of R² and R⁴ is a chlorine atom.

13. A compound, a prodrug thereof or a pharmaceutically acceptable salt of the compound or prodrug according to claim 1, 2, 3, 4 or 7, wherein R¹ is a group represented by the formula: -OR⁹ or a halogen atom and R⁴ is a halogen atom; or R³ is a group represented by the formula: -OR⁹ or a halogen atom and R² is a halogen atom.

14. A compound, a prodrug thereof or a pharmaceutically acceptable salt of the compound or prodrug according to claim 1, 2, 3, 4 or 7, wherein R¹ is a methoxy group and R⁴ is a chlorine atom; or R³ is a methoxy group and R² is a chlorine atom.

15. A compound, a prodrug thereof or a pharmaceutically acceptable salt of the compound or prodrug according to claim 1, 2, 3, 4 or 7, wherein R¹ is a fluorine atom and R⁴ is a chlorine atom; or R³ is a fluorine atom and R² is a chlorine atom.

16. A compound, a prodrug thereof or a pharmaceutically acceptable salt of the compound or prodrug according to any one of claims 1 to 15, wherein Z is a substituted or unsubstituted saturated monocyclic heterocyclic group or a substituted saturated monocyclic hydrocarbon ring group, and the substituent(s) of the saturated monocyclic hydrocarbon ring group is a group represented by the formula: -N(R^{Z1})R^{Z2} wherein R^{Z1} (or R^{Z1}s) and R^{Z2} (or R^{Z2}s), which may be the same or different, and are a hydrogen atom, a substituted or unsubstituted alkyl group or a substituted or unsubstituted aralkyl group, independently, when it exists more than one.

17. A compound, a prodrug thereof or a pharmaceutically acceptable salt of the compound or prodrug according to claim 1, said compound being 4-[trans-(4-aminocyclohexyl)amino]-2-fluorobenzamide, 4-[trans-(4-aminocyclohexyl)- amino]-5-chloro-2-fluorobenzamide, 5-chloro-4-{[trans-4-(isopropylamino)cyclohexyl]amino}-2-methoxybenzamide, 4-[trans-(4-aminocyclohexyl)amino]-2,5-difluorobenzamide or 5-chloro-2-fluoro-4-(piperidin-4-ylamino)benzamide.

18. A medicament comprising a compound, a prodrug thereof or a pharmaceutically acceptable salt of the compound or prodrug according to any one of claims 1 to 17.

19. A Rho kinase inhibitor comprising a compound, a prodrug thereof or a pharmaceutically acceptable salt of the compound or prodrug according to any one of claims 1 to 17.

20. A pharmaceutical composition for the treatment of hypertension, peripheral circulatory disorder, angina pectoris, cerebral vasospasm, premature birth, asthma, cerebrovascular accident, arteriosclerosis, fibroid lung, fibroid liver, fibroid kidney, renal glomerulosclerosis, kidney failure, prostatic hypertrophy, complications of diabetes, blood vessel restenosis, cancer, cardiac hypertrophy, heart failure, ischemic diseases, inflammation, autoimmune diseases, AIDS, osteopathy, brain functional disorder, infection of digestive tracts with bacteria, sepsis, adult respiratory distress syndrome, retinopathy, glaucoma, or erectile dysfunction, which comprises a compound represented by formula (1): 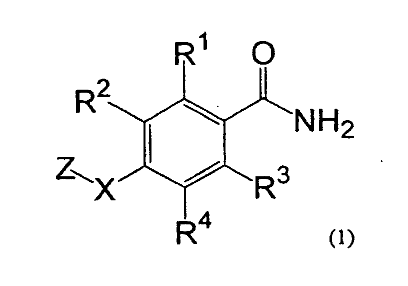 wherein X is a single bond or a substituted or unsubstituted lower alkylene group (the -CH₂- group(s) of said lower alkylene group may be substituted by one or more groups which may be the same or different and are selected from groups represented by the formulas: -O-, -S(O)ₙ-, -N(R⁵)-, -N(R⁶)C(=O)-, -C(=O)N(R⁶)-, -N(R⁶)S(O)₂-, -S(O)₂N(R⁶)- and -C(=O)-, benzene ring and cycloalkane rings (the -CH₂- group(s) in said cycloalkane ring may be substituted by one or more groups which may be the same or different and are selected from groups represented by the formulas: -O-, -S-, -N(R⁷)- and -C(=O)-), and a combination(s) of any adjacent two carbon atoms of said lower alkylene group may form one or more double or triple bonds which may be the same or different);
Z is a saturated or unsaturated monocyclic hydrocarbon ring group, a saturated or unsaturated polycyclic hydrocarbon ring group, a saturated or unsaturated monocyclic heterocyclic group, or a saturated or unsaturated polycyclic heterocyclic group (these groups may be unsubstituted or may have a substituent(s));
each of R¹, R², R³ and R⁴, which may be the same or different, is a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, a halogen atom, a nitro group, a cyano group, a carboxyl group, a substituted or unsubstituted alkyl group, a substituted or unsubstituted alkenyl group, a substituted or unsubstituted alkynyl group, a substituted or unsubstituted alkoxycarbonyl group, a substituted or unsubstituted acyl group, or a group represented by the formula: -OR⁹, -N(R¹⁰)R¹¹, -C(=O)N(R¹⁰)R¹¹, -S(O)₂N(R¹⁰)R¹¹ or -S(O)ₘR¹²;
n and m are independently 0, 1 or 2;
R⁵ (or R⁵s) and R⁷ (or R⁷s), which may be the same or different, and are a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, a substituted or unsubstituted alkyl group, a substituted or unsubstituted acyl group, a substituted or unsubstituted alkoxycarbonyl group, or a group represented by the formula: -C(=O)N(R¹⁰)R¹¹, -S(O)₂N(R¹⁰)R¹¹ or -S(O)ₘR¹², independently, when it exists more than one;
R⁶ (or R⁶s) and R⁹ (or R⁹s), which may be the same or different, and are a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, a substituted or unsubstituted alkyl group, a substituted or unsubstituted acyl group, or a substituted or unsubstituted alkoxycarbonyl group, independently, when it exists more than one;
R¹⁰ (or R¹⁰s) and R¹¹ (or R¹¹s), which may be the same or different, and are a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, a substituted or unsubstituted alkyl group, a substituted or unsubstituted acyl group, or a substituted or unsubstituted alkoxycarbonyl group, independently, when it exists more than one, or R¹⁰ and R¹¹, when taken together with the nitrogen atom to which they are bonded, represent a saturated 3- to 8-membered cyclic amino group which may contain other heteroatom(s) in the ring (said cyclic amino group may be substituted by one or more substituted or unsubstituted alkyl groups, -S(O)ₘR¹² groups or -OR⁹ groups); and
R¹² is a saturated or unsaturated monocyclic hydrocarbon ring group, a saturated or unsaturated polycyclic hydrocarbon ring group, a saturated or unsaturated monocyclic heterocyclic group, a saturated or unsaturated polycyclic heterocyclic group (these groups may be unsubstituted or may have a substituent(s)), a hydrogen atom, or a substituted or unsubstituted alkyl group;
said compound not including the following compounds:
compounds in which X is a group represented by the formula: -O-, and Z is pyrrolidin-3-yl having a substituent at the 4-position, said substituent being a hydroxyl group, an alkoxy group, a benzyloxycarbonyloxy group or an alkylcarbamoyloxy group,
a prodrug of said compound, or a pharmaceutically acceptable salt of said compound or prodrug.

21. A method for treating hypertension, peripheral circulatory disorder, angina pectoris, cerebral vasospasm, premature birth, asthma, cerebrovascular accident, arteriosclerosis, fibroid lung, fibroid liver, fibroid kidney, renal glomerulosclerosis, kidney failure, prostatic hypertrophy, complications of diabetes, blood vessel restenosis, cancer, cardiac hypertrophy, heart failure, ischemic diseases, inflammation, autoimmune diseases, AIDS, osteopathy, brain functional disorder, infection of digestive tracts with bacteria, sepsis, adult respiratory distress syndrome, retinopathy, glaucoma, or erectile dysfunction, which comprises administering an effective amount of a compound, a prodrug thereof or a pharmaceutically acceptable salt of the compound or prodrug according to claim 20 to a patient who needs the treatment.

22. Use of a compound, a prodrug thereof or a pharmaceutically acceptable salt of the compound or prodrug according to claim 20 in the manufacture of a medicament for the treatment of hypertension, peripheral circulatory disorder, angina pectoris, cerebral vasospasm, premature birth, asthma, cerebrovascular accident, arteriosclerosis, fibroid lung, fibroid liver, fibroid kidney, renal glomerulosclerosis, kidney failure, prostatic hypertrophy, complications of diabetes, blood vessel restenosis, cancer, cardiac hypertrophy, heart failure, ischemic diseases, inflammation, autoimmune diseases, AIDS, osteopathy, brain functional disorder, infection of digestive tracts with bacteria, sepsis, adult respiratory distress syndrome, retinopathy, glaucoma, or erectile dysfunction.
